# EUROPEAN PATENT SPECIFICATION

(11) **EP 1 551 448 B1**
(45) Date of publication and mention of the grant of the patent: **08.05.2013**
(21) Application number: 03759526.1
(22) Date of filing: 26.09.2003
(51) Int. Cl.: A61P 35/00, A61K 39/00, A61K 39/385, A61K 39/44

(54) **TARGETED CD1D MOLECULES**
CD1D-TARGETMOLEKÜLE
MOLECULES CD1D CIBLEES

(30) Priority: 27.09.2002 EP 02405838
(43) Date of publication of application: 13.07.2005
(73) Proprietor: Vaccinex, Inc., Rochester, NY 14620 (US)
(72) Inventor: ZAUDERER, Maurice, Pittsford, NY 14534 (US); ROBERT, Bruno, Appt 83 34000 Montpellier (FR); DONDA, Alena, CH-1080 Les Cullayes (CH); CESSON, Valerie, F-74500 Saint Gingolph (FR); MACH, Jean-Pierre, CH-1293 Bellevue (CH)
(74) Representative: O'Brien, Simon Warwick
(86) International application number: PCT/US2003/030238
(87) International publication number: WO 2004/029206

(56) References cited:
- WO-A-01/71005
- WO-A-01/90198
- WO-A2-01/78768
- US-A1- 2002 071 842
- STIRNEMANN K ET AL: "Sustained activation and tumor targeting of NKT cells using a CD1d-anti-HER2-scFv fusion protein induce antitumor effects in mice", JOURNAL OF CLINICAL INVESTIGATION 200803 US LNKD- DOI:10.1172/JCI33249, vol. 118, no. 3, March 2008 (2008-03), pages 994-1005, ISSN: 0021-9738
- "The structure of a typical antibody molecule" In: Janeway et al.: "Immunobiology: The immune system in health and disease", 2011, Garland Science, New York 5th ed

## Description

### BACKGROUND OF THE INVENTION

### Field of the Invention

The invention generally relates to the field of immunology.

### Background Art

The natural immune system strikes a complex balance between highly aggressive, protective immune responses to foreign pathogens and the need to maintain tolerance to normal tissues. In recent years there has been increasing recognition that interactions among many different cell types contribute to maintaining this balance. Such interactions can, for example, result in polarized responses with either production of pro-inflammatory cytokines by TH1 type T cells or production of interleukin-4 (IL-4) by TH2 type T cells that suppress TH1 activity. In a number of different animal models, T cell polarization to TH1 has been shown to favor protective immunity to tumors or infectious pathogens whereas T cell polarization to TH2 can be a critical factor in preventing development of cell-mediated autoimmune disease. The conditions that determine whether immune stimulation will result in aggressive cell-mediated immunity or in down regulation of such responses are highly localized in the sense that each tissue is comprised of a distinctive set of antigen presenting cells (APC) and lymphocyte lineages that interact to favor different immune responses. For example, under optimal conditions, the dendritic cells (DC) localized in a normal tissue may represent predominantly a lineage and stage of maturation that favors tolerogenic interactions and serves as a barrier to cell-mediated autoimmunity whereas a tumor or site of infection will attract mature myeloid dendritic cells that stimulate potent cell-mediated immune responses.

CD1d-restricted NKT cells are a unique class of non-conventional T cells that appear to play an important role in defining the outcome of immune stimulation in the local environment. They share with the larger class of NKT cells the expression of markers of both the T cell and natural killer (NK) cell lineages although several studies suggest that expression of NK markers such as CD161 and NKG2d by NKT cells is a function of both stage of maturation and state of activation (Chen et al., J Immunol 1997; 158 :5112-9). As such, NKT cells are considered as part of innate immunity like NK cells and in humans their frequency in normal individuals can be as high as 2.0% of total T lymphocytes (Gumperz et al., 2002. J Exp Med 195:625; Lee et al., 2002. J Exp Med 195:637).

CD1d-restricted NKT cells are distinguished from other NKT cells by their specificity for lipid and glycolipid antigens presented by the monomorphic MHC class Ib molecule, CD1d (Kawano et al., Science 278 (1997), pp. 1626-16292). CD1d is a non-MHC encoded molecule that associates with β2-microglobulin and is structurally related to classical MHC class I molecules. In contrast to MHC class I and class II molecules that sample peptides from the cytosol and endocytic compartments and transport them to the cell surface where they can be recognized by T cells, CD1d has a hydrophobic antigen-binding pocket that is specialized for binding the hydrocarbon chains of lipid tails or hydrophobic peptides (Zeng et al., Science 277 (1997), pp. 339-345). CD1d is known to bind a marine sponge derived α-glycosylated sphingolipid, α-galactosylceramide (α-GalCer), and related molecules such as sphingolipids with α-linked galactose or glucose but not mannose (Kawano et al., Science 278 (1997), pp. 1626-1629; and Zeng et al., Science 277 (1997), pp. 339-345). As discussed below, the ability to activate many CD1d-restriced NKT cells by stimulation with α-GalCer or related molecules bound to CD1d of antigen presenting cells has greatly facilitated functional analysis of this non-conventional T cell subset. In the absence of inflammation, CD1d-restricted NKT cells have been shown to localize preferentially in certain tissues like thymus, liver and bone marrow (Wilson et al., 2002. Trends Mol Med 8:225) and antitumor activity of NKT cells has been mainly investigated in mouse liver metastasis.

NKT cells have an unusual ability of secreting both TH1 and TH2 cytokines and potent cytotoxic as well as regulatory functions have been documented in inflammation, autoimmunity and tumor immunity (Bendelac et al., 1995Science 268:863; Chen and Paul. 1997. J Immunol 159:2240; and Exley et al.,1997. J Exp Med 186:109). Distinct functional subsets of NKT cells have been characterized as regards their cytokine profiles, expression of several NK receptors, tissue segregation and CD1d dependance (Gumperz et al., 2002. J Exp Med 195:625; Lee et al., 2002. J Exp Med 195:637; Eberl et al., 1999. J Immunol 162:6410; and MacDonald, 2002, Curr Opin Immunol 14:250).

Among the CD1d-restricted NKT cells is a subset that expresses a highly conserved αβT cell receptor (TCR). In man this invariant TCR is comprised of Vα24Jα15 in association with Vβ11 whereas in mice the receptor comprises the highly homologous Vα14Jα18 and Vβ8.2. Other CDld-restricted NKT cells express more variable TCR. Both TCR invariant and TCR variant classes of CD1d-restricted T cells can be detected by binding of CDld-tetramers loaded with α-GalCer (Benlagha et al., J Exp Med 191 (2000), pp. 1895-1903; Matsuda et al., J Exp Med 192 (2000), pp. 741-754; and Karadimitris et al., Proc Natl Acad Sci USA 98 (2001), pp. 3294-3298). CD1d-restricted NKT cells, as defined in this application (CD1d-NKT), include cells that express either invariant or variant TCR and that bind or are activated by CD1d loaded with either α-GalCer or with related sphingolipids that have α-linked galactose or glucose including molecules such as OCH, which differs from α-GalCer by having a shortened long-chain sphingosine base (C5 vs. C14) and acyl chain (C24 vs. C26) (Miyamoto et al., Nature 2001 413:531-4). A common feature of CD1d-NKT cells is that, in contrast to conventional T cells, these lymphocytes often have a surface phenotype (CD44hiCD62L-CD69+) that is characteristic of recently activated or memory T cells. This striking phenotype has been explained by an in vivo autoreactivity of these T cells to still unknown autologous ligands presented by CD1d (Kronenberg and Gapin. 2002Nat Rev Immunol 2:557).

CD1d-NKT have been shown to have direct cytotoxic activity against targets that express CD1d. It is likely, however, that the effect of CD1d-NKT on immune responses is amplified through recruitment of other lymphocytes either by direct interaction or, perhaps even more importantly, by indirect recruitment through interaction with DC. CD1d-NKT have the unique ability to secrete large quantities of IL-4 and IFN-γ early in an immune response. Secretion of IFN-γ induces activation of DC which produce interleukin-12 (IL-12). IL-12 stimulates further IFN-γ secretion by NKT cells and also leads to activation of NK cells which secrete more IFN-γ.

Since CD1d-NKT are able to rapidly secrete large amounts of both IL-4 and IFN-γ, the polarization of immune responses will depend on whether the effect of pro-inflammatory IFN-γ or anti-inflammatory IL-4 cytokines predominate. This has been reported to be, in part, a function of the relative frequency of different subsets of CD1d-NKT. These subsets include (i) an invariant CD1d-NKT population that is negative for both CD4 and CD8 and that gives rise to predominantly a TH1 type response including secretion of pro-inflammatory IFN-γ and TNF-α and (ii) a separate population of CD1d-NKT that is CD4+ and that gives rise to both a TH1 type and TH2 type response including secretion of the anti-inflammatory Th2-type cytokines IL-4, IL-5, IL-10 and IL-13 (Lee et al., J Exp Med 2002; 195 :637-41; and Gumperz et al., J Exp Med 2002; 195 :625-36). Local factors that influence activation of CD1d-NKT subsets include the cytokine environment and, importantly, the DC that are recruited to that environment.

The availability of a defined antigen, α-GalCer, that can be employed to specifically activate the CD1d-NKT cell subset has made it possible to examine the role of these non-conventional T cells in a variety of immune responses. Administration of the α-GalCer lipid antigen has a dramatic effect on a number of different microbial infections, including protective effects in murine malaria, fungal and hepatitis B virus infections (Kakimi et al, J Exp Med 192 (2000), pp. 921-930; Gonzalez-Aseguinolaza et al., Proc Natl Acad Sci USA 97 (2000), pp. 8461-8466; and Kawakami et al., Infect Immun 69 (2001), pp. 213-220). Dramatic effects of administration of α-GalCer have also been observed in animal models of tumor immunity. Stimulation with α-GalCer or with cytokines like IL-12 suppresses lung and liver metastases in an NKT dependent manner as shown by loss of protection in mice that do not develop CD1d-NKT because they are deficient in CD1d or in the NKT TCRα chain that is dominantly expressed in CD1d-NKT (Smyth et al., 2002. Blood 99:1259; Hayakawa et al., Eur J Immunol 31:1720; Takeda et al., Int Immunol 12:909). One study (Cui et al., Science 278 (1997), pp. 1623-1626) demonstrated that the antitumor response to melanomas observed in tumor-bearing mice treated with IL-12 is solely due to NKT cells. In contrast to normal mice with CD1d-NKT cells, IL-12 treated tumor-bearing mice deficient in the Jα15 gene (which are deficient in NKT cells, since most mouse CD1d-NKT cells express Jα15 positive invariant TCR) could not control B16 melanoma growth and metastases.

A number of indirect mechanisms contribute to the protective effect of CD1d-NKT cells. Activation of NKT cells by administration of α-GalCer in vivo results in concomitant activation of NK cells (Eberl and MacDonald, Eur. J. Immunol. 30 (2000), pp. 985-992; and Carnaud et al., J. Immunol. 163 (1999), pp. 4647-4650). In mice deficient in NKT cells, α-GalCer is unable to induce cytotoxic activity by NK cells. NKT cells also enhance the induction of classical MHC class I restricted cytotoxic T cells (Nishimura et al., Int Immunol 2000; 12 :987-94; and Stober et al., J Immunol 2003; 170:2540-8).

The participation of NKT cells at the earliest stages of the protective immune response to many pathogens (infections (Kakimi et al, J Exp Med 192 (2000), pp. 921-930; Gonzalez-Aseguinolaza et al., Proc Natl Acad Sci USA 97 (2000), pp. 8461-8466; Kawakami et al., Infect Immun 69 (2001), pp. 213-220; and Bendelac and Medzhitov, J Exp Med 2002; 195 :F19-23) and tumors (Kobayashi et al., Oncol Res 1995; 7 :529-34; and Smyth et al., Curr Opin Immunol 2002; 14:165-71) is a reflection of their direct cytotoxic activity as well as their important contribution to general mobilization of an aggressive cell-mediated immune response. Extensive evidence suggests, however, that NKT cells also function to suppress autoimmunity (Hong et al., Nature Med 2001; 7 :1052-6; Beaudoin et al., Immunity 2002; 17 :725-36; Wilson et al., Trends Mol Med 2002; 8 :225-31; Shi et al., Proc Natl Acad Sci USA 2001; 98 :6777-82; Naumov et al., Proc Natl Acad Sci USA 2001; 98 :13838-43; Sharif et al., Nature Med 2001; 7 :1057-62; Wang et al., J Exp Med 2001; 194 :313-20; Jahng et al., J Exp Med 2001; 194 :1789-99; Singh et al., J Exp Med 2001; 194:1801-11), maintain immune privilege (Sonoda et al., J Exp Med 1999; 190 :1215-26; Hong and Van Kaer, J Exp Med 1999; 190 :1197-1200), and support engraflment of transplanted tissues (Seino et al., Proc Natl Acad Sci USA 2001; 98 :2577-81; Zeng et al., J Exp Med 1999; 189 :1073-81). These include experiments in which administration of α-GalCer has been shown to protect against autoimmune diabetes in non-obese diabetic (NOD) mice (Hong et al., Nature Med 2001; 7 :1052-6; Wilson et al., Trends Mol Med 2002; 8 :225-31; Sharif et al., Nature Med 2001; 7 :1057-62) and against experimental autoimmune encephalomyelitis (EAE), a murine model of demyelinating disease (Jahng et al., J Exp Med 2001; 194 :1789-99; and Singh et al., J Exp Med 2001; 194 :1801-11). Conversely, deletion of NKT cells results in exacerbation or potentiation of disease in these models (Shi et al., Proc Natl Acad Sci USA 2001; 98 :6777-82). Parallel studies of the frequency of CD1d-NKT cells in human cancer and certain autoimmune diseases have demonstrated that a deficiency in such T cells is associated with progressive disease (Tahir et al. J. Immunol. 167:4046-50,2001; Sharif et al. J. Mol. Med. 80:290-300,2002; Sumidha et al. J.Exp.Med. 182:1163-68,1995; Illes et al. J. Immunol. 164:4375-81,2000; van der Vliet et al. Clin. Immunol. 100:144-148,2401). In contrast, some autoimmune diseases, including myasthenia gravis (Reinhaldt et al. Neurology 52:1485-87,1999), psoriasis (Bonish , J. Immunol. 165:4076-85,2000), ulcerative colitis (Sanbermann et al. Gastroenterology 119:119-128, 2000), and primary biliary cirrhosis (Kita et al. Gastroenterology 123:1031-43,2002) may have an etiology that reflects excessive IL-4 and IFN-γ production by CD1d-NKT cells. In the case of psoriasis this appears to be related to overexpression of CD1d in keratinocytes of chronic, active psoriatic plaques.

The divergent pro-inflammatory and anti-inflammatory effects of CD1d-NKT cells in different circumstances have been attributed to different functional subsets of CD1d-NKT and dendritic cells and to differences in the representation of these subsets in the local tissue environment. An interesting example is a study demonstrating that transfer of myeloid DC derived from pancreatic lymph nodes, but not from inguinal lymph nodes, of mice treated systemically with α-GalCer completely protects NOD female mice from diabetes [30]. As a result of this potential for divergent effects, systemic activation of CD1d-NKT, for example by administration of α-GalCer, may have an undesirable outcome in the treatment of specific disease. There is a need, therefore, for a means of targeted activation of CD1d-NKT in a specific local environment and/or by targeted interaction with a defined subset of dendritic cells.
WO 01/90198 discloses conjugates comprising a specific cell surface molecule and an antigenic complex of an MHC molecule and a peptide for activating T lymphoctyes.
WO 01/78768 discloses compounds comprising one or more MHC-peptide complexes linked to an antibody specific for a cell surface marker for modulting an immune response.

### SUMMARY OF THE INVENTION

The present invention provides compounds useful for modulating, i.e., either inhibiting or stimulating, an immune response. The compounds of the invention comprise one or more CD1d complexes linked to an antibody or fragment thereof specific for a cell surface marker. The CD1d complexes comprise a CD1d molecule or fragment thereof, a β2-microglobulin molecule or fragment thereof, and may further comprise a lipid, glycolipid, or hydrophobic peptide linked to the CD1d molecule. In certain embodiments, the compounds of the invention further comprise a costimulatory molecule.

The CD1d complexes may be directly linked or fused to the antibody, either directly or through a linker sequence or molecule. In certain embodiments, the CD1d molecules are linked to the antibody or fragment thereof through a multivalent compound.

In certain embodiments, the antibody is specific for a cell surface marker of a tumor cell. In other embodiments, the antibody is specific for a cell surface marker of a target of autoimmunity. In other embodiments, the antibody is specific for a cell surface marker of infected cells. This marker may be either directly encoded by the pathogen or induced by infection of the host cell as taught by WO 0227027, published 4 Apr 2002 In still other embodiments, the antibody is specific for a cell surface marker of a dendritic cell.

Also provided are methods of modulating, i.e., either stimulating or inhibiting, an immune response, comprising administering to an animal an effective amount of a compound or composition of the invention. In certain embodiments, an immune response against tumor cells is stimulated by administering a compound of the invention to an animal. In other embodiments, an autoimmune response is inhibited by administering a compound of the invention to an animal.

It is to be understood that both the foregoing summary and the following detailed description are exemplary and explanatory and are intended to provide further explanation of the invention as claimed.

### BRIEF DESCRIPTION OF THE DRAWINGS/FIGURES

The present invention will be described with reference to the accompanying drawings, wherein like reference numbers indicate identical or functionally similar elements. Also, the leftmost digit(s) of the reference numbers identify the drawings in which the associated elements are first introduced.

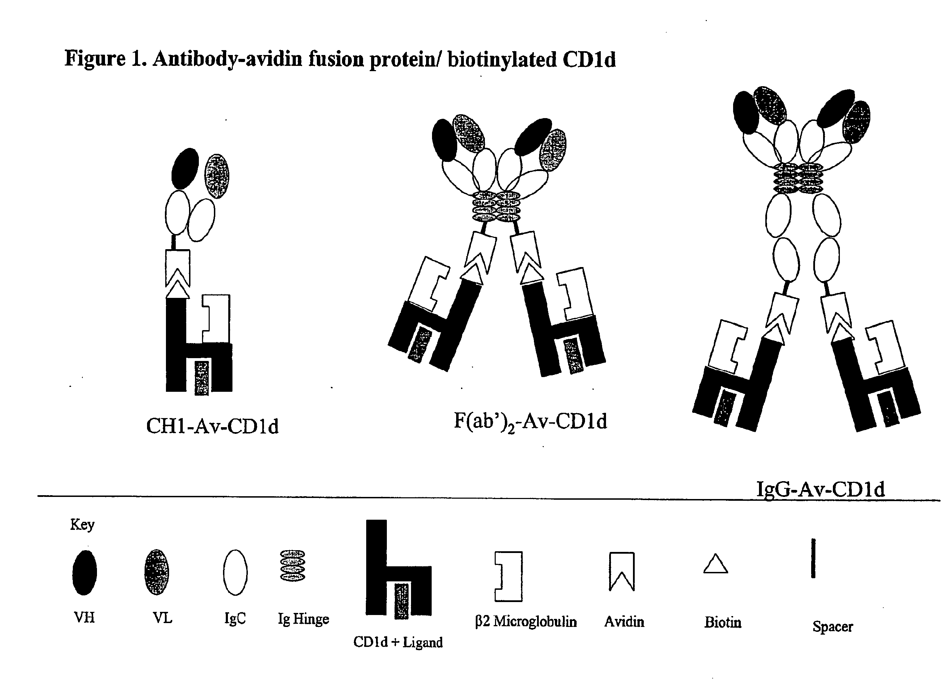

Figure 1: Schematic representation of Antibody-avidin fusion protein/biotinylated CD1d. In three separate embodiments, CD1d with bound ligand is biotinylated at the CD I d carboxyl terminus and binds to a fusion protein of Fab-avidin, F(ab')2-avidin, or IgG-avidin. All 3 fusion proteins are represented with avidin bound at the carboxyl terminus of the immunoglobulin heavy chain or fragment thereof. As described below, alternatives include avidin fusion to the carboxyl terminus of the immunoglobulin light chain.

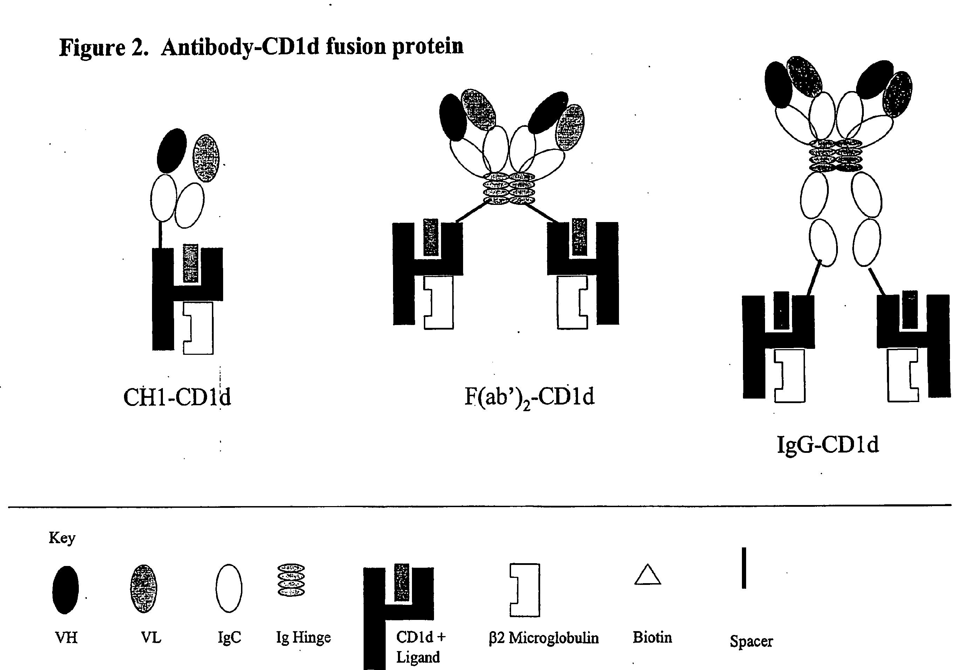

Figure 2: Schematic representation of Antibody-CD1d fusion protein. In three separate embodiments, CD1d with bound ligand is fused directly to Fab, F(ab')2, or full IgG. All 3 fusion proteins are represented with the amino terminus of CD1d fused to the carboxyl terminus of the immunoglobulin heavy chain or fragment thereof. As described below, alternatives include CD1d fusion to the carboxyl terminus of the immunoglobulin light chain or to amino terminus of either the immunoglobulin light chain or immunoglobulin heavy chain or fragment thereof.

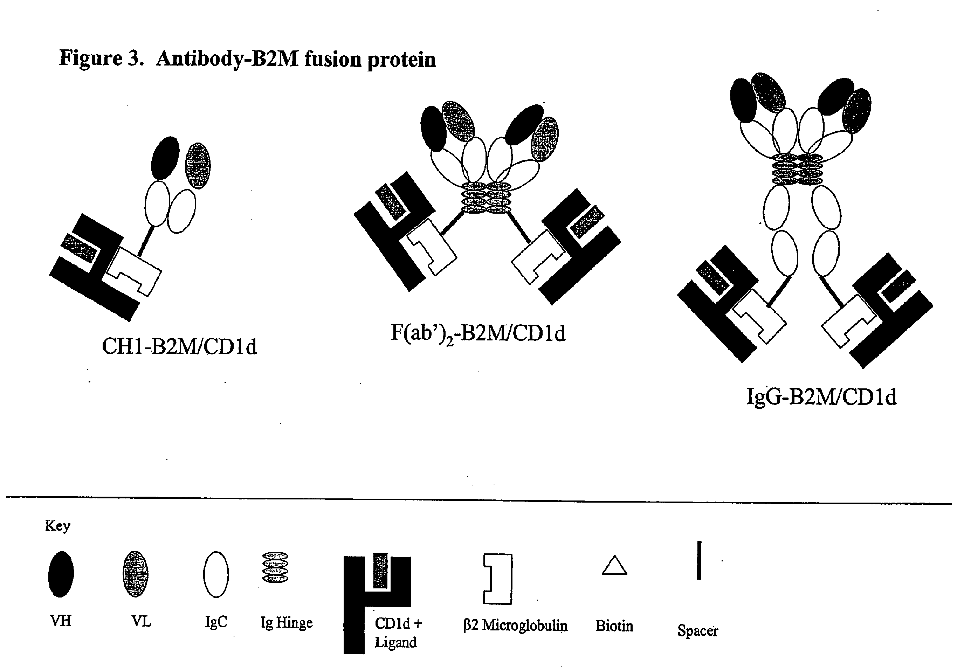

Figure 3: Schematic representation of Antibody-B2M fusion protein associated with CD1d. In three separate embodiments, β2-microglobulin is fused to the carboxyl terminus of Fab, F(ab')2, or full IgG. All 3 fusion proteins are represented with the amino terminus of β2-microglobulin fused to the carboxyl terminus of the immunoglobulin heavy chain or fragment thereof. As described below, alternatives include β2-microglobulin fusion to the carboxyl terminus of the immunoglobulin light chain or to amino terminus of either the immunoglobulin light chain or immunoglobulin heavy chain or fragment thereof. In all cases, the heavy chain of CD1d associates with the antibody- β2-microglobulin fusion protein and binds ligand. As taught by WO 9964597 published 16 December 1999 it is possible to introduce mutations into β2-microglobulin that increase affinity for the class I heavy chain so as to facilitate assembly and increase stability of the CD1d complex.

### DETAILED DESCRIPTION OF THE INVENTION

The present invention provides compounds for use as a medicament to generate an immune response against a tumor cell comprising:
(a) one or more antigen-loaded CD1d complexes which comprise a CD1d molecule associated with β2-microglobulin , wherein said antigen comprises a glycosylated sphingolipid, and wherein said antigen is bound in the antigen-binding groove of said CD1d molecule; and
(b) an antibody or fragment thereof specific for a cell surface marker of a tumor cell, wherein said antibody or fragment thereof is linked to said CD1d complex.
The present invention targets a CD1d molecule to particular cells by linking one or more CD1d complexes to an antibody or fragment thereof specific for a surface antigen of the targeted cell type. Depending on the targeted cell type, this will lead to either very efficient stimulation or inhibition of antigen specific T cell activity.

Previously, antibodies coupled to classical MHC class I molecules have been employed to target these MHC class I:peptide complexes to tumors for the purpose of activating classical MHC class I restricted cytotoxic T cells. (Robert et al., 2000, Eur. J. Immunol. 30: 3165-3170; Robert et al., 2001, Cancer Immunity, 1:2-15; and Donda et al., 2003, Cancer Immunity 3:11). A significant limitation of that method is that it does not lead to activation of the important class of non-conventional CD1d-NKT cells with their unique regulatory functions. In addition, because of the extensive polymorphism of classical MHC class I, such a strategy would require coupling of antibodies to many different MHC molecules in order to be suitable for application to a large and heterogeneous population. In marked contrast, the monomorphic CD1d molecule is suitable for activation of a broad spectrum of CD1d-NKT in an entire species. Moreover, targeted CD1d has application as a diagnostic or therapeutic agent not only for cancer and infectious diseases but also for a large class of autoimmune and inflammatory diseases that result from a failure to down modulate cell-mediated immune responses.

The present invention provides a means of targeting an activation or inhibitory signal for CD1d-NKT to a specific tissue or dendritic cell subset by coupling CD1d loaded with α-GalCer or related lipid molecules to an antibody specific for a tissue antigen or for a dendritic cell marker. This strategy allows regulated expression and/or expansion of CD1d-NKT at the site of a tumor or of an infection, or in a tissue that is the target of an organ-specific autoimmune response. The combination of the powerful regulatory and effector activities of CD1d-NKT together with tissue or tumor targeting properties of specific monoclonal antibodies affords a unique means of inducing a response appropriate to a specific tissue and disease.

The CD1d complexes (both the CD1d and β2-microglobulin portions) useful in the present invention may be autologous to any mammalian or avian species, for example, primates (esp. humans), rodents, rabbits, equines, bovines, canines, felines, etc. β2-microglobulin is typically not inflammatory in vivo. However, it is preferable to employ β2-microglobulin derived from the same species as is to be vaccinated so as to reduce the risk of a xenogeneic immune response.

In one embodiment, the CD1d complexes comprise a CD1d molecule or fragment thereof, a β2-microglobulin molecule or fragment thereof, and an antigen. The antigen is a lipid, glycolipid, or hydrophobic peptide bound in the antigen binding groove of the CD1d molecule.

In certain embodiments, the compound further comprises another protein with immunological activity. Preferably, the protein with immunological activity is a costimulatory molecule, such as B7.1 or B7.2. "B7" is used herein to generically refer to either B7.1 or B7.2. Particularly preferred is the extracellular domain of B7-1 (CD80) or B7-2 (CD86) that interacts with CD28 on T- and NK-cells, for example, as an amino terminal fusion to β2-microglobulin incorporated into the structure of the assembled CD1d molecule (WO 9964597, published 16 Dec 1999) or, alternatively, as an amino-terminal fusion to the CD1d heavy chain. CD28 acts as a costimulatory molecule on T lymphocytes, which is absolutely required in order to mediate the so-called second signal during primary T cell activation through antigen specific TCR- engagement (the complementary so-called first signal). On NK cells CD28 contributes to the induction of cytotoxicity against target cells expressing CD28 ligands (Chambers (1996) Immunity 5: 311). Like NK cells, CD1d-NKT are subject to inhibitory signals delivered by classical MHC class I molecules on the surface of interacting cells. Ikarashi et al. (J.Exp.Med. 194:1179-86,2001) have reported that in the interaction of CD28 positive NKT cells with B7 positive DC, the B7/CD28 interaction plays a critical role in overcoming the inhibitory signal otherwise delivered to NKT cells by classical MHC class I molecule expressed on the target cell surface. In this embodiment of the invention, incorporation of a B7 signaling molecule in the conjugate of the invention allows more effective and prolonged activation of CD1d-NKT cells.

Additionally, the protein with immunological activity may be a lymphokine or cytokine that modulates immune cell activation such as interleukins IL-1, IL-2, IL-3, IL-4, IL-5, IL-6, IL-10, IL-12, IL-15, IL-18; granulocyte-macrophage colony stimulating factor (GM-CSF); transforming growth factor. (TGF, e.g., TGFα and TGFβ); α interferons (e.g. IFNα); β interferons (e.g. IFNβ); γ interferons (e.g. IFNγ) or lymphocyte function-associated protein, such as LFA-1 or LFA-3; or an intercellular adhesion molecule, such as ICAM-1 or ICAM-2.
The CD1d complexes are linked to the antibody through either the CD1d molecule or fragment thereof or the β2 microglobulin molecule or fragment thereof. The CD1d complexes may be linked to either the light chain or the heavy chain of the antibody. As taught by WO 9964597, published 16 December 1999 it is possible to introduce mutations into β2-microglobulin that increase affinity for the class I heavy chain so as to facilitate assembly and increase stability of the CD1d complex in the fusion protein.

The CD1d complexes may be linked to either the carboxyl or amino terminus of the antibody, or they may be linked to the antibody at a site other than the carboxyl or amino termini. Preferably, the CD1d complexes are linked to the carboxyl terminus of the antibody.

The attachment of the CD1d complexes to the antibody chains may be direct, *i.e.*, without any intermediate sequence, or through a linker amino acid sequence, a linker molecule, or a chemical bond. For example, the coupling may be of a physical and/or chemical type. The antibody and CD1d complex may be coupled physically utilizing a carrier for example a Sepharose carrier (available from Pharmacia, Uppsala, Sweden) or recently developed microsphere technology. (Southern Research Institute).

Alternatively, the CD1d complexes domain and the antibodies may be linked together directly. A number of reagents capable of cross-linking proteins are known in the art, illustrative entities include: azidobenzoyl hydrazide, N-[4-(p- azidosalicylamino)butyl]-3'-[2'-pyridyldithio] propionamide), bis-sulfosuccinimidyl suberate, dimethyladipimidate, disuccinimidyltartrate, N-γ-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, formaldehyde and succinimidyl 4-[N-maleimidomethyl] cyclohexane-1-carboxylate.

In certain embodiments, the CD1d complexes domain may be linked in a fusion protein with the antibody. Fusion antibodies can be made using conventional recombinant nucleic acid techniques. The fusion may be direct or may contain spacers. A short linker amino acid sequence may be inserted between the CD1d complex and the antibody. The length of the linker sequence will vary depending upon the desired flexibility to regulate the degree of antigen binding and cross-linking. If a linker sequence is included, this sequence will preferably contain at least 3 and not more than 30 amino acids. More preferably, the linker is about 5, 6, 7, 8, 9, 10, 11, 12, 13, 14, 15, 20, or 25 amino acids long. Generally, the linker consists of short glycine/serine spacers, but any know amino acid may be used. Examples of linkers known to those skilled in the art include (Gly4Ser)3 (SEQ ID NO:1) and (Gly4Ser)2Gly3AlaSer (SEQ ID NO:2).

CD1d complexes fused to the carboxyl terminus of the exceptionally long IgG3 hinge region or to the CH3 domain, are especially far removed from possible interference with the antigen binding site or its ligand. Fc binding function is preserved in the compounds of this invention that are based on CH3 fusions. It is possible that this would extend the half-life of these compounds in vivo.

In certain embodiments, the CD1d complexes are linked to the antibody through a multivalent compound. The CD1d complexes may be linked to the multivalent compound through any site. In a preferred embodiment CD1d molecules are linked to the multivalent compound through the CD1d carboxyl terminus.] These compounds comprise 2 or more CD1d complexes. The compounds may comprise 2, 3, 4, 5, 6, 7, 8, 9 or 10 CD1d complexes.

Likewise, the antibody may be linked to the multivalent compound through any site. The antibody may be linked to the multivalent compound through the light chain, the heavy chain, both light chains, both heavy chains, one light chain and one heavy chain, or both light and both heavy chains and at either the amino or the carboxyl terminus. Preferably, the antibody is linked to the multivalent compound at the carboxyl terminus of the heavy chain.

Examples of multivalent compounds are chicken avidin or streptavidin (Shin, S.U. et al., J. Immunology 158: 4797-4804 (1997)) to which biotinylated CD1d complexes are bound (Altman, J. et al, Science 274:94-96 (1996); Boniface, J.J. et al., Immunity 9:459-66 (1998)); or a leucine zipper system.

Alternatively, the Cd1d complex can be genetically modified by including sequences encoding amino acid residues with chemically reactive side chains such as Cys or His. Such amino acids with chemically reactive side chains may be positioned in a variety of positions on the CD1d complex, preferably distal to the site where β2-microglobulin and CD1d interact. Suitable side chains can be used to chemically link two or more CD1d complexes to a suitable dendrimer particle. Dendrimers are synthetic chemical polymers that can have any one of a number of different functional groups on their surface (D. Tomalia, Aldrichimica Acta 26:91:101 (1993)). Exemplary dendrimers for use in accordance with the present invention include e.g. E9 starburst polyamine dendrimer and E9 combburst polyamine dendrimer, which can link cysteine residues. The CD1d molecule is modified to introduce a cysteine residue at the carboxyl terminus. Following synthesis in eukaryotic cells, a complete cysteine modified CD1d complex is assembled in vitro in the presence of β2-microglobulin. Cysteine modified CD1d will react with the maleimide groups on the various peptide backbones with either two, three, or four modified lysine residues for formation of CD1d dimers, trimers, and tetramers. In a preferred embodiment, a carboxyl terminal cysteine modified immunoglobulin chain or fragment thereof could also be synthesized for reaction with a maleimide-modified lysine residue on the same backbone peptide and at the same time as the cysteine modified CD1d.

Cochran, J.R. et al., Immunity 12:241-50 (2000) describe the use of chemically synthesized peptide-based cross-linking reagents in which two or more thiol-reactive maleimide groups are linked to lysine side chains in a flexible peptide of 8 to 19 residues containing glycine, serine, and glutamic acid in addition to the modified lysine residues. An isolated CD1d molecule is modified to introduce a cysteine residue at the carboxyl terminus. Cysteine modified CD1d molecules react with the maleimide groups on the various peptide backbones with either two, three, or four modified lysine residues for formation of dimers, trimers, and tetramers.

Another means of assembling polymeric complexes on specific antibody is to exploit the observation that defined amino acid substitutions in the GCN4 leucine zipper dimerization domain results in formation of highly stable trimeric and tetrameric structures of the synthetic peptide (Harbury, P.B. et al., Science 262:1401-7 (1993)). Pack, P. et al. J. Mol. Biol. 246:28-34 (1995) constructed tetravalent miniantibodies by fusing the modified GCN4-zipper to the carboxyl terminus of a single-chain Fv fragment via a flexible hinge region. Several additional modifications of the fusion protein improved yield from bacterial synthesis. Addition of a carboxyl terminal tag would facilitate purification. Targeted tetravalent CD1d could be assembled from a mixture of single chain antibody and CD1d complexes each separately fused through a hinge region to the modified GCN4-zipper motif.

The alternative embodiments of this invention, direct fusion of antibody and CD1d complexes or indirect association of antibody and CD1d complexes through a multivalent entity, are respectively advantageous in different situations. The direct fusion simplifies production of the compound while the multivalent entity, as indicated above, can present a larger number of more diverse ligands. In both cases it is desirable to design products that induce minimal immune reactivity. In the case of direct immunoglobulin-CD1d fusion proteins, this is accomplished by employing species compatible antibodies and CD1d complexes joined by simple linkers with a relatively non-immunogenic composition. Multivalent entities may be similarly selected to minimize immunogenicity. Chicken avidin is thought to be relatively nonimmunogenic because of its high concentration in egg products and the well-known propensity of oral infusion to induce immune tolerance (Shin, S.U. et al., J. Immunology 158: 4797-4804 (1997)). It may, in addition, be possible to develop protocols, including some that employ compounds of this invention, that induce specific tolerance to the multivalent entity.

The attachment site on the CD1d complex or antibody for binding to a multivalent compound may be naturally occurring, or may be introduced through genetic engineering. The site will be a specific binding pair member or one that is modified to provide a specific binding pair member, where the complementary pair has a multiplicity of specific binding sites. Binding to the complementary binding member can be a chemical reaction, epitope-receptor binding or hapten-receptor binding where a hapten is linked to the subunit chain.

In a preferred embodiment, the CD1d, β2 microglobulin, or antibody, contain an amino acid sequence which is a recognition site for a modifying enzyme. Modifying enzymes include BirA, various glycosylases, farnesyl protein transferase, and protein kinases. The group introduced by the modifying enzyme, e.g. biotin, sugar, phosphate, farnesyl, etc. provides a complementary binding pair member, or a unique site for further modification, such as chemical cross-linking, biotinylation, etc. that will provide a complementary binding pair member.

For example, the CD1d molecule may be engineered to contain a site for biotinylation, for example a BirA-dependent site. The antibody or fragment thereof can be linked to avidin either directly or indirectly. Direct linkage is accomplished by making an antibody-avidin fusion protein through genetic engineering as described in, for example, Shin et al., Shin, S.-U. et al., J. Immunol. 158:4797-4804 (1997); and Penichet et al., J. Immunol. 163:4421-4426.

The CD1d molecule may contain some or all of the amino acids from the transmembrane domain. Preferably, not more than about 20, 19, 18, 17, 16, 15, 14, 13, 12, 11, 10, 9, 8, 7, 6, 5, 4, 3, 2 or 1, and preferably none of the amino acids of the transmembrane domain will be included.

Additionally, fragments of β2-microglobulin are useful in the present invention. To be useful in the present invention, the fragment of β₂-microglobulin would have to retain the ability to associate with the CD1d molecule. Preferably, not more than about 20, 19, 18, 17, 16, 15, 14, 13, 12, 11, 10, 9, 8, 7, 6, 5, 4, 3, 2 or 1, and preferably none of the amino acids of β₂-microglobulin will be deleted.

One may wish to introduce a small number of amino acids at the polypeptide termini of either the CD1d molecule or the β₂-microglobulin, usually not more than 20, 19, 18, 17, 16, 15, 14, 13, 12, 11, 10, 9, 8, 7, 6, 5, 4, 3, 2 or 1. The deletion or insertion of amino acids will usually be as a result of the needs of the construction, providing for convenient restriction sites, addition of processing signals, ease of manipulation, improvement in levels of expression, or the like. In addition, one may wish to substitute one or more amino acids with a different amino acid for similar reasons, usually not substituting more than about 10, 9, 8, 7, 6, 5, 4, 3, 2 or 1 amino acids in any one domain.

The CD1d molecule and β2-microglobulin may be separately produced and allowed to associate to form a stable heteroduplex complex, or both of the subunits may be expressed in a single cell.

CD1d and β2-microglobulin molecules useful in the compounds of the present invention may be isolated from a multiplicity of cells, e.g., transformed cell lines JY, BM92, WIN, MOC, and MG, and CHO using a variety of techniques known to those skilled in the art.

Additionally, the amino acid sequences of CD1d and β2-microglobulin are known, and the genes have been cloned, therefore, the proteins can be made using recombinant methods. For example, CD1d molecule is synthesized and the amino termini coding sequence can be arbitrarily chosen to facilitate the ligation of the coding region for an antibody chain or fragment or a binding intermediate. The coding sequence for the CD1d and β₂ microglobulin chains or their fusion products are then inserted into expression vectors, expressed separately in an appropriate host, such as E. *coli,* yeast, insect cells, mammalian cells or other suitable cells, and the recombinant proteins obtained are recombined in the presence of the CD1d lipid, glycolipid (e.g. α-GalCer) or hydrophobic peptide ligand.

Antigens useful within the present invention include any lipid, glycopeptide or peptide which is capable of modulating an immune response in an animal when presented in conjunction with a CD1d molecule. The antigens may be derived from foreign antigens or from autoantigens. Further, the antigens may be synthetic. Particularly preferred as an antigen is αGalCer.

The compounds of the invention may contain a homogenous or heterogeneous population of antigens and/or costimulatory molecules. That is, each CD1d molecule in the compound may be linked to the same antigen or each CD1d molecule may be linked to different antigen. Likewise, the CD1d complex may be linked to the same costimulatory molecule or different CD1d complex β2-microglobulin molecules may be linked to different costimulatory molecules. Alternatively, some of the CD1d molecules may be linked to an antigen, while some of the CD1d molecules may be linked to a costimulatory molecule.

Antibodies are constructed of one, or several, units, each of which consists of two heavy (H) polypeptide chains and two light (L) polypeptide chains. The H and L chains are made up of a series of domains. The L chains, of which there are two major types (κ and λ), consists of two domains. The H chains are of several types, including µ, δ, and γ (of which there are several subclasses), α and ε. In humans, there are eight genetically and structurally identified antibody classes and subclasses as defined by heavy chain isotypes: IgM, IgD, IgG3, IgG1, IgG2, IgG4, IgE, and IgA. Further, for example, "IgG" means an antibody of the G class, and that, "IgG1" refers to an IgG molecules of subclass 1 of the G class. IgG1 antibodies, like all antibodies of the IgG class, are comprised of 4 domains, one of which is variable and the other 3 are constant An Fab antibody fragment is comprised of an intact light chain and a truncated heavy chain that each comprise two domains, one variable and one constant.

As used herein, the term "antibody" (Ab) or "monoclonal antibody" (MAb) is meant to include intact molecules as well as antibody portions (such as, for example, Fab and F(ab')2 portions and Fv fragments) which are capable of specifically binding to a cell surface marker. Such portions are typically produced by proteolytic cleavage, using enzymes such as papain (to produce Fab portions) or pepsin (to produce F(ab')2 portions). Especially preferred in the compounds of the invention are Fab portions. Alternatively, antigen-binding portions can be produced through the application of recombinant DNA technology.

The immunoglobulin may be a "bifunctional" or "hybrid" antibody, that is, an antibody which may have one arm having a specificity for one antigenic site, such as a tumor associated antigen while the other arm recognizes a different target, for example, an immunologically active cytokine or lymphokine, or CD1d. In any case, the hybrid antibodies have a dual specificity, preferably with one or more binding sites specific for an antigen expressed on the surface of a target cell, for example, an antigen associated with a tumor, an infectious organism, or antigenic marker of another disease state.

In addition, the immunoglobin may be a single chain antibody ("SCA"). These may consist of single chain Fv fragments ("scFv") in which the variable light ("V[L]") and variable heavy ("V[H]") domains are linked by a peptide bridge or by disulfide bonds. Also, the immunoglobulin may consist of single V[H]domains (dAbs) which possess antigen-binding activity. See, e.g., G. Winter and C. Milstein, Nature 349:295 (1991); R. Glockshuber et al., Biochemistry 29:1362 (1990); and, E. S. Ward et al., Nature 341:544 (1989).

Also preferred for use in the present invention are chimeric monoclonal antibodies, preferably those chimeric antibodies having specificity toward a tumor associated surface membrane antigen, a surface membrane antigen of a tissue or organ affected by autoimmune disease, or an antigen of a pathogen infected cell. As used in this example, the term "chimeric antibody" refers to a monoclonal antibody comprising a variable region, i.e. binding region, from one source or species and at least a portion of a constant region derived from a different source or species, usually prepared by recombinant DNA techniques. Chimeric antibodies comprising a murine variable region and a human constant region are preferred in certain applications of the invention, particularly human therapy, because such antibodies are readily prepared and may be less immunogenic than purely murine monoclonal antibodies. Such murine/human chimeric antibodies are the product of expressed immunoglobulin genes comprising DNA segments encoding murine immunoglobulin variable regions and DNA segments encoding human immunoglobulin constant regions. Other forms of chimeric antibodies encompassed by the invention are those in which the class or subclass has been modified or changed from that of the original antibody. Such "chimeric" antibodies are also referred to as "class-switched antibodies". Methods for producing chimeric antibodies involve conventional recombinant DNA and gene transfection techniques now well known in the art. See, e.g., Morrison, S. L. et al., Proc. Nat'1 Acad. Sci. 81:6851 (1984).

Encompassed by the term "chimeric antibody" is the concept of "humanized antibody", that is those antibodies in which the framework or "complementarity" determining regions ("CDR") have been modified to comprise the CDR of an immunoglobulin of different specificity as compared to that of the parent immunoglobulin. In a preferred embodiment, a murine CDR is grafted into the framework region of a human antibody to prepare the "humanized antibody". See, e.g., L. Riechmann et al., Nature 332:323 (1988); M. S. Neuberger et al., Nature 314:268 (1985). Particularly preferred CDR'S correspond to those representing sequences recognizing the antigens noted above for the chimeric and bifunctional antibodies. The reader is referred to the teaching of EPA 0 239 400 (published Sep. 30, 1987), for its teaching of CDR modified antibodies. See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).

Most preferably, fully human antibodies or fragments thereof are used in the compounds of the invention, preferably those fully human antibodies having specificity toward a tumor associated surface membrane antigen , a surface membrane antigen of a tissue or organ affected by autoimmune disease, or an antigen of a pathogen infected cell. Methods have been described for selection of fully human antibodies in human immunoglobulin transgenic mice, from libraries of human immunoglobulin genes constructed in phage and expressed in bacteria or constructed in a mammalian viral expression vector for expression in mammalian cells, and from human hybridoma cells. A method for selection of fully human antibodies from libraries of human immunoglobulin genes constructed in vaccinia virus is described in Zauderer, M. et al. WO 01/72995, published 4 October 2001.

One skilled in the art will recognize that a bifunctional-chimeric antibody can be prepared which would have the benefits of lower immunogenicity of the chimeric, humanized or fully human antibody, as well as the flexibility, especially for therapeutic treatment, of the bifunctional antibodies described above. Such bifunctional-chimeric antibodies can be synthesized, for instance, by chemical synthesis using cross-linking agents and/or recombinant methods of the type described above. In any event, the present invention should not be construed as limited in scope by any particular method of production of an antibody whether bifunctional, chimeric, bifunctional-chimeric, humanized, fully human or an antigen-recognizing fragment or derivative thereof.

In addition, the invention encompasses within its scope immunoglobulins (as defined above) or immunoglobulin fragments to which are fused active proteins, for example, an enzyme of the type disclosed in Neuberger et al., PCT application, WO86/0153 published Mar. 13, 1986.

As noted, "bifunctional", "fused", "chimeric" (including humanized), "fully human", and "bifunctional-chimeric" (including humanized) or "bifunctional-fully human" antibody constructions also include, within their individual contexts constructions comprising antigen recognizing fragments. As one skilled in the art will recognize, such fragments could be prepared by traditional enzymatic cleavage of intact bifunctional, chimeric, humanized, fully human or chimeric-bifunctional or fully human-bifunctional antibodies. If, however, intact antibodies are not susceptible to such cleavage, because of the nature of the construction involved, the noted constructions can be prepared with immunoglobulin fragments used as the starting materials; or, if recombinant techniques are used, the DNA sequences, themselves, can be tailored to encode the desired "fragment" which, when expressed, can be combined in vivo or in vitro, by chemical or biological means, to prepare the final desired intact immunoglobulin "fragment". It is in this context, therefore, that the term "fragment" is used.

Furthermore, as noted above, the immunoglobulin (antibody), or fragment thereof, used in the present invention may be polyclonal or monoclonal in nature. Monoclonal antibodies are the preferred immunoglobulins, however. The preparation of such polyclonal or monoclonal antibodies now is well known to those skilled in the art who, of course, are fully capable of producing useful immunoglobulins which can be used in the invention. See, e.g., G. Kohler and C. Milstein, Nature 256:495 (1975). Kohler et al., Nature 256:495 (1975); Kohler et al., Eur. J. Immunol. 6:511 (1976); Kohler et al., Eur. J. Immunol. 6:292 (1976); Hammerling et al., In: Monoclonal Antibodies and T-Cell Hybridomas, Elsevier, N.Y., pp. 563-681 (1981)). In general, such procedures involve immunizing an animal (preferably a mouse) with a protein antigen or, more preferably, with a protein-expressing cell. Suitable cells can be recognized by their capacity to bind antibody. Such cells may be cultured in any suitable tissue culture medium; however, it is preferable to culture cells in Excell hybridoma medium (JRH Biosciences, Lenexa, KS) with 5% fetal bovine serum. The splenocytes of such immunized mice are extracted and fused with a suitable myeloma cell line. Any suitable myeloma cell line may be employed in accordance with the present invention; however, it is preferable to employ the parent myeloma cell line (SP2O), available from the American Type Culture Collection, 10801 University Boulevard, Manassas, Virginia 20110-2209. After fusion, the resulting hybridoma cells are selectively maintained in HAT medium, and then cloned by limiting dilution as described by Wands et al., Gastroenterology 80:225-232 (1981). The hybridoma cells obtained through such a selection are then assayed to identify clones which secrete antibodies capable of binding the antigen.

In addition, hybridomas and/or monoclonal antibodies which are produced by such hybridomas and which are useful in the practice of the present invention are publicly available from sources such as the American Type Culture Collection ("ATCC") 10801 University Boulevard, Manassas, Virginia 20110-2209 or, commercially, for example, from Boehringer-Mannheim Biochemicals, P.O. Box 50816, Indianapolis, Ind. 46250.

The antibodies of the present invention may be prepared by any of a variety of methods. For example, cells expressing the cell surface marker or an antigenic portion thereof can be administered to an animal in order to induce the production of sera containing polyclonal antibodies. In a preferred method, a preparation of protein is prepared and purified as to render it substantially free of natural contaminants. Such a preparation is then introduced into an animal in order to produce polyclonal antisera of greater specific activity.

In certain embodiments, the compounds of the present invention comprise, instead of, or in addition to an antibody, a receptor or ligand that has a matching or counterpart ligand or receptor expressed on a cell surface of a target cell. In these embodiments, the compound comprises one or more CD1d complexes and a ligand or receptor specific for a cell surface marker. Examples include: CD4 coupled to CD1d for interaction with HIV infected cells; chemokine or chemokine receptor coupled to CD1d for interaction with DC subset; or heregulins coupled to CD1d for interaction with ErbB2 positive tumor cells.

In one embodiment, the antibody is specific for a cell surface marker of a tumor cell. CD1d-NKT can be directly cytolytic to tumor cells and can also recruit either directly or through activation of DC both NK cells and cytolytic T cells. The present invention allows CD1d loaded with a stimulatory ligand such as α-GalCer to be targeted to tumors by coupling the complex to an antibody specific for a tumor cell surface antigen. The CD1d complex concentrated on tumor cells will then recruit and activate CD1d-NKT cells and trigger a cascade of events that promote tumor cell-killing and growth inhibition.

In a preferred embodiment of the use of the present invention said cancer is a head and neck cancer, gastric cancer, esophageal cancer, stomach cancer, colorectal cancer, coloncarcinoma, cancer of liver and intrahepatic bile ducts, pancreatic cancer, lung cancer, small cell lung cancer, cancer of the larynx, breast cancer, malignant melanoma, multiple myeloma, sarcomas, rhabdomyosarcoma, lymphomas, folicular non-Hodgkin-lymphoma, leukemias, T- and B-cell-leukemias, Hodgkin-lymphoma, B-cell lymphoma, ovarian cancer, cancer of the uterus, cervical cancer, prostate cancer, genital cancer, renal cancer, cancer of the testis, thyroid cancer, bladder cancer, plasmacytoma or brain cancer.

Tumor associated antigens comprise pan-carcinoma antigens like CEA (Sundblad Hum. Pathol. 27, (1996) 297-301, Ilantzis Lab. Invest. 76(1997), 703-16), EGFR type I (Nouri, Int. J. Mol. Med. 6 (2000), 495-500) and EpCAM (17-1A/KSA/GA733-2, Balzar J. Mol. Med. 77 (1999), 699-712). EGFR type I is especially overexpressed in glioma and EpCAM in colon carcinoma. EGFR type II (Her-2/neu, ERBB2 Sugano Int. J. Cancer 89 (2000), 329-36) and TAG-72 glycoprotein (sTN antigen, Kathan Arch. Pathol. Lab. Med. 124 (2000), 234-9) are upregulated in breast cancer. EGFR deletion neoepitope might also play a role as tumor associated antigen (Sampson Proc. Nati. Acad. Sci. U S A 97 (2000), 7503-8). The antigens A33 (Ritter Biochem. Biophys. Res. Commun. 236 (1997), 682-6), Lewis-Y (DiCarlo Oncol. Rep. 8 (2001), 387-92), Cora Antigen (CEA-related Cell Adhesion Molecule CEACAM 6, CD66c, NCA-90, Kinugasa Int. J. Cancer 76 (1998), 148-53) and MUC-1 (Mucin) are associated with colon carcinoma (lida Oncol. Res. 10 (1998), 407-14). Thomsen-Friedenreich-antigen (TF, Gal1B-3GaINAca1-0-Thr/Ser) is not only found in colon carcinoma (Baldus Cancer 82 (1998), 1019-27) but also in breast cancer (Glinsky Cancer. Res. 60 (2000), 2584-8). Overexpression of Ly-6 (Eshel J. Biol. Chem. 275 (2000), 12833-40) and desmoglein 4 in head and neck cancer and of E-cadherin neoepitope in gastric carcinoma has been described (Fukudome Int. J. Cancer 88 (2000), 579-83). Prostate-specific membrane antigen (PSMA, Lapidus Prostate 45 (2000), 350-4), prostate stem cell antigen (PSCA, Gu Oncogene 191 (2000) 288-96) and STEAP (Hubert, Proc Natl Acad Sci U S A 96 (1999), 14523-8) are associated with prostate cancer. The alpha and gamma subunit of the fetal type acetylcholine receptor (AChR) are specific immunohistochemical markers for rhabdomyosarcoma (RMS, Gattenlohner Diagn. Mol. Pathol. 3 (1998), 129-34).

Association of CD20 with follicular non-Hodgkin lymphoma (Yatabe Blood 95 (2000), 2253-61, Vose Oncology (Huntingt) 2 (2001) 141-7), of CD19 with B-cell lymphoma (Kroft Am. J. Clin. Pathol. 115 (2001), 385-95), of Wue-1 plasma cell antigen with multiple myeloma (Greiner Virchows Arch 437 (2000), 372-9), of CD22 with B cell leukemia (dArena Am. J. Hematol. 64 (2000), 275-81), of CD7 with T-cell leukemia (Porwit-MacDonald Leukemia 14 (2000), 816-25) and CD25 with certain T and B cell leukemias has been described (Wu Arch. Pathol. Lab. Med. 124 (2000), 1710-3). CD30 is associated with Hodgkin-lymphoma (Mir Blood 96 (2000), 4307-12). Expression of melanoma chondroitin sulfate proteoglycan (MCSP, Eisenmann Nat. Cell. Biol. 8 (1999), 507-13) and gangiloside GD3 is observed in melanoma (Welte Exp Dermatol 2 (1997), 64-9), while GD3 is also found in small cell lung cancer (SCLC, Brezicka Lung Cancer 1 (2000), 29-36). Expression of gangiloside GD2 is, also upregulated in SCLC and in neuroblastoma (Cheresh et al. Cancer Res. 10 (1986), 5112-8). Ovarian carcinoma is associated with Muellerian Inhibitory Substance (MIS) receptor type II (Masiakos Clin. Cancer Res. 11 (1999), 3488-99) and renal as well as cervical carcinoma with expression of carboanhydrase 9 (MN/CAIX, Grabmaier Int. J. Cancer 85 (2000) 865-70). Elevated expression levels of CA 19-9 were found in pancreatic cancer (Nazli Hepatogastroenterology 47 (2000), 1750-2).

Other examples of tumor cell surface antigens are Her2/neu, expressed in breast and ovarian carcinomas (Zhang, H. et al., Experimental & Molecular Pathology 67:15-25 (1999)); CM-1, expressed in breast cancer (Chen, L. et al., Acta Academiae Medicinae Sinicae 19(2):150-3); 28K2, expressed in Lung adenocarcinoma and large cell carcinoma (Yoshinari, K. et al., Lung Cancer 25:95-103 (1999)); E48 and U 36 expressed in head and neck squamous cell carcinoma (Van Dongen, G.A.M.S. et al., Anticancer Res. 16:2409-14 (1996)); NY-ESO-1, expressed in esophageal carcinoma, and melanoma Jager, E. et al., J. Exp. Med. 187:265-70 (1998); Jager, E. et al., International J. Cancer 84:506-10 (1999)); KU-BL 1-5, expressed in bladder carcinoma (Ito, K. et al., AUA 2000 Annual Meeting, Abstract 3291 (2000)); NY CO 1-48, expressed in colon carcinoma (Scanlan, M.J. et al., International J. Cancer 76:652-8 (1998)); HOM MEL 40, expressed in melanoma (Tureci, O. et al., Cancer Res. 56:4766-72 (1996)); OV569, expressed in ovarian carcinoma (Scholler, N. et al., Proc. Natl. Acad, Sci. USA 96:11531-6 (1999)); ChCE7, expressed in neuroblastoma and renal cell carcinoma (Meli, M.L. et al., International J. Cancer 83:401-8 (1999)); CA19-9, expressed in colon carcinoma (Han, J.S. et al., Cancer 76:195-200 (1995)); CA125, expressed in ovarian carcinoma (O'Brien, T.J. et al., International J. Biological Markers 13:188-95 (1998)); and Gangliosides (GM2, GD2, 9-o-acetyl-GD3, GD3), expressed in melanoma and neuroblastoma (Zhang, S. et al., Cancer Immunol. Immunotherapy 40:88-94 (1995)).

In a most preferred embodiment of the method of the present invention said tumor-associated antigen is selected from the group consisting of Lewis Y, CEA, Muc-1, erbB-2, -3 and -4, Ep-CAM, E-cadherin neoepitope, EGF-raceptor (e.g. EGFR type I or EGFR type II), EGFR deletion neoepitope, CA19-9, Muc-1, LeY, TF-, Tn- and sTn-antigen, TAG-72, PSMA, STEAP, Cora antigen, CD7, CD19 and CD20, CD22, CD25, Ig-a and Ig-B, A33 and G250, CD30, MCSP and gp100, CD44-v6, MT-MMPS, (MIS) receptor type II, carboanhydrase 9, F19-antigen, Ly6, desmoglein 4, PSCA, Wue-1, GD2 and GD3 as well as TM4SF-antigens (CD63, L6, CO-29, SAS), the alpha and gamma subunit of the fetal type acetylcholinreceptor (AChR), CM-1, 28K2, E48, U36, NY-ESO-1, KU-BL 1-5, NY CO 1-48, HOM MEL 40, OV569, ChCE7, CA19-9, CA125, GM2, GD2, 9-o-acetyl-GD3, or GD3.

In another embodiment, the antibody is specific for a cell surface marker of a CD1d-restricted NKT cells. In this embodiment, an inhibitory signal can be delivered to the NKT cells through their CD1d-restricted TCR , which can result in systemic downregulation of CD1d-NKT cell activity. Even in the absence of a linked inhibitory signal, just direct binding of CD1d:α-GalCer complexes to NKT cells has been reported to induce apoptosis of the NKT (Matsuda, J.L. et al. J. Exp. Med. 192:741-754,2000). Suitable NKT cell markers to which to target specific antibodies are CD161, CD56, or (for NKT subsets) CCR4 on CD4+ CD1d-NKT or CCR1 or CCR6 on double negative CD1d-NKT. This is particularly useful in treating diseases or symptoms which are the result of high NKT activity. These include myasthenia gravis (Reinhardt et al. Neurology 52:1485-87,1999), psoriasis (Bonish, J. Immunol. 165:4076-85,2000), ulcerative colitis (Saubermann et al. Gastroenterology 119:119-128, 2000), and primary biliary cirrhosis (Kita et al. Gastroenterology 123:1031-43,2002). In these embodiments, localized downregulation of CD1d-NKT may ameliorate disease.

In another embodiment, the antibody is specific for a cell surface marker of a target tissue of autoimmune disease or inflammatory response. CD1d-NKT stimulated to secrete IL-4 by normal antigen presenting cells and DC at the site of an autoimmune lesion or inflammatory site can regulate the development and expression of aggressive cell-mediated immune responses. For those autoimmune or inflammatory diseases whose etiology is related to a paucity of CD1d-NKT in a specific tissue, this defect can be ameliorated by allowing CD1d loaded with a stimulatory ligand such as α-GalCer to be targeted to that tissue. In a preferred embodiment, CD1d:α-GalCer is targeted to such sites by coupling the complex to an antibody specific for a local tissue antigen. The CD1d complex concentrated on local tissue cells will then lead to recruitment and activation of CD1d-NKT cells and trigger a cascade of events that regulate the autoimmune or inflammatory response. The relevant specific tissue antigens may be different for different autoimmune and inflammatory diseases.

Alternatively, in this embodiment, the CD1d complexes comprise an antigen (for example, a lipid, glycolipid, or hydrophobin peptide) with antagonist properties (Miyamoto Nature 413:531-534, 2001). This embodiment of the invention is of particular relevance to those autoimmune diseases that appear to result from excessive CD1d-NKT activity. These include myasthenia gravis (Reinhardt et al. Neurology 52:1485-87,1999), psoriasis (Bonish , J. Immunol. 165:4076-85,2000), ulcerative colitis (Saubermann et al. Gastroenterology 119:119-128, 2000), and primary biliary cirrhosis (Kita et al. Gastroenterology 123:1031-43,2002). In these embodiments, localized downregulation of CD1d-NKT may ameliorate disease.

In the case of demyelinating diseases including, most especially, multiple sclerosis, the antibody is specific for MBP (myelin basic protein), PLP (myelin proteolipid protein), or MOG (myelin oligodendrocyte glycoprotein). In a most preferred embodiment, the antibody is specific for MOG.

In the case of juvenile onset type I diabetes which follows from destruction of insulin producing pancreatic islet beta cells, the antibody is specific for target antigens of the islet beta cells such as GT3 ganglioside, IGRP (islet-specific glucose-6-phosphatase), or SUR1 (Proks P et al., Diabetes:51 Suppl 3:S368-76, 2002). Recently, peri-islet Schwann cells have been described as an early target of autoimmune destruction in this disease (Winer S et al: Nature Medicine 9:198-205, 2003). In another preferred embodiment of the invention, therefore, the antibody is specific for Schwann cell specific antigens glial fibrillary acidic protein (GFAP), and S100beta for treatment or diagnosis of juvenile onset type I diabetes.

In other embodiments for treatment of autoimmune and inflammatory diseases, conjugates comprising CD1d complexes and antibodies specific for type II collagen are injected into affected joints for treatment of rheumatoid arthritis; antibodies specific for thyroglobulin or TSH receptor are targeted to the thyroid for treatment of Hashimoto's thyroiditis and Graves' disease; and antibodies specific for the K+/H+ ATPase are employed for treatment of pernicious anemia or atrophic gastritis (targeting the gastric parietal cells).

In other embodiments, the conjugate is employed to treat other autoimmune conditions: ankylosing spondylitis, acute anterior uveitis, Goodpasture's syndrome, Myasthenia gravis, Systemic Lupus Erythematosus, systemic sclerosis, Pemphigus vulgaris, or autoimmune Hepatitis. As discussed below, in some manifestations of autoimmune or inflammatory disease there is a surfeit rather than a paucity of CD1d-NKT cells or an excess of one subset of CD1d-NKT relative to another e.g. CD4+ CD1d-NKT relative to double negative (CD4-/CD8-) CD1d-NKT. Conjugates of the invention described here that can result in inhibition rather than activation of CD1d-NKT or that, by targeting different DC subsets, can activate one CH1d-NKT cell subset in preference to another.

In other embodiments, the antibody is specific for a cell surface marker of an infected cell or tissue. CD1d-NKT have been shown to have an important role in resistance to malaria, fungal and hepatitis B virus infections [12-14]. In a preferred embodiment, CD1d loaded with a stimulatory ligand such as α-GalCer is targeted to the site of infection by coupling the complex to an antibody specific for an antigen encoded by the infectious agent. The CD1d complex concentrated on infected cells will then lead to recruitment and activation of CD1d-NKT cells and trigger a cascade of events that lead to elimination or inhibition of growth of the infectious agent. The relevant antigens are different in the case of different infectious agents. In a preferred embodiment of the method of the present invention said surface marker for an infected cell is selected from the group consisting of viral envelope antigens, e.g. of human retroviruses (HTLV I and II, HIV1 and 2) or human herpes viruses (HSV1 and 2, CMV, EBV), haemagglutinin e.g. of influenza virus (influenza A, B or C), glycoproteins E1 and E2 from rubella virus or RGP of rabies virus.

In other embodiments, the antibody is targeted to antigens encoded by other infectious viruses, bacteria, fungi, protozoa or helminthes.

In other embodiments, the antibody is specific for a cell surface marker of a target of allogenicity. CD1d-NKT have been reported to play a major role in blocking graft vs. host disease following allogeneic bone marrow transplantation (Zeng D. et al. J. Exp. Med. 189:1073-81,1999) and in maintenance of tolerance to allograft transplants (Seino KI et al. Proc. Natl. Acad. Sci. USA 98:2577-81,2001). An interesting feature of CD1d-NKT mediated allograft tolerance is that it is reported to depend on production of IFN-γ but not IL-4 by CD1d-NKT.

In a embodiment, CD1d loaded with a stimulatory ligand such as α-GalCer is targeted to an allograft by coupling the complex to an antibody specific for a major or minor histocompatibility antigen of the allogeneic bone marrow or organ graft. The CD1d complex concentrated on cells of the allograft will then lead to recruitment and activation of CD1d-NKT cells. Examples of histocompatibility antigens include HLA specificities such as A (e.g.. A1-A74), B (e.g., B1-B77), C (e.g., C1--C11), D (e.g., D1-D26), DR (e.g., DR1-DR8), DQ (e.g., DQ1-DQ9) and DP (e.g. DP1-DP6). More preferably, HLA specificities include A1, A2, A3, A11, A23, A24, A28, A30, A33, B7, B8, B35, B44, B53, B60, B62, DR1, DR2, DR3, DR4, DR7, DR8, and DR 11. It is possible to tissue type a person by serological or genetic analysis to define which MHC class I or II molecule variants each person has using methods known in the art.

In one embodiment, the antibody is specific for a cell surface marker of a professional antigen presenting cell. Preferably, the antibody is specific for a cell surface marker of a dendritic cell, for example, CD83, DEC205, CMRF-44 (Fearnley DB et al. Blood 89:3708-16,1997), CMRF-56 (Hock BD et al. Tissue Antigens 53:320-34,1999), BDCA-1, BDCA-2, BDCA-3, and BDCA-4 (Dzionek A et al. J. Immunol. 165:6037-6046,2000). In other embodiments, the antibody is specific for markers of antigen presenting cells including Toll-like receptors (TLR1, TLR2, TLR3, TLR4, TLR5, TLR7, TLR9) mannose receptor, and mannan-binding lectin (MBL). As well as additional markers specific to dendritic cells, including, DC-SIGN (the C-type lectin, non-integrin, ICAM-3 receptor on DC), ALCAM, DC-LAMP, and any of a number of other receptors for apoptotic cells including phosphatidylserine receptor. The antibody may be specific for a cell surface marker of another professional antigen presenting cell, such as a B cell or a macrophage. CD 19, CD20 and CD22 are expressed on B cells, and other markers have been described for other antigen presenting cells.

In other embodiments, the antibody is specific for a cell surface marker of a dendritic cell subset. As noted above, whether CD1d-NKT give rise to a predominantly pro-inflammatory or anti-inflammatory response is, in part, a function of the relative frequency of two CD1d-NKT subsets. CD1d-NKT that are negative for expression of both CD4 and CD8 (double negative) and express the chemokine receptors CCR1 and CCR6 give rise to predominantly a pro-inflammatory TH1 type response including secretion of IFN-γ and TNF-a. In contrast, CD1d-NKT that are CD4+ and express the chemokine receptor CCR4 give rise to both a TH1 type and TH2 type response including secretion of the anti-inflammatory Th2-type cytokines IL-4, IL-5, IL-10 and IL-13. The activation of different CD1d-NKT subsets and their radiating influence in promulgating pro-inflammatory or anti-inflammatory responses is mediated by their interaction with different DC subsets (Wilson SB and Delovitch TL Nature Rev. Immunol. 3:211-222,2003; Vincent MS et al. Nature Immunol. 3:1163-68,2002).

Human DC have been broadly distinguished as myeloid and plasmacytoid. Myeloid DC are characterized by a monocytic morphology, express myeloid markers including the β2 integrin CD11c, and produce high levels of IL-12. Plasmacytoid DC, in contrast, have a morphology that is plama cell like, are CD11c negative, and produce high levels of IFN-α. Based on their ability to induce, under appropriate conditions, predominantly TH1 or TH2 type responses, mature myeloid DC have been designated as DC1 and mature plasmacytoid DC as DC2 (Rissoan MC et al. Science 283:1183, 1999). There is, however, a considerable degree of plasticity in the function of DC subsets that is influenced by the antigen they present, particularly antigens of microbial origin that match pattern recognition receptors on DC, by the cytokine environment in which they differentiate from earlier precursors, and by the properties of the T cells with which they interact (Liu YJ et al. Nature Immunol. 2:585-589,2001; Pulendran B et al. Science 293:253-256,2001; Banchereau J et al. Ann. Rev. Immunol. 18:767-811,2000). Cross-talk between T cells and DC has emerged as an important factor in the maturation, polarization and survival of both T cells and DC (Shreedhar V et al. Immunity 11:625-636,1999) including interactions between CD1d expressing DC and CD1d-restricted NKT (Wilson SB and Delovitch TL Nature Rev. Immunol. 3:211-222,2003; Vincent MS et al. Nature Immunol. 3:1163-68,2002).

In a embodiment CD1d loaded with a stimulatory ligand such as α-GalCer is targeted to DC by coupling the complex to an antibody specific for a surface antigen marker of DC such as CD83, DEC205, CMRF-44, CMRF-56, DC-SIGN, Toll-like Receptors (TLR) including TLR1, TLR2, TLR3, TLR4, TLR5, TLR7, TLR9, mannose receptor, mannan-binding lectin (MBL), ALCAM, DC-LAMP, phosphatidylserine receptor, BDCA-1, BDCA-2, BDCA-3 or BDCA-4 (neuropilin, Tordjman R et al. Nature Immunol. 3:477-82,2002). The CD1d complex concentrated on DC will then lead to recruitment and activation of CD1d-NKT cells.

In a embodiment CD1d loaded with a stimulatory ligand such as α-GalCer is targeted to a particular DC subset by coupling the complex to an antibody specific for a surface antigen marker of said DC subset such as TLR2, TLR4, TLR7, TLR9, BDCA-1, BDCA-2, BDCA-3, and BDCA-4 (Dzionek A et al. J. Immunol. 165:6037-46,2000; Liu YJ et al. Nature Immunol. 2:585-589,2001; Penna G et al. J. Immunol. 167:1862-66,2001) or mannose receptor. TLR2, TLR4, BDCA-1 (CD1c), BDCA-3, and mannose receptor are expressed on subsets of myeloid DC while TLR7, TLR9, BDCA-2 and BDCA-4 are expressed on plasmacytoid DC. The CD1d complex concentrated on the DC subset will then lead to recruitment and activation of a matched subset of CD1d-NKT cells. This embodiment offers a focused means of directing polarization of immune responses to aggressive cell-mediated immunity or to potential tolerogenic interactions. The former is appropriate for inducing immunity to tumors and the latter for downregulation of autoimmune responses that are associated, for example, with multiple sclerosis or type I diabetes.

The strategy of targeting CD1d complexes to DC subsets can be employed either alone or in combination with targeting CD1d complexes to specific tissues or organs.

The compounds of the present invention may be labeled, so as to be directly detectable, or will be used in conjunction with secondary labeled immunoreagents which will specifically bind the compound for example, for detection or diagnostic purposes. Labels of interest may include dyes, enzymes, chemiluminescers, particles, radioisotopes, or other directly or indirectly detectable agent. Alternatively, a second stage label may be used, e.g. labeled antibody directed to one of the constituents of the compound of the invention.

Examples of suitable enzyme labels include malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholine esterase.

Examples of suitable radioisotopic labels include 3H, 111In, 125I, 131I, 32P, 35S, 14C, 51Cr, 57To, 58Co, 59Fe, 75Se, 152Eu, 90Y, 67Cu, 217Ci, 211At, 212Pb, 47Sc, 109Pd, etc. 111In is a preferred isotope where in vivo imaging is used since its avoids the problem of dehalogenation of the 125I or 131I-labeled monoclonal antibody by the liver. In addition, this radio nucleotide has a more favorable gamma emission energy for imaging (Perkins et al., Eur. J. Nucl. Med. 10:296-301 (1985); Carasquillo et al., J. Nucl. Med. 28:281-287 (1987)). For example, 111In coupled to monoclonal antibodies with 1-(P-isothiocyanatobenzyl)-DPTA has shown little uptake in non-tumorous tissues, particularly the liver, and therefore enhances specificity of tumor localization (Esteban et al., J. Nucl. Med. 28:861-870 (1987)).

Examples of suitable non-radioactive isotopic labels include 157Gd, 55Mn, 162Dy, 52Tr, and 56Fe.

Examples of suitable fluorescent labels include an 152Eu label, a fluorescein label, an isothiocyanate label, a rhodamine label, a phycoerythrin label, a phycocyanin label, an allophycocyanin label, an o-phthaldehyde label, and a fluorescamine label.

Examples of suitable toxin labels include diphtheria toxin, ricin, and cholera toxin.

Examples of chemiluminescent labels include a luminal label, an isoluminal label, an aromatic acridinium ester label, an imidazole label, an acridinium salt label, an oxalate ester label, a luciferin label, a luciferase label, and an aequorin label.

Examples of nuclear magnetic resonance contrasting agents include heavy metal nuclei such as Gd, Mn, and Fe.

Typical techniques for binding the above-described labels to antibodies are provided by Kennedy et al., Clin. Chim. Acta 70:1-31 (1976), and Schurs et al., Clin. Chim. Acta 81:1-40 (1977). Coupling techniques mentioned in the latter are the glutaraldehyde method, the periodate method, the dimaleimide method, the m-maleimidobenzyl-N-hydroxy-succinimide ester method,

The compound of the invention may further comprise other therapeutic agents. The therapeutic agent or agents may be linked to the multivalent compound, the antibody, or the CD1d complex. Examples of therapeutic agents include, but are not limited to, antimetabolites, alkylating agents, anthracyclines, antibiotics, and anti-mitotic agents. Antimetabolites include methotrexate, 6-mercaptopurine, 6-thioguanine, cytarabine, 5-fluorouracil decarbazine. Alkylating agents include mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin. Anthracyclines include daunorubicin (formerly daunomycin) and doxorubicin (also referred to herein as adriamycin). Additional examples include mitozantrone and bisantrene. Antibiotics include dactinomycin (formerly actinomycin), bleomycin, mithramycin, and anthramycin (AMC). Antimytotic agents include vincristine and vinblastine (which are commonly referred to as vinca alkaloids). Other cytotoxic agents include procarbazine, hydroxyurea, asparaginase, corticosteroids, mytotane (O,P'-(DDD)), interferons. Further examples of cytotoxic agents include, but are not limited to, ricin, doxorubicin, taxol, cytochalasin B, gramicidin D, ethidium bromide, etoposide, tenoposide, colchicin, dihydroxy anthracin dione, 1-dehydrotestosterone, and glucocorticoid.

Analogs and homologs of such therapeutic and cytotoxic agents are encompassed by the present invention. For example, the chemotherapuetic agent aminopterin has a correlative improved analog namely methotrexate. Further, the improved analog of doxorubicin is an Fe-chelate. Also, the improved analog for 1-methylnitrosourea is lomustine. Further, the improved analog of vinblastine is vincristine. Also, the improved analog of mechlorethamine is cyclophosphamide.

The present invention also relates to vectors which include a nucleotide sequence encoding a compound of the present invention or parts thereof, host cells which are genetically engineered with the recombinant vectors, and the production of the compounds of the present invention or parts thereof by recombinant techniques.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The DNA insert should be operatively linked to an appropriate promoter, such as the phage lambda PL promoter, the E. coli lac, trp and tac promoters, or, in mammalian cells, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase or neomycin resistance for eukaryotic cell culture and tetracycline or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS and Bowes melanoma cells; and plant cells. Appropriate culture mediums and conditions for the above-described host cells are known in the art.

Several eukaryotic expression systems have been used for production of soluble CD1d complex, such as SC2 Drosophila melanogaster cells (Benlagha et al., 2000. J Exp Med 191:1895), baculovirus system (61) and Chinese hamster ovary CHO cells (Gumperz et al., 2002. J Exp Med 195:625). Regarding fusion proteins, a single chain composed of β2 microglobulin fused to N-terminus of CD1d heavy chain fused to the Fc part of IgG2a has been successfully produced in CHO cells both for human and mouse CD1d (Gumperz et al., 2002. J Exp Med 195:625, Gumperz et al., 2000. Immunity 12:211). The Fc portion of IgG2a can be replaced with the variable portion of the desired anti-TAA mAb, possibly a single chain scFv antibody fragment.

In parallel, a chemically coupled CD1d-antibody Fab fragment can be obtained essentially as done for MHC Class I-antibody conjugates (14) except that the CD1d-β2 microglobulin complex is produced in a eukaryotic system. Briefly, a free cysteine will be engineered at the carboxyl terminus of the CD1d molecule and after expression and purification, the CD1d complex is chemically coupled to the free cysteines of the Fab fragment via a thiol reactive bis-maleimide linker. For both the fusion and the conjugate strategy, α-GalCer can be loaded on CD1d either at the time of cell-based synthesis or after production and purification of the soluble CD1d complex.

Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods In Molecular Biology (1986).

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected and purified in the advantageous manner described. This is the case when the Fc portion proves to be a hindrance to use in therapy and diagnosis, for example when the fusion protein is to be used as an antigen for immunizations. In drug discovery, for example, human proteins, such as the hIL5-receptor, have been fused with Fc portions for the purpose of high-throughput screening assays to identify antagonists of hIL-5. See, D. Bennett et al., J. Mol. Recognition 8:52-58 (1995) and K. Johanson et al., J. of Biol. Chem. 270(16):9459-9471 (1995).

Several reports have described secretion and assembly of fusion proteins comprised of diverse sequences linked to the carboxyl terminus of immunoglobulin chains (Harvill, E.T. et al., J. Immunol. 157:3165-70 (1996); Shin, S.U. et al., J. Immunology 158: 4797-4804 (1997); Penichet, M.L. et al., J. Immunol. 163:4421-26 (1999); Zhang, H.F. et al., J. Clin. Invest 103:55-61 (1999)). Fusion proteins of the compounds of this invention will likewise retain amino terminal sequences of the immunoglobulin chain that direct secretion. CD1d molecules linked to the carboxyl terminus of the immunoglobulin chains are stripped of hydrophobic transmembrane sequences and should not interfere with secretion.

The polypeptide can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides useful in the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

The ability of a compound of the present invention to modulate an immune response can be readily determined by an in vitro assay. NKT cells for use in the assays include transformed NKT cell lines, or NKT cells which are isolated from a mammal, e.g., from a human or from a rodent such as a mouse. NKT cells can be isolated from a mammal by sorting cells that bind CD1d:α-GalCer tetramers. See, for example, Benlagha et al., J Exp Med 191 (2000), pp. 1895-1903; Matsuda et al., J Exp Med 192 (2000), pp. 741-754; and Karadimitris et al., Proc Natl Acad Sci USA 98 (2001), pp. 3294-3298. A suitable assay to determine if a compound of the present invention is capable of modulating the activity of NKT cells is conducted by coculturing NKT cells and antigen presenting cells, adding the particular compound of interest to the culture medium that targets either the antigen presenting cells or the NKT cells directly, and measuring IL-4 or IFN-γ production. A significant increase or decrease in IL-4 or IFN-γ production over the same co-culture of cells in the absence of the compound of the invention or, preferably, in the presence of a compound of the invention with a non-targeting antibody indicates stimulation or inhibition ofNKT cells.

The NKT cells employed in the assays are incubated under conditions suitable for proliferation. For example, an NKT cell hybridoma is suitably incubated at about 37°C and 5% CO2 in complete culture medium (RPMI 1640 supplemented with 10% FBS, penicillin/streptomycin, L-glutamine and 5x10-5 M 2-mercaptoethanol). Serial dilutions of the compound can be added to the NKT cell culture medium. Suitable concentrations of the compound added to the NKT cells typically will be in the range of from 10⁻¹² to 10⁻⁶ M. Use of antigen dose and APC numbers giving slightly submaximal NKT cell activation is preferred to detect stimulation or inhibition of NKT cell responses by the compounds of the invention.

Alternatively, rather than measurement of an expressed protein such as IL-4 or IFN-γ, modulation of NKT cell activation can be determined by changes in antigen-dependent T cell proliferation as measured by radiolabelling techniques as are recognized in the art. For example, a labeled (e.g., tritiated) nucleotide may be introduced to an assay culture medium. Incorporation of such a tagged nucleotide into DNA serves as a measure of T cell proliferation. This assay is not suitable for NKT cells that do not require antigen presentation for growth, e.g., NKT cell hybridomas. A difference in the level of T cell proliferation following contact with the compound of the invention indicates the complex modulates activity of the T cells. For example, a decrease in NKT cell proliferation indicates the compound can suppress an immune response. An increase in NKT cell proliferation indicates the compound can stimulate an immune response.

Additionally, the ⁵¹Cr release assay, described below, can be used to determine cytotoxic activity.

These in vitro assays can be employed to select and identify CD1d complexes that are capable of modulating an immune response. Assays described above, e.g., measurement of IL-4 or IFN-γ production or NKT cell proliferation, are employed to determine if contact with the compound modulates T cell activation.

In vivo assays also may be suitably employed to determine the ability of a compound of the invention to modulate the activity of NKT cells. For example, a compound of interest can be assayed for its ability to stimulate NKT cell activation or inhibit tumor growth. For example, a compound of the invention can be administered to a mammal such as a mouse, before or after challenge with a tumorigenic dose of transformed cells and the presence or size of growing tumors may be monitored.

The present invention also includes pharmaceutical compositions comprising a compound described above in combination with a suitable pharmaceutical carrier. Such compositions comprise a therapeutically effective amount of the compound and a pharmaceutically acceptable carrier or excipient. Such a carrier includes but is not limited to saline, buffered saline, dextrose, water, glycerol, ethanol, and combinations thereof. The formulation should suit the mode of administration.

The present invention also includes a method of modulating, i.e., either stimulating or inhibiting an immune response, comprising administering to an animal an effective amount of a compound or composition of the invention.

The compounds of the present invention may be administered in pharmaceutical compositions in combination with one or more pharmaceutically acceptable excipients. It will be understood that, when administered to a human patient, the total daily usage of the pharmaceutical compositions of the present invention will be decided by the attending physician within the scope of sound medical judgment. The specific therapeutically effective dose level for any particular patient will depend upon a variety of factors including the type and degree of the response to be achieved; the specific composition of another agent, if any, employed; the age, body weight, general health, sex and diet of the patient; the time of administration, route of administration, and rate of excretion of the composition; the duration of the treatment; drugs (such as a chemotherapeutic agent) used in combination or coincidental with the specific composition; and like factors well known in the medical arts. Suitable formulations, known in the art, can be found in Remington's Pharmaceutical Sciences (latest edition), Mack Publishing Company, Easton, PA.

The compound to be used in the therapy will be formulated and dosed in a fashion consistent with good medical practice, taking into account the clinical condition of the individual patient (especially the side effects of treatment with the compounds alone), the site of delivery of the compound, the method of administration, the scheduling of administration, and other factors known to practitioners. The "effective amount" of the compounds of the invention for purposes herein is thus determined by such considerations.

Pharmaceutical compositions of the invention may be administered orally, intravenously, rectally, parenterally, intracistemally, intradermally, intravaginally, intraperitoneally, topically (as by powders, ointments, gels, creams, drops or transdermal patch), bucally, or as an oral or nasal spray. The term "parenteral" as used herein refers to modes of administration which include intravenous, intramuscular, intraperitoneal, intrasternal, subcutaneous and intraarticular injection and infusion.

The pharmaceutical compositions are administered in an amount which is effective for treating and/or prophylaxis of the specific indication. In most cases, the dosage is from about 1 µg/kg to about 30 mg/kg body weight daily, taking into account the routes of administration, symptoms, etc. However, the dosage can be as low as 0.001 µg/kg.

As a general proposition, the total pharmaceutically effective amount of the compositions administered parenterally per dose will be in the range of about 1 µg/kg/day to 100 mg/kg/day of patient body weight, although, as noted above, this will be subject to therapeutic discretion. If given continuously, the composition is typically administered at a dose rate of about 1 µg/kg/hour to about 5 mg/kg/hour, either by 1-4 injections per day or by continuous subcutaneous infusions, for example, using a mini-pump. An intravenous bag solution or bottle solution may also be employed.

The compounds of the invention may also be suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (U. Sidman et al., Biopolymers 22:547-556(1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D- (-)-3-hydroxybutyric acid (EP 133,988). Sustained-release compositions also include liposomally entrapped compositions of the present invention. Liposomes are prepared by methods known per se: DE 3,218,121; Epstein, et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, in one embodiment, the composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compositions that are known to be deleterious to polypeptides.

Generally, the formulations are prepared by contacting the compounds of the invention uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. Suitable formulations, known in the art, can be found in Remington's Pharmaceutical Sciences (latest edition), Mack Publishing Company, Easton, PA.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The compositions are typically formulated in such vehicles at a concentration of about 0.01 µg/ml to 100 mg/ml, preferably 0.01 µg/ml to10 mg/ml, at a pH of about 3 to 8. It will be understood that the use of certain of the foregoing excipients, carriers, or stabilizers will result in the formation of salts.

Compositions to be used for therapeutic administration must be sterile. Sterility is readily accomplished by filtration through sterile filtration membranes (e.g., 0.2 micron membranes). Therapeutic compositions generally are placed into a container having a sterile access port, for example, an intravenous solution bag or vial having a stopper pierceable by a hypodermic injection needle.

The compounds of the invention ordinarily will be stored in unit or multi-dose containers, for example, sealed ampules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized composition using bacteriostatic Water-for-Injection.

Dosaging may also be arranged in a patient specific manner to provide a predetermined concentration of activity in the blood, as determined by an RIA technique, for instance. Thus patient dosaging may be adjusted to achieve regular on-going trough blood levels, as measured by RIA, on the order of from 50 to 1000 ng/ml, preferably 150 to 500 ng/ml.

The compounds of the invention are useful for administration to any animal, preferably a mammal (such as apes, cows, horses, pigs, boars, sheep, rodents, goats, dogs, cats, chickens, monkeys, rabbits, ferrets, whales, and dolphins), and more preferably a human.

The invention also provides a pharmaceutical pack or kit comprising one or more containers filled with one or more of the ingredients of the pharmaceutical compositions of the invention. Associated with such containers can be a notice in the form prescribed by a governmental agency regulating the manufacture, use or sale of pharmaceuticals or biological products, which notice reflects approval by the agency of manufacture, use or sale for human administration. In addition, the compositions of the present invention may be employed in conjunction with other therapeutic compositions.

Other therapeutic compositions useful for administration along with a compound of the present invention include cytotoxic drugs, particularly those which are used for cancer therapy. Such drugs include, in general, alkylating agents, anti-proliferative agents, tubulin binding agents and the like. Preferred classes of cytotoxic agents include, for example, the anthracycline family of drugs, the vinca drugs, the mitomycins, the bleomycins, the cytotoxic nucleosides, the pteridine family of drugs, diynenes, and the podophyllotoxins. Particularly useful members of those classes include, for example, adriamycin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, or podophyllotoxin derivatives such as etoposide or etoposide phosphate, melphalan, vinblastine, vincristine, leurosidine, vindesine, leurosine and the like. As noted previously, one skilled in the art may make chemical modifications to the desired compound in order to make reactions of that compound more convenient for purposes of preparing conjugates of the invention.

The compounds of the invention can be used to treat tumor-bearing animals, including humans, to generate an immune response against tumor cells. The generation of an adequate and appropriate immune response leads to tumor regression in vivo. Such "vaccines" can be used either alone or in combination with other therapeutic regimens, including but not limited to chemotherapy, radiation therapy, surgery, bone marrow transplantation, etc. for the treatment of tumors. For example, surgical or radiation techniques could be used to debulk the tumor mass, after which, the vaccine formulations of the invention can be administered to ensure the regression and prevent the progression of remaining tumor masses or micrometastases in the body. Alternatively, administration of the "vaccine" can precede such surgical, radiation or chemotherapeutic treatment.

Alternatively, the compounds of the invention can be used to immunize or "vaccinate" tumor-free subjects to prevent tumor formation. With the advent of genetic testing, it is now possible to predict a subject's predisposition for certain cancers. Such subjects, therefore, may be immunized using a compound comprising one or more antigenic ligands derived from tumors.

Suitable preparations of such vaccines include injectables, either as liquid solutions or suspensions; solid forms suitable for solution in, or suspension in liquid prior to injection, may also be prepared. The preparation may also be emulsified, or the polypeptides encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof. In addition, if desired, the vaccine preparation may also include minor amounts of auxiliary substances such as wetting or emulsifying agents, pH buffering agents, and/or adjuvants which enhance the effectiveness of the vaccine.

Examples of adjuvants which may be effective, include, but are not limited to: aluminum hydroxide, N-acetyl-muramyl-L-threonyl-D-isoglutamine (thr-MDP), N-acetyl-nor-muramyl-L-alanyl-D-isoglutamine, N-acetylmuramyl-L-alanyl-D-isoglutaminyl-L-alanine-2-(1'-2'-dipalmitoyl-sn-glycero-3-hydroxyphosphoryloxy)-ethylamine, GM-CSF, QS-21 (investigational drug, Progenics Pharmaceuticals,Inc.), DETOX (investigational drug, Ribi Pharmaceuticals), BCG, and CpG rich oligonucleotides.

The composition, if desired, can also contain minor amounts of wetting or emulsifying agents, or pH buffering agents. The composition can be a liquid solution, suspension, emulsion, tablet, pill, capsule, sustained release formulation, or powder. Oral formulation can include standard carriers such as pharmaceutical grades of mannitol, lactose, starch, magnesium stearate, sodium saccharine, cellulose, magnesium carbonate, etc.

In an alternate embodiment, compounds of the present invention may be used in adoptive immunotherapeutic methods for the .activation of NKT lymphocytes that are histocompatible with the patient. (for methods of adoptive immunotherapy, see, e.g., Rosenberg, U.S. Patent No. 4,690,915, issued September 1, 1987; Zarling, et al., U.S. Patent No. 5,081,029, issued January 14, 1992). Such NKT lymphocytes may be isolated from the patient or a histocompatible donor. The NKT lymphocytes are activated in vitro by exposure to the compound of the invention. Activated NKT lymphocytes are expanded and inoculated into the patient in order to transfer NKT cell immunity directed against the particular antigenic peptide or peptides.

The compounds of the present invention may be administered along with other compounds which modulate an immune response, for example, cytokines. The term "cytokine" refers to polypeptides, including, but not limited to, interleukins (e.g., IL-1, IL-2, IL-3, IL-4, IL-5, IL-6, IL-7, IL-8, IL-9, IL-10, IL-11, IL-12, IL- 13, IL-14, IL-15, IL-16, IL-17, and IL-18), α interferons (e.g., IFN-α), β interferon (IFN-β), γ interferons (e.g., IFN-γ), , colony stimulating factors (CSFs, e.g., CSF-1, CSF-2, and CSF-3), granulocyte- macrophage colony stimulating factor (GMCSF), transforming growth factor (TGF, e.g.,.TGFα and TGFβ), and insulin-like growth factors (IGFs, e.g., IGF-I and IGF-II).

The compounds of the invention may also be employed in accordance with the present invention by expression of such compounds, especially CD 1d-antibody fusion compounds, in vivo, which is often referred to as "gene r

Polynucleotide that encodes a compound of this invention that is a direct fusion of antibody and a CD1d molecule, as well as a polynucleotide encoding a β2-microglobulin fusion, may be introduced directly into cells by transfection or infection with a suitable vector so as to give rise to synthesis and secretion of that compound by the successfully transfected or infected cells. This can be accomplished by cotransfection with separate DNA vector constructs or by co-expression in the same vector.

Thus, for example, cells from a patient may be engineered with a polynucleotide (DNA or RNA) encoding a compound of the invention ex vivo, with the engineered cells then being provided to a patient to be treated with the compounds. Such methods are well-known in the art. For example, cells may be engineered by procedures known in the art by use of a retroviral particle containing RNA encoding a compound of the present invention.

Similarly, cells may be engineered in vivo for expression of a compound in vivo by, for example, procedures known in the art. As known in the art, a producer cell for producing a retroviral particle containing RNA encoding the compound of the present invention may be administered to a patient for engineering cells in vivo and expression of the polypeptide in vivo. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle. Examples of other delivery vehicles include an HSV-based vector system, adeno-associated virus vectors, pox viruses, and inert vehicles, for example, dextran coated ferrite particles.

Retroviruses from which the retroviral plasmid vectors hereinabove mentioned may be derived include, but are not limited to, lentiviruses, Moloney Murine Leukemia virus, spleen necrosis virus, retroviruses such as Rous Sarcoma Virus, Harvey Sarcoma virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

The nucleic acid sequence encoding the compound of the present invention is under the control of a suitable promoter. Suitable promoters which may be employed include, but are not limited to, adenoviral promoters, such as the adenoviral major late promoter; or heterologous promoters, such as cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs hereinabove described); the β-actin promoter; and human growth hormone promoters.

The retroviral plasmid vector is employed to transduce packaging cell lines to form producer cell lines. Examples of packaging cell lines which may be transfected include, but are not limited to, the PE501, PA317, 2-2, 2-AM, PA12, T19-14x, VT-19-17-H2, 2CRE, 2CRIP, GP+E-86, GP+envAm12, and DAN cell lines as described in Miller, Human Gene Therapy 1:5-14 (1990), The vector may transduce the packaging cells through any means known in the art. Such means include, but are not limited to, electroporation, the use of liposomes, and CaPO4 precipitation. In one alternative, the retroviral plasmid vector may be encapsulated into a liposome, or coupled to a lipid, and then administered to a host.

The producer cell line generates infectious retroviral vector particles which include the nucleic acid sequence(s) encoding the polypeptides. Such retroviral vector particles then may be employed to transduce eukaryotic cells, either in vitro or in vivo. The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

In certain embodiments, the polynucleotide constructs may be delivered as naked polynucleotides. By "naked" polynucleotides is meant that the polynucleotides are free from any delivery vehicle that acts to assist, promote, or facilitate entry into the cell, including viral sequences, viral particles, liposome formulation, lipofectin, precipitating agents and the like. Such methods are well known in the art and described, for example, in U.S. Patent Nos. 5,593,972, 5,589,466, and 5,580,859.

The naked polynucleotides used in the invention can be those which do not integrate into the genome of the host cell. These may be non-replicating sequences, or specific replicating sequences genetically engineered to lack the genome-integration ability. Alternatively, the naked polynucleotides used in the invention may integrate into the genome of the host cell by, for example, homologous recombination, as discussed below. Preferably, the naked polynucleotide construct is contained in a plasmid. Suitable expression vectors for delivery include, but are not limited to, vectors such as pRSVcat (ATCC 37152), pSVL and MSG (Pharmacia, Uppsala, Sweden), pSV2dhfr (ATCC 37146) and pBC12MI (ATCC 67109). Additional suitable plasmids are discussed in more detail above.

The naked polynucleotides can be administered to any tissue (such as muscle tissue) or organ, as described above. In another embodiment, the naked polynucleotides are administered to the tissue surrounding the tissue of origin. In another embodiment, the naked polynucleotides are administered systemically, through intravenous injection.

For naked polynucleotide injection, an effective dosage amount of polynucleotide will be in the range of from about 0.05 µg/kg body weight to about 50 mg/kg body weight. Preferably, the dosage will be from about 0.005 mg/kg to about 20 mg/kg and more preferably from about 0.05 mg/kg to about 5 mg/kg. The appropriate and effective dosage of the polynucleotide construct can readily be determined by those of ordinary skill in the art and may depend on the condition being treated and the route of administration.

The constructs may also be delivered with delivery vehicles such as viral sequences, viral particles, liposome formulations, lipofectin, precipitating agents, etc. Such methods of delivery are known in the art. For example, the polynucleotide construct can be delivered specifically to hepatocytes through the method of Wu et al., J. Biol. Chem. 264:6985-16987 (1989).

In certain embodiments, the polynucleotide constructs are complexed in a liposome preparation. Liposomal preparations for use in the instant invention include cationic (positively charged), anionic (negatively charged) and neutral preparations. However, cationic liposomes are particularly preferred because a tight charge complex can be formed between the cationic liposome and the polyanionic nucleic acid. Cationic liposomes have been shown to mediate intracellular delivery of plasmid DNA (Felgner et al., Proc. Natl. Acad. Sci. USA (1987) 84:7413-7416); mRNA (Malone et al., Proc. Natl. Acad. Sci. USA (1989) 86:6077-6081); and purified transcription factors (Debs et al., J. Biol. Chem. (1990) 265:10189-10192), in functional form.

Cationic liposomes are readily available. For example, N[1-2,3-dioleyloxy)propyl]-N,N,N-triethylammonium (DOTMA) liposomes are particularly useful and are available under the trademark Lipofectin, from GIBCO BRL, Grand Island, N.Y. (See, also, Felgner et al., Proc. Natl Acad. Sci. USA (1987) 84:7413-7416). Other commercially available liposomes include transfectace (DDAB/DOPE) and DOTAP/DOPE (Boehringer).

Other cationic liposomes can be prepared from readily available materials using techniques well known in the art. See, e.g. PCT Application No. WO 90/11092 for a description of the synthesis of DOTAP (1,2-bis(oleoyloxy)-3-(trimethylammonio)propane) liposomes. Preparation of DOTMA liposomes is explained in the literature, see, e.g., P. Felgner et al., Proc. Natl. Acad. Sci. USA 84:7413-7417. Similar methods can be used to prepare liposomes from other cationic lipid materials.

Similarly, anionic and neutral liposomes are readily available, such as from Avanti Polar Lipids (Birmingham, Ala.), or can be easily prepared using readily available materials. Such materials include phosphatidyl, choline, cholesterol, phosphatidyl ethanolamine, dioleoylphosphatidyl choline (DOPC), dioleoylphosphatidyl glycerol (DOPG), dioleoylphosphatidyl ethanolamine (DOPE), among others. These materials can also be mixed with the DOTMA and DOTAP starting materials in appropriate ratios. Methods for making liposomes using these materials are well known in the art.

For example, commercially dioleoylphosphatidyl choline (DOPC), dioleoylphosphatidyl glycerol (DOPG), and dioleoylphosphatidyl ethanolamine (DOPE) can be used in various combinations to make conventional liposomes, with or without the addition of cholesterol. Thus, for example, DOPG/DOPC vesicles can be prepared by drying 50 mg each of DOPG and DOPC under a stream of nitrogen gas into a sonication vial. The sample is placed under a vacuum pump overnight and is hydrated the following day with deionized water. The sample is then sonicated for 2 hours in a capped vial, using a Heat Systems model 350 sonicator equipped with an inverted cup (bath type) probe at the maximum setting while the bath is circulated at 15□C. Alternatively, negatively charged vesicles can be prepared without sonication to produce multilamellar vesicles or by extrusion through nucleopore membranes to produce unilamellar vesicles of discrete size. Other methods are known and available to those of skill in the art.

The liposomes can comprise multilamellar vesicles (MLVs), small unilamellar vesicles (SUVs), or large unilamellar vesicles (LUVs), with SUVs being preferred. The various liposome-nucleic acid complexes are prepared using methods well known in the art. See, e.g., Straubinger et al., Methods of Immunology (1983), 101:512-527. For example, MLVs containing nucleic acid can be prepared by depositing a thin film of phospholipid on the walls of a glass tube and subsequently hydrating with a solution of the material to be encapsulated. SUVs are prepared by extended sonication of MLVs to produce a homogeneous population of unilamellar liposomes. The material to be entrapped is added to a suspension of preformed MLVs and then sonicated. When using liposomes containing cationic lipids, the dried lipid film is resuspended in an appropriate solution such as sterile water or an isotonic buffer solution such as 10 mM Tris/NaCl, sonicated, and then the preformed liposomes are mixed directly with the DNA. The liposome and DNA form a very stable complex due to binding of the positively charged liposomes to the cationic DNA. SUVs find use with small nucleic acid fragments. LUVs are prepared by a number of methods, well known in the art. Commonly used methods include Ca2+-EDTA chelation (Papahadjopoulos et al., Biochim. Biophys. Acta (1975) 394:483; Wilson et al., Cell (1979) 17:77); ether injection (Deamer, D. and Bangham, A., Biochim. Biophys. Acta (1976) 443:629; Ostro et al., Biochem. Biophys. Res. Commun. (1977) 76:836; Fraley et al., Proc. Natl. Acad. Sci. USA (1979) 76:3348); detergent dialysis (Enoch, H. and Strittmatter, P., Proc. Natl. Acad. Sci. USA (1979) 76:145); and reverse-phase evaporation (REV) (Fraley et al., J. Biol. Chem. (1980) 255:10431; Szoka, F. and Papahadjopoulos, D., Proc. Natl. Acad. Sci. USA (1978) 75:145; Schaefer-Ridder et al., Science (1982) 215:166).

Additional examples of useful cationic lipids include dipalmitoyl-phophatidylethanolamine 5-carboxyspen-nylamide (DPPES); 5-carboxyspermylglycine dioctadecylamide (DOGS); dimethyldioctdecyl-ammonium bromide (DDAB); and (□)-N,N-dimethyl-N-[2-(sperminecarboxamido)ethyl]-2,3-bis(dioleyloxy)-1-propaniminium pentahydrochloride (DOSPA). Non-diether cationic lipids, such as DL-1,2-dioleoyl-3-dimethylaminopropyl-□-hydroxyethylammonium (DORI diester), 1,2-O-dioleyl-3-dimethylaminopropyl-□-hydroxyethylammonium (DORIE diether), 1-O-oleyl-2-oleoyl-3-dimethylaminopropyl-□-hydroxyethylammonium (DORI ester/ether), and their salts promote in vivo gene delivery. Cationic cholesterol derivatives such as, {3□[N-N,N□-dimethylamino)ethane]-carbomoyl}-cholesterol (DC-Chol), are also useful.

Preferred cationic lipids include: (±)-N-(2-hydroxyethyl)-N,N-dimethyl-2,3-bis(tetradecyloxy)-1-propaniminium bromide; 3,5-(N,N-di-lysyl)diamino-benzoylglycyl-3-(DL-1,2-dioleoyl-dimethylaminopropyl-□-hydroxyethylamine) (DLYS-DABA-GLY-DORI diester); 3,5-(NN-dilysyl)-diaminobenzoyl-3-(DL-1,2-dioleoyl-dimethylaminopropyl-□-hydroxyethylamine) (DLYS-DABA-DORI diester); and 1,2-dioleoyl-sn-glycero-3-phosphoethanolamine. Also preferred is the combinations of the following lipids: (±)-N-(2-hydroxyethyl)-N,N-dimethyl-2,3-bis (tetradecyloxy)-1-propaniminium bromide and 1,2-dioleoyl-sn-glycero-3-phosphoethanolamine; and (±)-N-(2-hydroxyethyl)-N,N-dimethyl-2,3-bis(tetradecyloxy)-1-propaniminium bromide, and 1,2-dioleoyl-sn-glycero-3-phosphoethanolamine in a 1:1 ratio.

The lipid formulations may have a cationic lipid alone, or also include a neutral lipid such as cardiolipin, phosphatidylcholine, phosphatidylethanolamine, dioleoylphosphatylcholine, dioleoylphosphatidyl-ethanolamine, 1,2-dioleoyl-sn-glycero-3-phosphatidylethanolamine (DOPE), sphingomyelin, and mono-, di- or tri-acylglycerol).

Lipid formulations may also have cationic lipid together with a lysophosphatide. The lysophosphatide may have a neutral or a negative head group. Useful lysophosphatides include lysophosphatidylcholine, lysophosphatidyl-ethanolamine, and 1-oleoyl lysophosphatidylcholine. Lysophosphatide lipids are present Other additives, such as cholesterol, fatty acid, ganglioside, glycolipid, neobee, niosome, prostaglandin, sphingolipid, and any other natural or synthetic amphiphiles, can be used. A preferred molar ratio of cationic lipid to neutral lipid in these lipid formulations is from about 9:1 to about 1:9; an equimolar ratio is more preferred in the lipid-containing formulation in a 1:2 ratio of lysolipid to cationic lipid.

Generally, the ratio of DNA to liposomes will be from about 10:1 to about 1:10. Preferably, the ratio will be from about 5:1 to about 1:5. More preferably, the ratio will be about 3:1 to about 1:3. Still more preferably, the ratio will be about 1:1.

U.S. Patent No. 5,676,954 reports on the injection of genetic material, complexed with cationic liposomes carriers, into mice. U.S. Patent Nos. 4,897,355, 4,946,787, 5,049,386, 5,459,127, 5,589,466, 5,693,622, 5,580,859, 5,703,055, and international publication no. WO 94/9469 provide cationic lipids for use in transfecting DNA into cells and mammals. U.S. Patent Nos. 5,589,466, 5,693,622, 5,580,859, 5,703,055, and international publication no. WO 94/9469 provide methods for delivering DNA-cationic lipid complexes to mammals.

In certain other embodiments, cells are engineered, ex vivo or in vivo, with the polynucleotide operably linked to a promoter contained in an adenovirus vector. Adenovirus can be manipulated such that it encodes and expresses the desired gene product, and at the same time is inactivated in terms of its ability to replicate in a normal lytic viral life cycle. Adenovirus expression is achieved without integration of the viral DNA into the host cell chromosome, thereby alleviating concerns about insertional mutagenesis. Furthermore, adenoviruses have been used as live enteric vaccines for many years with an excellent safety profile (Schwartz, A. R. et al. (1974) Am. Rev. Respir. Dis.109:233-23S). Finally, adenovirus mediated gene transfer has been demonstrated in a number of instances including transfer of alpha-1-antitrypsin and CFTR to the lungs of cotton rats (Rosenfeld, M. A. et al. (1991) Science 252:431-434; Rosenfeld et al., (1992) Cell 68:143-155). Furthermore, extensive studies to attempt to establish adenovirus as a causative agent in human cancer were uniformly negative (Green, M. et al. (1979) Proc. Natl. Acad. Sci. USA 76:6606).

Suitable adenoviral vectors useful in the present invention are described, for example, in Kozarshy and Wilson, Curr. Opin. Genet. Devel. 3:499-503 (1993); Rosenfeld et al., Cell 68:143-155 (1992); Engelhardt et al., Human Genet. Ther. 4:759-769 (1993); Yang et al., Nature Genet. 7:362-369 (1994); Wilson et al., Nature 365:691-692 (1993); and U.S. Patent No. 5,652,224.For example, the adenovirus vector Ad2 is useful and can be grown in human 293 cells. These cells contain the E1 region of adenovirus and constitutively express E1a and Elb, which complement the defective adenoviruses by providing the products of the genes deleted from the vector. In addition to Ad2, other varieties of adenovirus (e.g., Ad3, Ad5, and Ad7) are also useful in the present invention.

Preferably, the adenoviruses used in the present invention are replication deficient. Replication deficient adenoviruses require the aid of a helper virus and/or packaging cell line to form infectious particles. The resulting virus is capable of infecting cells and can express a polynucleotide of interest which is operably linked to a promoter, for example, the polynucleotide of the present invention, but cannot replicate in most cells. Replication deficient adenoviruses may be deleted in one or more of all or a portion of the following genes: E1a, Elb, E3, E4, E2a, or L1 through L5.

In certain other embodiments, the cells are engineered, ex vivo or in vivo, using an adeno-associated virus (AAV). AAVs are naturally occurring defective viruses that require helper viruses to produce infectious particles (Muzyczka, N., Curr. Topics in Microbiol. Immunol. 158:97 (1992)). It is also one of the few viruses that may integrate its DNA into non-dividing cells. Vectors containing as little as 300 base pairs of AAV can be packaged and can integrate, but space for exogenous DNA is limited to about 4.5 kb. Methods for producing and using such AAVs are known in the art. See, for example, U.S. Patent Nos. 5,139,941, 5,173,414, 5,354,678, 5,436,146, 5,474,935, 5,478,745, and 5,589,377.

For example, an appropriate AAV vector for use in the present invention will include all the sequences necessary for DNA replication, encapsidation, and host cell integration. The polynucleotide construct is inserted into the AAV vector using standard cloning methods, such as those found in Sambrook et al., Molecular Cloning: A Laboratory Manual, Cold Spring Harbor Press (1989). The recombinant AAV vector is then transfected into packaging cells which are infected with a helper virus, using any standard technique, including lipofection, electroporation, calcium phosphate precipitation, etc. Appropriate helper viruses include adenoviruses, cytomegaloviruses, vaccinia viruses, or herpes viruses. Once the packaging cells are transfected and infected, they will produce infectious AAV viral particles which contain the polynucleotide construct. These viral particles are then used to transduce eukaryotic cells, either ex vivo or in vivo. The transduced cells will contain the polynucleotide construct integrated into its genome, and will express the molecule of interest.

Any mode of administration of any of the above-described polynucleotides constructs can be used so long as the mode results in the expression of one or more molecules in an amount sufficient to provide a therapeutic effect. This includes direct needle injection, systemic injection, catheter infusion, biolistic injectors, particle accelerators (i.e., "gene guns"), gelfoam sponge depots, other commercially available depot materials, osmotic pumps (e.g., Alza minipumps), oral or suppositorial solid (tablet or pill) pharmaceutical formulations, and decanting or topical applications. For example, direct injection of naked calcium phosphate-precipitated plasmid into rat liver and rat spleen or a protein-coated plasmid into the portal vein has resulted in gene expression of the foreign gene in the rat livers (Kaneda et al., Science 243:375 (1989)).

A preferred method of local administration is by direct injection. Preferably, a recombinant molecule of the present invention complexed with a delivery vehicle is administered by direct injection into or locally within the area of the liver. Administration of a composition locally within the area of the liver refers to injecting the composition centimeters and preferably, millimeters within the liver.

Another method of local administration is to contact a polynucleotide-promoter construct of the present invention in or around a surgical wound. For example, a patient can undergo surgery and the polynucleotide construct can be coated on the surface of tissue inside the wound or the construct can be injected into areas of tissue inside the wound.

Therapeutic compositions useful in systemic administration, include recombinant molecules of the present invention complexed to a targeted delivery vehicle of the present invention. Suitable delivery vehicles for use with systemic administration comprise liposomes comprising ligands for targeting the vehicle to a particular site, for example, ligands for targeting the vehicle to a tissue of interest. Targeting vehicles for other tissues and organs are well known to skilled artisans.

Preferred methods of systemic administration, include intravenous injection, aerosol, oral and percutaneous (topical) delivery. Intravenous injections can be performed using methods standard in the art. Aerosol delivery can also be performed using methods standard in the art (see, for example, Stribling et al., Proc. Natl. Acad. Sci. USA 189:11277-11281, 1992. Oral delivery can be performed by complexing a polynucleotide construct of the present invention to a carrier complexing a polynucleotide construct of the present invention to a carrier capable of withstanding degradation by digestive enzymes in the gut of an animal. Examples of such carriers, include plastic capsules or tablets, such as those known in the art. Topical delivery can be performed by mixing a polynucleotide construct of the present invention with a lipophilic reagent (e.g., DMSO) that is capable of passing into the skin.

Determining an effective amount of substance to be delivered can depend upon a number of factors including, for example, the chemical structure and biological activity of the substance, the age and weight of the animal, the precise condition requiring treatment and its severity, and the route of administration. The frequency of treatments depends upon a number of factors, such as the amount of polynucleotide constructs administered per dose, as well as the health and history of the subject. The precise amount, number of doses, and timing of doses will be determined by the attending physician or veterinarian.

Direct administration of a DNA construct coding for a compound of the invention can be suitably accomplished for expression of the fusion compound within cells of the subject. Also, rather than directly administering nucleic acids coding for a compound of the invention to a subject, host compatible cells into which such nucleic acids have been introduced may be administered to the subject. Upon administration to a subject, such engineered cells can then express in vivo the compound of the invention. Such engineered cells can be administered to a subject to induce an immune response or alternatively to suppress an immune response, as disclosed herein.

A treatment method for suppression of an immune response provides for administration of a compound of the invention in which the peptide is a TCR antagonist or partial agonist. See Sette et al., Ann. Rev. Immunol. 12:413-431 (1994)). Peptides that are TCR antagonists or partial agonists can be readily identified and selected by the in vitro protocols identified above. A compound of the invention that contains a peptide that is a TCR antagonist or partial agonist is particularly preferred for treatment of allergies and autoimmune diseases.

Immunosuppressive therapies of the invention also may be used in combination with other known immunosuppressive agents such as anti-inflammatory drugs to provide a more effective treatment of a T cell-mediated disorder. For example, other immunosuppressive agents useful in conjunction with the compounds of the invention include anti-inflammatory agents such as corticosteroids and nonsteroidal drugs.

The invention also provides methods for invoking an immune response in a mammal such as a human, including vaccinating a mammal with a compound or composition described herein.

The compounds of the invention are useful for raising an immune response and treating hyperproliferative disorders. Examples of hyperproliferative disorders that can be treated by the compounds of the invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated by the compounds of the invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

The compounds of the present invention are also useful for raising an immune response against infectious agents. Viruses are one example of an infectious agent that can cause disease or symptoms that can be treated by the compounds of the invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox, hemorrhagic fever, measles, mumps, parainfluenza, rabies, the common cold, Polio, leukemia, Rubella, sexually transmitted diseases, skin diseases (e.g., Kaposi's, warts), and viremia.

Similarly, bacterial or fungal agents that can cause disease or symptoms and that can be treated by the compounds of the invention include, but are not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections.

Moreover, parasitic agents causing disease or symptoms that can be treated by the compounds of the invention include, but are not limited to, the following families: amebiasis, babesiosis, coccidiosis, cryptosporidiosis, dientamoebiasis, dourine, ectoparasitic, giardiasis, helminthiasis, leishmaniasis, theileriasis, toxoplasmosis, trypanosomiasis, and trichomonas.

Additionally, the compounds of the invention are useful for treating autoimmune diseases. An autoimmune disease is characterized by the attack by the immune system on the tissues of the victim. In autoimmune diseases, the recognition of tissues as "self" apparently does not occur, and the tissue of the afflicted subject is treated as an invader--i.e., the immune system sets about destroying this presumed foreign target. The compounds of the present invention are therefor useful for treating autoimmune diseases by desensitizing the immune system to these self antigens by provided a TCR signal to T cells without a costimulatory signal or with an inhibitory signal.

Examples of autoimmune diseases which may be treated using the compounds of the present invention include, but are not limited to Addison's Disease, hemolytic anemia, antiphospholipid syndrome, rheumatoid arthritis, dermatitis, allergic encephalomyelitis, glomerulonephritis, Goodpasture's Syndrome, Graves' Disease, multiple sclerosis, myasthenia gravis, neuritis, ophthalmia, bullous pemphigoid, pemphigus, polyendocrinopathies, purpura, Reiter's Disease, Stiff-Man Syndrome, autoimmune thyroiditis, systemic lupus erythematosus, autoimmune pulmonary inflammation, Guillain-Barre Syndrome, insulin dependent diabetes mellitis, autoimmune inflammatory eye disease, autoimmune hemolysis, psoriasis, juvenile diabetes, primary idiopathic myxedema, autoimmune asthma, scleroderma, chronic hepatitis, hypogonadism, pernicious anemia, vitiligo, alopecia areata, Coeliac disease, autoimmune enteropathy syndrome, idiopathic thrombocytic purpura, acquired splenic atrophy, idiopathic diabetes insipidus, infertility due to antispermatazoan antibodies, sudden hearing loss, sensoneural hearing loss, polymyositis, autoimmune demyelinating diseases, traverse myelitis, ataxic sclerosis, progressive systemic sclerosis, dermatomyositis, polyarteritis nodosa, idiopathic facial paralysis, cryoglobulinemia, inflammatory bowel diseases, Hashimoto's disease, adrenalitis, hypoparathyroidism, and ulcerative colitis.

Similarly, allergic reactions and conditions, such as asthma (particularly allergic asthma) or other respiratory problems, may also be treated by compounds of the invention. Moreover, the compounds of the invention can be used to treat anaphylaxis, hypersensitivity to an antigenic molecule, or blood group incompatibility.

The compounds of the invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of the compounds of the invention that inhibit an immune response may be an effective therapy in preventing organ rejection or GVHD.

The compounds of the invention which can inhibit an immune response are also useful for treating and/or preventing atherosclerosis; olitis; regional enteritis; adult respiratory distress syndrome; local manifestations of drug reactions, such as dermatitis, etc.; inflammation-associated or allergic reaction patterns of the skin; atopic dermatitis and infantile eczema; contact dermatitis; psoriasis; lichen planus; allergic enteropathies; allergic rhinitis; bronchial asthma; hypersensitivity or destructive responses to infectious agents; poststreptococcal diseases, e.g. cardiac manifestations of rheumatic fever, and the like.

### EXAMPLES

### EXAMPLE 1

### Anti-tumor antibody-MIC-A or B or ULBP conjugates or fusion proteins

Targeting a high density of MHC class I-related molecule such as MIC-A or -B, ULBP or CD1d on the surface of tumor cells will activate different effector cells such as NK, NKT, or T cells and induce them to kill target tumor cells.

It has previously been shown experimentally and clinically that radiolabeled or fluorescent labeled mAbs directed against TAA can be specifically targeted in vivo on tumor cells (Delaloye et al., J. Clin. Invest. 77:301 (1986); and Folli et al., Proc. Natl. Acad. Sci. USA 89:7973 (1992)). Thus our general strategy will be to couple monomorphic MHC class I-related proteins to anti-TAA mAbs or fragments of mAbs in order to target them on tumor cells. The interest of the proposed strategy is that it will take advantage of both the efficient targeting properties of high affinity anti-TAA mAbs and the powerful and rapid activation of effector cells known to play an essential role in innate immunity.

A combined strategy of tumor targeting has been developed, using monoclonal antibodies (mAbs) specific for a tumor-associated antigen (TAA), which are coupled to a soluble classical MHC class I molecule loaded with a highly antigenic viral peptide specific for cytotoxic T lymphocytes (CTL). Thus, the CTL are activated and selectively kill the targeted cancer cells.

In a first approach, they have developed tetramers of HLA-A2 loaded with the immunodominant Flu-MA peptide and coupled to either one of 3 different Fab' fragments from mAbs specific for TAA (anti-CEA, anti-ErbB-2 or anti-CD20). It was demonstrated that tumor expressing the relevant TAA were rendered susceptible to efficient lysis by Flu-Ma peptide specific cytotoxic T lymphocytes (Robert et al., Eur. J. Immunol. 30:3165 (2000)).

In a second step, the inventors have demonstrated that also monomers of Fab-HLA-A2/Flu-peptide conjugates are active, but only when they reach their target and are oligomerized on the surface of the tumor cells *(*Robert et al., Cancer Immunity (2001)). This property presents several advantages to the multimerized conjugates also developed by others (Ogg et al., Br. J. Cancer 82:1058 (2000); Savage et al., Int. J. Cancer 98:561 (2002)). First, monomers do not induce significant activation in solution and hence can be injected in larger amounts without side effects. Second, technically, it is easier to produce monomeric forms of conjugates or fusion proteins in amounts necessary for patient treatment (see PCT/US01/17184, WO 01/90198).

One limitation of MHC class I/ peptide complexes are their polymorphism, which means that a large pool of conjugates should be developed to be able to treat the great majority of patients. That is the reason why in the present invention, we are introducing the so-called non-polymorphic MHC class I related molecules. They differ from MHC class I molecules, not only because they are non-polymorphic, but also because they contain, either no antigenic peptide for the MIC-A/B or ULBP molecules, or a glycolipid antigen for the CD1d molecules. In addition, these class I related molecules activate other types of effector cells belonging to the innate immune response, namely NK and NKT cells.

Among the first monomorphic class I related molecules, which have been described, are the MIC-A and -B, which have a high degree of homology between each other (84%) (Bauer et al., Science 285:727 (1999); Li et al., Immunity 10:577 (1999)). Contrary to the classical MHC class I molecules which form an heterodimer between the heavy chain and the B-2 microglobulin, the MIC-A and -B consist of a single heavy chain, which has only a low degree of homology with the heavy chain of classical MHC class I (about 30%) (Cosman et al., Immunity 14:123 (2001)).

The crystal structure of MIC-A has been recently established (Li et al., Immunity 10:577 (1999)). It revealed a dramatically altered MHC class I fold, both in detail and overall domain organization. There is only a remnant of a peptide binding groove and a loss of B-2 microglobulin binding site. The expression of MIC-A and -B protein is essentially restricted to gut epithelium and the corresponding genes appear to be under the control of a heat-shock promoter element. Thus, the expression of MIC-A can be induced on the epithelial cells by various stresses, including viral infection and malignant transformation (Groh et al., Proc. Natl. Acad. Sci. USA 96:6879 (1999)).

One of the major biological properties of MIC-A and -B is to bind specifically to the NKG2D activating receptor present on NK cells, as well as on CD8 T cells expressing also, either the y-8 or the a-B antigen receptor. Through this binding to NKG2D receptor, the MIC-A and -B can act as a natural antigen and activate NK and T cells and induce them to kill the epithelial cells overexpressing the MIC proteins. This activation of effector cells is more rapid than the system of acquired immunity and represents a first line of defense belonging to the innate immunity (Groh et al., Proc. Natl. Acad. Sci. USA 96:6879 (1999)).

ULBPs are a different group of monomorphic MHC class I related proteins, which are GPI- anchored and have only 25% amino acid sequence homology with classical MHC class I molecules and also a low sequence homology with MIC-A or-B (23%) (Cosman et al., Immunity 14:123 (2001)). The ULBPs are overexpressed in cells infected with the human cytomegaloviras (HCMV) and they share with MIC-A and -B the property of binding the activating NKG2D receptor. Thus, human cells infected with HCMV through overexpression of ULBPs can stimulate cytokine and chemokine production by NK cells and confer susceptibility to NK cells cytotoxicity (Cosman et al., Immunity 14:123 (2001)). In the present strategy, ULBPs conjugated to antibodies are used as ligands for NKG2D receptor and are targeted on tumor cells independently of HCMV infection.

The CD1 family represents another non-polymorphic lineage of class I related antigen-presenting molecules. Some T cells recognize bacterial and self-glycolipids associated with CD1 molecules (Porcelli et al., Annu. Rev. Immunol. 17:297 (1999); Shamshiev et al., Eur. J. Immunol. 29:1667 (1999); Park and Bendelac, Nature 406:788 (2000); and Matsuda and Kronenberg, Curr. Opin. Immunol. 13:19 (2001)). Five CD1 monomorphic isoforms have been identified (CD1 a, b, c, d and e), which have a high degree of homology among themselves and a lower degree of homology with the classical MHC class I molecules (almost no homology on alpha-1 and up to 35% on alpha-2 and alpha-3 domains). Despite low sequence homology, the resolution of the crystal structure of mouse CD1d revealed a folding similar to MHC class I (Zeng et al., Science 277:339 (1997)). The major difference resides in the antigen binding groove which is composed of only two very hydrophobic and deep pockets, that are likely to accomodate the lipidic tail of the antigen, while presenting the carbohydrate part to the T cell receptor. Numerous reports have described foreign and self-glycolipid presentation by CD1 molecules in bacterial infections and autoimmunity and their role in innate as well as acquired imunity has been established (Shamshiev et al., Eur. J. Immunol. 29:1667 (1999); Park and Bendelac, Nature 406:788 (2000); and Matsuda and Kronenberg, Curr. Opin. Immunol. 13:19 (2001)).

Of importance for the present invention, the CD1d molecule has been broadly associated with anti-tumor immunity (Wilson et al., Trends Mol. Med. 8:225 (2002); and Brutkiewicz et al., Crit. Rev. Oncol. Hematol. 41:287 (2002)). Several other characteristics have drawn much attention on CD1d in the past few years which makes this molecule very attractive for our strategy. First, CD1d-restricted T cells are characterized both in mice and humans by a highly restricted TCR repertoire and expression of some NK markers (CD161, NKG2d). They were therefore called NKT cells and are considered as part of innate immunity (Bendelac et al., Science 268:863 (1995)). Second, NKT cells have an unsual ability of secreting both Th1 and Th2 cytokines and their role in inflammation, autoimmunity and tumor immunity is being extensively investigated (Bendelac et al., Science 268:863 (1995); Chen and Paul, J,. Immunol. 159:2240 (1997); and Exley et al., J. Exp. Med. 186:109 (1997)). Last, the natural ligands of CD1d are still largely unknown but a high affinity ligand and potent activator of NKT cells has been isolated from an extract of a marine sponge known in japanese medicine to have antimetastatic effects. This glycoshingolipid absent in mammal tissues was characterized as alpha-galactosylceramide (alpha-GalCer) and has been used both in vitro and in vivo in various tumor models. It was confirmed that alpha-GalCer antimetastatic activity is mediated by CD1d-restricted NKT cells (Wilson et al., Trends Mol. Med. 8:225 (2002); and Brutkiewicz et al., Crit. Rev. Oncol. Hematol. 41:287 (2002)).

a) Chemical conjugate. MIC-A or -B or ULBP molecules are produced in bacteria or in an eukaryotic cells with a mutation introducing a cysteine at the C-terminus, as described for HLA-A2 by Robert et al. (Robert et al., Cancer Immunity (2001)). Then, the free SH group on the cysteine is saturated with an excess of bismaleimide coupling reagent. After elimination of the excess bis-maleimide, the maleimide derivatized MIC-A/B or ULBP is chemically coupled to the free SH groups of the cysteines from the Fab' fragments of a high affinity mAb anti-TAA, as described (Robert et al., Cancer Immunity (2001)).

b) Fusion protein. The genes encoding MIC-A or -B, or ULBP are fused to the genes encoding either a single chain antibody fragment or a Fab fragment directed against a human TAA. The genes are fused so that the synthesis starts either at the N-terminus of the MIC-A/B or ULBP and continues with the antibody fragment or at the N-terminus of the antibody fragment and be followed by the MIC-A/B or ULBP.

In both cases, a semi-rigid amino-acid spacer sequence is placed between the two molecules to avoid steric hindrance. The best form of fusion protein is selected by in vitro testing. For these tests the different fusion proteins are incubated on ⁵¹Cr-labeled target tumor cells bearing the relevant, TAA and different amounts of NK cells are added. The conjugate, which induces the most efficient lysis, as determined by ⁵¹Cr release is selected.

c) *In* vivo evaluation of the conjugate or fusion proteins. The conjugates or fusion proteins which gave the best in vitro results in the ⁵¹Cr release assay are then tested in vivo first in experimental animal models and subsequently in tumor bearing patients.

The experimental animal bearing a tumor known to express the relevant TAA are injected i.v. with increasing amounts of antibody-MIC-A/-B or ULBP conjugates or fusion proteins. One or two days after injection of the conjugate or fusion proteins, the animal or the patients are treated with a cytokine or chemokine such as interferon-gamma or IL-12 or IL-2, known to stimulate NK cells activity. The tumor size is monitored to verify that the tumor cells, which have been targeted with an optimal dose of conjugates or fusion proteins, are selectively killed by NK cells.

### EXAMPLE 2

### Anti-tumor antibody CD1d conjugates or fusion proteins

a) Chemical coupling. Recombinant soluble human CD1d are produced in eukaryotic cells with a mutation at the C-terminus introducing a single cysteine residue. After derivation of the free cysteine with an excess of bis-maleimide and elimination of the excess of free bis-maleimide, the maleimide derivatized CD1d is coupled to the free SH group of eysteines from an Fab' fragment of a high affinity anti-TAA mAb, as described previously (Robert et al., Cancer Immunity (2001)).

b) Fusion protein. The cDNA of soluble CD1d protein is fused to the sequences encoding scFv or Fab fragment from a high affinity anti-TAA mAb. Two types of fusions will be made so that either the CD-1d will be expressed at the N-terminus, followed by the antibody fragment or the opposite. In both cases, a semi-rigid amino-acid spacer sequence is placed between the antibody fragment and the CD1d molecule to avoid steric hindrance. The most active form of fusion protein is selected in vitro on target cancer cells expressing the relevant TAA, as described in Example 1b, except that the CD1d in the fusion protein is loaded with alpha-galactosylceramide and that NKT cells are used as effector cells instead of NK cells.

c) In vivo evaluation of the conjugate or fusion protein. The conjugate or fusion proteins which gave the best in vitro results are tested in vivo, first in tumor bearing experimental animals and subsequently in patients, as described in example 1c, except that the antibody fragment-CD1d conjugate or fusion protein is loaded with alpha-galactosylceramide or with different activating glycolipids.

Alternatively, the antibody-CD1d conjugate or fusion protein is injected without alpha-galactosylceramide or glycolipids, in order that the CD1d molecules, targeted on tumor cells, become progressively loaded with endogenous glycolipids.

A third strategy is evaluated in vivo in experimental animals. It consists of the injection in a first step of alpha-galactosylceramide in order to stimulate activation and proliferation of NKT cells, followed in a second step 8 to 24 hours later, by the injection of the antibody-CD1d conjugate.

### EXAMPLE 3

### Anti-neoangiogenesis antibody-MHC class I related conjugates or fusion proteins

It is now broadly accepted that neoangiogenesis represents an essential condition for tumor development and growth. Thus, one application of our strategy consists in antibody targeting of monomorphic MHC class I related protein such as MIC-A/B, ULCBPs or CD1d molecules in the neovessels. The presence of monomorphic MHC class I related molecules in the neovessels will focus NK and NKT cells activity in the tumor area. The activation of NK and NKT cells in the tumor neovessels have three beneficial effects: 1) It increases inflammation in the tumor area and enhances anti-tumor immune response by recruitment of antigen presenting cells and T lymphocytes through local secretion of cytokines and interleukins; 2) It has a direct cytotoxic effect against endothelial cells from tumor neovessels and thus decreases blood flow in the tumor and 3) The increased secretion of TNF and other cytokines by macrophages ultimately induce the collapse of the tumor neovasculature.

Numerous mAbs directed against antigens associated with tumor neovessels have been described, such as mAbs against avB3 integrins, TNF receptors, platelet derived growth factor receptor, or the ED-B oncofoetal domain of fibronection (Halin et al., Nat. Biotechnol. 20:2264 (2002)) and many others (for review, Nature Biotechnol. 17:963) and are be used in this strategy of tumor treatment.

b) Chemical coupling and fusion between mAbs against neoangiogenesis antigens and monomorphic MHC class I related proteins. The synthesis of chemical conjugates and fusion proteins between the mAbs anti-neoangiogenesis antigens and the MICA-A/B, ULBPs or CD1d is performed as described in Examples 1 and 2, paragraph a) and b).

c) In vitro evaluation of the conjugates or fusion proteins on endothelial cells. The effect of the different conjugates or fusion proteins is tested on human umbilical vein endothelial cells (HUVEC), or on tumor cell lines expressing neoangiogenesis antigens in the presence of either NK or NKT cells. The target cells are ⁵¹Cr-labeled and their lysis by effector cells is measured in presence or absence of conjugate or fusion protein as described for tumor target cells in Examples 1 and 2, c). Alternatively, the degree of activation of NK or NKT cells in presence of target cells coated or not with the conjugates or fusion proteins is evaluated by measurement of the release of cytokines or interleukins such as IFN-gamma or IL4 by NK or NKT cells.

d) In vivo evaluation of conjugates or fusion proteins on tumor bearing experimental animals. The effect of intravenous injection of conjugates or fusion proteins on tumor growth is evaluated in tumor bearing mice. Furthermore the tumor vascular parameters, such as blood flow, blood volume and vascular permeability is analyzed during conjugate or fusion protein treatment, as previously described for TNF treatment (Folli et al., Proc. Natl. Acad. Sci. USA 89:7973 (1992)).

### EXAMPLE 4

### Construction of IgG-Avidin fusion protein complexed with soluble CD1d

An antibody fusion molecule is formed that contains either all (IgG-avidin), or a portion (F(ab)-avidin and F(ab')2-avidin) of an antibody molecule fused in frame with the chicken avidin open reading frame. The chimeric Ig Heavy chain-avidin along with the complementing Ig Light Chain are produced in Drosophila cells in three steps. In step one, PCR is used to create the cloning cassette for VH, including appropriate regions preceded by a signal sequence (SS) for secretion and followed by a linker with an embedded KpnI restriction site. In step two, PCR is used to amplify the avidin gene. Finally, in step three, restriction digestion is used at the KpnI site followed by ligation to combine the two fragments. The IgG1 and avidin proteins are separated by a 12 amino acid linker.

The extracellular portion of CD1d along with β2-microglobulin (β2M) is produced separately in Drosophila. The CD1d/B2M molecule is biotinylated, incubated with the Ig-Avidin fusion protein and purified.

1. Construction of VH cassette. Standard PCR is used to amplify the relevant portions of IgG with a pre-configured VH insertion site from a previously described template. The PCR product is gel purified according to standard procedure. Specifically, an IgG1 construct that allows for insertion of a variable gene of interest through BssHI and BstEII restriction sites has been generated (U.S. Appl. Publ. No. 2003/0104402, published June 5, 2003). This construct is available as template for PCR using the following primers:

For the F(ab) fragment: (7) Sense 5' AATTG**CGGCCGC** AAA**CCATGG**GATGGAGCTGTATCATC 3' (SEQ ID NO:3) (NotI and NcoI sites in bold); and (8) Anti-sense 5' **CGGGGTACC TGACCCACCGCCTCC**TTTCTTGTCCACCTTGGTGTT 3' (SEQ ID NO:4) (linker is in bold; KpnI site is bolded and underlined.).

For the (Fab'2) fragment: (7) Sense 5' AATT**GCGGCCGC** AAA**CCATGGG**ATGGAGCTGTATCATC 3' (SEQ ID NO:5) (NotI and NcoI sites in bold); and (8) Anti-sense 5' **CGGGGTACC TGACCCACCGCCTCC**TGGGCACGGTGGGCATGTGTG 3' (SEQ ID NO:6) (linker is in bold; KpnI site is bolded and underlined).

For full IgG1: (7) Sense 5' AATTGCGGCCGCAAA**CCATGGG** ATGGAGCTGTATCATC 3' (SEQ ID NO:7) (NotI and NcoI sites in bold); and (8) Anti-sense 5' **CGGGGTACCTGACCCACCGCCTCC** TTTACCCGGAGACAGGGAGAG 3' (SEQ ID NO:8) (linker is in bold; KpnI site is bolded and underlined).

In other embodiments, the CH1 region derives from other immunoglobulin isotypes, including IgG2, IgG3, IgG4, IgA, IgM, IgD or IgE. Particularly preferred is the longer and more flexible IgG3 hinge region.

2. Construction of Chick Avidin. The fragment containing the mature avidin polypeptide (minus the signal sequence) is generated by standard PCR using plasmid DNA as template and the following primers: (1) Sense 5' **CGGGGTACCGGAGGCGGTGGGTCAGCCAGAAAGTGCTCGC** 3' (SEQ ID NO:9) (linker is in bold; KpnI restriction site is bolded and underlined); and (14) Anti-sense 5' - CG**ACCGGT** CTCCTTCTGTGTGCGCAGGC - 3' (SEQ ID NO:10) (AgeI restriction site is in bold). The PCR product is gel purified according to standard procedure.

3. Assembled product. The above fragments "1" and "2" are joined by restriction digestion at the KpnI site followed by ligation employing standard protocols. The complete gene is designed for insertion in frame with a C terminal 6-His tag into the Drosophila expression vector pMT/V5-His (Invitrogen). This strategy is not limited to the use of this vector or a drosophila expression system. Specifically, the use of other expression vectors simply requires re-engineering of the restriction digestion sites flanking the complete construct (NotI and AgeI). The nucleotide and protein sequences of each construct below are shown without an inserted VH-gene. Any given VH-gene can be inserted between the BssHII (bold) and BstEII (dashed underline) sites.

The final sequence is for the F(ab) construct is: GCGGCCGCAAACC ATGGGATGGAGCTGTATCATCCTCTTCTTGGTAGCAACAGCTACAG GCGCGCATATGGTCACCGTCTCCTCAGCCTCCACCAAGGGCCCATC GGTCTTCCCCCTGGCACCCTCCTCCAAGAGCACCTCTGGGGGCACA GCGGCCCTGGGCTGCCTGGTCAAGGACTACTTCCCCGAACCGGTGA CGGTGTCGTGGAACTCAGGCGCCCTGACCAGCGGCGTGCACACCTT CCCGGCTGTCCTACAGTCCTCAGGACTCTACTCCCTCAGCAGCGTC GTGACCGTGCCCTCCAGCAGCTTGGGCACCCAGACCTACATCTGCA ACGTGAATCACAAGCCCAGCAACACCAAGGTGGACAAGAAA**GGA GGCGGTGGGTCAGGTACCGGAGGCGGTGGGTCA**GCCAGAAAGT GCTCGCTGACTGGGAAATGGACCAACGATCTGGGCTCCAACATGAC CATCGGGGCTGTGAACAGCAGAGGTGAATTCACAGGCACCTACAT CACAGCCGTAACAGCCACATCAAATGAGATCAAAGAGTCACCACT GCATGGGACACAAAACACCATCAACAAGAGGACCCAGCCCACCTT TGGCTTCACCGTCAATTGGAAGTTTTCAGAGTCCACCACTGTCTTCA CGGGCCAGTGCTTCATAGACAGGAATGGGAAGGAGGTCCTGAAGA CCATGTGGCTGCTGCGGTCAAGTGTTAATGACATTGGTGATGACTG GAAAGCTACCAGGGTCGGCATCAACATCTTCACTCGCCTGCGCACA CAGAAGGAGACCGGT*CATCATCACCATCACCAT*TGA (SEQ ID NO:11) (double underline: NotI restriction site; single underline: Signal sequence; bold: BssHII restriction site; dashed underline: BstEII restriction site; bold and underlined: linker; wavy underline: AgeI restriction site; italics and underlined: 6-His tag).

The F(ab) construct polypeptide sequence is: MGWSCIILFLVATATGA**HM**VTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKK**GGGGSGTGGGGS**ARKCSLTGK WTNDLGSNMTIGAVNSRGEFTGTYITAVTATSNEIKESPLHGTQNTINK RTQPTFGFTVNWKFSESTTVFTGQCFIDRNGKEVLKTMWLLRSSVNDI GDDWKATRVGINIFTRLRTQKETG*HHHHHH* (SEQ ID NO:12). The variable gene sequence is introduced at the upstream histidine in bold and the rest of the bolded amino acids are removed upon insertion of the variable gene sequence. The linker is bolded and underlined.

The F(ab'2) construct nucleotide sequence is: GCGGCCGCAAACC ATGGGATGGAGCTGTATCATCCTCTTCTTGGTAGCAACAGCTACAG GCGCGCATATGGTCACCGTCTCCTCAGCCTCCACCAAGGGCCCATC GGTCTTCCCCCTGGCACCCTCCTCCAAGAGCACCTCTGGGGGCACA GCGGCCCTGGGCTGCCTGGTCAAGGACTACTTCCCCGAACCGGTGA CGGTGTCGTGGAACTCAGGCGCCCTGACCAGCGGCGTGCACACCTT CCCGGCTGTCCTACAGTCCTCAGGACTCTACTCCCTCAGCAGCGTC GTGACCGTGCCCTCCAGCAGCTTGGGCACCCAGACCTACATCTGCA ACGTGAATCACAAGCCCAGCAACACCAAGGTGGACAAGAAAGTTG AGCCCAAATCTTGTGACAAAACTCACACATGCCCACCGTGCCCA**GG AGGCGGTGGGTCAGGTACCGGAGGCGGTGGGTCA**GCCAGAAA GTGCTCGCTGACTGGGAAATGGACCAACGATCTGGGCTCCAACATG ACCATCGGGGCTGTGAACAGCAGAGGTGAATTCACAGGCACCTAC ATCACAGCCGTAACAGCCACATCAAATGAGATCAAAGAGTCACCA CTGCATGGGACACAAAACACCATCAACAAGAGGACCCAGCCCACC TTTGGCTTCACCGTCAATTGGAAGTTTTCAGAGTCCACCACTGTCTT CACGGGCCAGTGCTTCATAGACAGGAATGGGAAGGAGGTCCTGAA GACCATGTGGCTGCTGCGGTCAAGTGTTAATGACATTGGTGATGAC TGGAAAGCTACCAGGGTCGGCATCAACATCTTCACTCGCCTGCGCA CACAGAAGGAGACCGGT*CATCATCACCATCACCAT*TGA (SEQ ID NO:13) (double underline: NotI restriction site; single underline: Signal sequence; bold: BssHII restriction site; dashed underline: BstEII restriction site; bold and underlined: linker; wavy underline: BamHI restriction site.)

The F(ab'2) construct polypeptide sequence is: MGWSCIILFLVATATGA**HM**VTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCP**GGGGSG TGGGGS**ARKCSLTGKWTNDLGSNMTIGAVNSRGEFTGTYITAVTATS NEIKESPLHGTQNTINKRTQPTFGFTVNWKFSESTTVFTGQCFIDRNGK EVLKTMWLLRSSVNDIGDDWKATRVGINIFTRLRTQKETG*HHHHHH* (SEQ ID NO:14). The variable gene sequence is introduced at the histidine in bold and the rest of the bolded amino acids are removed upon insertion of the variable gene sequence. The linker is bolded and underlined.

The full IgG1 construct nucleotide sequence is: GCGGCCGCAAACC ATGGGATGGAGCTGTATCATCCTCTTCTTGGTAGCAACAGCTACAG **GCGCGC**ATATGGTCACCGTCTCCTCAGCCTCCACCAAGGGCCCATC GGTCTTCCCCCTGGCACCCTCCTCCAAGAGCACCTCTGGGGGCACA GCGGCCCTGGGCTGCCTGGTCAAGGACTACTTCCCCGAACCGGTGA CGGTGTCGTGGAACTCAGGCGCCCTGACCAGCGGCGTGCACACCTT CCCGGCTGTCCTACAGTCCTCAGGACTCTACTCCCTCAGCAGCGTC GTGACCGTGCCCTCCAGCAGCTTGGGCACCCAGACCTACATCTGCA ACGTGAATCACAAGCCCAGCAACACCAAGGTGGACAAGAAAGTTG AGCCCAAATCTTGTGACAAAACTCACACATGCCCACCGTGCCCAGC ACCTGAACTCCTGGGGGGACCGTCAGTCTTCCTCTTCCCCCCAAAA CCCAAGGACACCCTCATGATCTCCCGGACCCCTGAGGTCACATGCG TGGTGGTGGACGTGAGCCACGAAGACCCTGAGGTCAAGTTCAACT GGTACGTGGACGGCGTGGAGGTGCATAATGCCAAGACAAAGCCGC GGGAGGAGCAGTACAACAGCACGTACCGTGTGGTCAGCGTCCTCA CCGTCCTGCACCAGGACTGGCTGAATGGCAAGGAGTACAAGTGCA AGGTCTCCAACAAAGCCCTCCCAGCCCCCATCGAGAAAACCATCTC CAAAGCCAAAGGGCAGCCCCGAGAACCACAGGTGTACACCCTGCC CCCATCCCGGGATGAGCTGACCAAGAACCAGGTCAGCCTGACCTGC CTGGTCAAAGGCTTCTATCCCAGCGACATCGCCGTGGAGTGGGAGA GCAATGGGCAGCCGGAGAACAACTACAAGACCACGCCTCCCGTGC TGGACTCCGACGGCTCCTTCTTCCTCTACAGCAAGCTCACCGTGGA CAAGAGCAGGTGGCAGCAGGGGAACGTCTTCTCATGCTCCGTGATG CATGAGGCTCTGCACAACCACTACACGCAGAAGAGCCTCTCCCTGT CTCCGGGTAAA**GGAGGCGGTGGGTCAGGTACCGGAGGCGGTGG GTCAG**CCAGAAAGTGCTCGCTGACTGGGAAATGGACCAACGATCT GGGCTCCAACATGACCATCGGGGCTGTGAACAGCAGAGGTGAATT CACAGGCACCTACATCACAGCCGTAACAGCCACATCAAATGAGAT CAAAGAGTCACCACTGCATGGGACACAAAACACCATCAACAAGAG GACCCAGCCCACCTTTGGCTTCACCGTCAATTGGAAGTTTTCAGAG TCCACCACTGTCTTCACGGGCCAGTGCTTCATAGACAGGAATGGGA AGGAGGTCCTGAAGACCATGTGGCTGCTGCGGTCAAGTGTTAATGA CATTGGTGATGACTGGAAAGCTACCAGGGTCGGCATCAACATCTTC ACTCGCCTGCGCACACAGAAGGAGACCGGT*CATCATCACCATCACCA T*TGA (SEQ ID NO:15) (double underline: NotI restriction site; single underline: Signal sequence; bold: BssHII restriction site; dashed underline: BstEII restriction site; bold and underlined: linker; wavy underline: BamHI restriction site).

The full IgG1 construct polypeptide sequence is: MGWSCIILFLVATATGA**HM**VTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGG PSVFLFPPKPKDTLMISRTPEVTCVWDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK**GGGGSGTGGGGS**ARKCSLTGKWTND LGSNMTIGAVNSRGEFTGTYITAVTATSNEIKESPLHGTQNTINKRTQP TFGFTVNWKFSESTTVFTGQCFIDRNGKEVLKTMWLLRSSVNDIGDD WKATRVGINIFTRLRTQKETG*HHHHHH* (SEQ ID NO:16). The variable gene sequence is introduced at the histidine in bold and the rest of the bolded amino acids are removed upon insertion of the variable gene sequence. The linker is bolded and underlined.

### EXAMPLE 5

### Chimeric kappa L chain-avidin

Employing the same strategy described above for fusion products with immunoglobulin heavy chain or heavy chain fragments, a chimeric kappa L chain is coupled in frame with avidin. Assembly takes place in a three-step process. In step one, PCR is employed to create the CL region preceded by a signal sequence for secretion and followed by a linker with an embedded KpnI restriction site. The kappa light chain constant region (CK) is PCR amplified from a previously described plasmid template with a pre-configured VL insertion site that allows for directional cloning of any immunoglobulin light chain variable region gene of interest at ApaLI and XhoI restriction sites (US20020123057, published Sept 5, 2002). In step two the avidin gene preceded by the linker with a KpnI restriction site is amplified exactly as described above for heavy chain fusion products. Finally in step three the two fragments are joined by restriction digestion at the KpnI site followed by ligation employing standard protocols. The modifications of primer sequences required for amplification of the immunoglobulin light chain with either kappa or lambda light chain constant regions will be apparent to those skilled in the art.

### EXAMPLE 6

### N terminal fusion proteins

Employing strategies similar to those described above, chimeric molecules are constructed where avidin is fused in frame with the amino terminus of the immunoglobulin heavy or light chain. These molecules are assembled in a 3 step process. In step 1, avidin, including the signal sequence, is PCR amplified from a plasmid template. This PCR modification adds a NotI site at the 5' end, and a portion of the gly,ser linker and a KPNI site at the 3' end. In step 2, the entire immunoglobulin heavy chain or light chain genes (variable and constant domains, or constant domain with restriction endonuclease sites for the insertion of a V gene), without the signal sequence, is PCR amplified. This PCR modification adds a portion of the gly,ser linker and a KpnI site at the 5' end, and an AgeI site at the 3' end. Finally, in step three, the two fragments are joined by restriction digestion at the KpnI site followed by ligation employing standard protocols. The modifications of primer sequences required for amplification of these genes and the construction of these molecules will be apparent to those skilled in the art.

### EXAMPLE 7

### Construction of extracellular Domain of CD 1d

1. Addition of Biotinylation Sequence to pMT/V5-His. The pMT/V5-His vector (Invitrogen) is a Drosophila expression vector. The purpose of this step is to modify this vector so that it contains a biotinylation sequence (BirA recognition sequence) in frame with a 6-His Tag at the C terminus. This allows for cloning of polypeptides of interest in frame with the BirA sequence and the 6-His Tag: (1) BirA Sense: 5'CCGGTggtggcggtctgaac gacatcttcgaggctcagaaaatcgaatggcacgaaT (SEQ ID NO:17); and (2) BirA Antisense: 5' CCGGAttcgtgccattcgattttctgagcctcgaagatgtcgttcagaccgccaccA (SEQ ID NO:18).

These 2 oligonucleotides are annealed together in vitro to create a double stranded oligo that contains an AGEI sticky end (bold) at both ends. This double stranded oligo is cloned into the AgeI site of pMT/V5-His, creating pMT/V5-BirA-His. The 3' AGEI site is destroyed by this cloning reaction, leaving a single AGEI site upstream and in frame with the BirA sequence.

2. Extracellular Domain of CD1d. Plasmid DNA or cDNA isolated from human bone marrow or spleen is used as a source of CD1d mRNA. The extracellular domain of CD1d is PCR amplified using the primers: (E1 sense): CACGGTACCGATATGGGGTGCCTGCTGTTTCTGC (SEQ ID NO:19) (KPNI site is underlined); and (E1 antisense): CAGACCGGT CCAGTAGAGGACGATGTCCTG (SEQ ID NO:20) Age I site is underlined.

The CD1d extracellular product is digested with Kpn I and Age I and cloned into the KpnI and AgeI sites of pMT/V5-BirA-His A vector (E.1). The C terminus of CD1d is in frame with the BirA sequence and the 6 His tag encoded by the vector, and contains an additional Thr and Gly that are encoded by the 5' AgeI site, and a Ser and Gly encoded by the 3' AgeI site.

The final sequence is: GGTACCGAT ATGGGGTGCCTGCTGTTTCTGCTGCTCTGGGCGCTGCTCCAGGCTTG GGGAAGCGCTGAAGTCCCGCAAAGGCTTTTCCCCCTCCGCTGCCTC CAGATCTCGTCCTTCGCCAATAGCAGCTGGACGCGCACCGACGGCT TGGCGTGGCTGGGGGAGCTGCAGACGCACAGCTGGAGCAACGACT CGGACACCGTCCGCTCTCTGAAGCCTTGGTCCCAGGGCACGTTCAG CGACCAGCAGTGGGAGACGCTGCAGCATATATTTCGGGTTTATCGA AGCAGCTTCACCAGGGACGTGAAGGAATTCGCCAAAATGCTACGC TTATCCTATCCCTTGGAGCTCCAGGTGTCCGCTGGCTGTGAGGTGC ACCCTGGGAACGCCTCAAATAACTTCTTCCATGTAGCATTTCAAGG AAAAGATATCCTGAGTTTCCAAGGAACTTCTTGGGAGCCAACCCAA GAGGCCCCACTTTGGGTAAACTTGGCCATTCAAGTGCTCAACCAGG ACAAGTGGACGAGGGAAACAGTGCAGTGGCTCCTTAATGGCACCT GCCCCCAATTTGTCAGTGGCCTCCTTGAGTCAGGGAAGTCGGAACT GAAGAAGCAAGTGAAGCCCAAGGCCTGGCTGTCCCGTGGCCCCAG TCCTGGCCCTGGCCGTCTGCTGCTGGTGTGCCATGTCTCAGGATTCT ACCCAAAGCCTGTATGGGTGAAGTGGATGCGGGGTGAGCAGGAGC AGCAGGGCACTCAGCCAGGGGACATCCTGCCCAATGCTGACGAGA CATGGTATCTCCGAGCAACCCTGGATGTGGTGGCTGGGGAGGCAGC TGGCCTGTCCTGTCGGGTGAAGCACAGCAGTCTAGAGGGCCAGGA CATCGTCCTCTACTGGACCGGT**GGTGGCGGTCTGAACGACATCTT CGAGGCTCAGAAAATCGAATGGCACGAA**TCCGGT*CATCATCACCA TCACCAT*TGA (SEQ ID NO:21) (single underline: KPNI site; double underline: Signal Sequence; wavy Underline: 5' AgeI site; bold: BirA sequence; dashed underline: 3' AgeI site; italics: 6 His Tag).

The translated polypeptide sequence is: MGCLLFLLLWALLOAWGSAEVPQRLFPLRCLQISSFANSSWTRTDGLA WLGELQTHSWSNDSDTVRSLKPWSQGTFSDQQWETLQHIFRVYRSSF TRDVKEFAKMLRLSYPLELQVSAGCEVHPGNASNNFFHVAFQGKDILS FQGTSWEPTQEAPLWVNLAIQVLNQDKWTRETVQWLLNGTCPQFVS GLLESGKSELKKQVKPKAWLSRGPSPGPGRLLLVCHVSGFYPKPVWV KWMRGEQEQQGTQPGDILPNADETWYLRATLDVVAGEAAGLSCRVK HSSLEGQDIVLYW**TGGGGLNDIFEAQKIEWHE**SG*HHHHHH* (SEQ ID NO:22).

### EXAMPLE 8

### Construction of β2 microglobulin

The fragment encoding the entire open reading frame of β2-microglobulin is generated by standard PCR using plasmid DNA as template. This fragment is cloned into a Drosophila expression vector such as pMT/V5-His. Cloning is designed so that there is not a 6-His Tag at the C terminal. This is easily accomplished by incorporating a "Stop" codon in the antisense primer immediately following the β2-microglobulin open reading frame. Methods to accomplish this construction are well known to those skilled in the art.

### EXAMPLE 9

### Production and purification of the chimeric antibody-avidin / CD1d-β2-microglobulin complex

Production of the antibody-avidin CD1d-β2-microglobulin complex is accomplished in 2 steps. In the first step, Drosophila S2 cells are transfected with the constructs encoding the chimeric antibody-avidin and the antibody light chain gene. High expressing clones are selected. At the same time a different aliquot of S2 cells is transfected with the CD1d construct and a construct encoding B2M and clones expressing high levels of the CD1d-β2-microglobulin complex are isolated.

A. Generation of Drosophila S2 cells stably transfected and producing antibody-avidin. Drosophila S2 cells are triply transfected with plasmids that encode 1) antibody-avidin (full IgG, F(ab), or F(ab')2; 2) antibody light chain; and 3) pCOHYGRO (Invitrogen). This plasmid confers resistance to the drug hygromycin. The triply transfected cells are selected with hygromycin and clones isolated. Expression of the recombinant antibody-avidin is induced in the cells by the addition of copper sulfate or cadmium chloride, and the cell supernatants are screened by ELISA using antibodies specific for antibody and avidin. The clones that express the highest amount of antibody-avidin complex are expanded and used to produce quantities of this molecule.

B. Production of the chimeric antibody-β2-microglobulin / CD1d complex. Different S2 cells are cotransfected with plasmids encoding CD1d (I.E), β2-microglobulin (I.F) and pCOBLAST (Invitrogen). pCOBLAST confers resistance to Blasticidin. Stable clones are selected using Blasticidin. Expression of the recombinant CD1d-β2-microglobulin complex is induced in the cells by the addition of copper sulfate or cadmium chloride, and the cell supernatants screened by ELISA using antibodies specific for B2M, and CD1d. The clones that express the highest amount of CD1d-β2-microglobulin complex are expanded and used to produce quantities of this molecule.

C. Purification. The highest expressing clone from (A) and (B) of Example 9 is expanded to 10+ L scale cultures in serum free X-press serum free media, and induced to express the recombinant molecules by the addition of copper sulfate or cadmium chloride to the media. For both the antibody-avidin and CD1d-B2M molecules, the complex containing media is concentrated and dialyzed against 0.15M sodium phosphate buffer (pH 7.4). The antibody-avidin and CD1d-B2M complexes are purified by Ni-NTA agarose chromatography, and then further purified by HPLC. BirA enzymatic biotinylation of CD1d is carried out according to the manufacturers protocol (Avidity, Inc). The remaining free biotin is removed by dialysis into phosphate buffered saline, pH 7.4. The biotinylated CD1d/B2M complex is incubated with a 3 fold molar excess of α-GalCer (CD1d ligand) overnight at room temperatue. Excess α-GalCer is removed by dialysis. The CD1d/B2M/α-GalCer complex is incubated with the antibody-avidin molecule for 4 hours at room temperature. Assembled complex is purified by HPLC.

### EXAMPLE 10

### Construction of IgG-B2M fusion protein complexed with soluble CD1d

An antibody fusion molecule is formed that contains all (IgG-B2M), or a portion (F(ab)-B2M and F(ab')2-B2M) of an antibody molecule fused in frame with the β2-microglobulin open reading frame. Assembly takes place in a three-step process. In step one, PCR is used to create the cloning cassette for VH, including the appropriate regions preceded by a signal sequence (SS) for secretion and followed by a linker with an embedded KpnI restriction site. In step two, PCR is used to amplify the β2-microglobulin gene. Finally, in step three, restriction digestion at the KpnI site followed by ligation is used to combine the two fragments. The IgG1 and β2-microglobulin proteins are separated by a 12 amino acid linker.

The chimeric Heavy Chain along with the complementing Ig Light Chain is produced in Drosophila cells along with the extracellular portion of CD1d. This trimeric complex is purified.

1. Construction of IgG1/VH cassette. Standard PCR is used to amplify the appropriate regions of IgG1 with a pre-configured VH insertion site from a previously described template. The PCR product is gel purified according to standard procedure. Specifically, an IgG1 construct that allows for insertion of a variable gene of interest through BssHI and BstEII restriction sites is used. This construct is described elsewhere (U.S. Appl. Publ. No. 2003/0104402, published June 5, 2003) and is available as template for PCR using the following primers.

For the F(ab) construct: (7) Sense 5' AATT**GCGGCCGC**AAA **CCATGG**GATGGAGCTGTATCATC 3' (SEQ ID NO:23) (NotI and NcoI sites in bold); and (8) Anti-sense 5' **CGGGGTACC TGACCCACCGCCTCC**TTTCTTGTCCACCTTGGTGTT 3' (SEQ ID NO:24) (linker is in bold; KpnI site is bolded and underlined). For the F(ab') 2 construct: (7) Sense 5' AATT**GCGGCCGCAAACCATGG** GATGGAGCTGTATCATC 3' (SEQ ID NO:25) (NotI and NcoI sites in bold); and (8) Anti-sense 5' **CGGGGTACCTGACCCACCGCCTCC** TGGGCACGGTGGGCATGTGTG 3' (SEQ ID NO:26) (linker is in bold; KpnI site is bolded and underlined).

For the full length IGg1 construct: (7) Sense 5' AATT**GCGGCCGC** AAA**CCATGG**GATGGAGCTGTATCATC 3' (SEQ ID NO:27) (NotI and NcoI sites in bold); and (8) Anti-sense 5' **CGGGGTACC TGACCCACCGCCTCC**TTTACCCGGAGACAGGGAGAG 3' (SEQ ID NO:28) (linker is in bold; KpnI site is bolded and underlined).

In other embodiments, the CH1 region derives from other immunoglobulin isotypes, including IgG2, IgG3, IgG4, IgA, IgM, IgD or IgE. Particularly preferred is the longer and more flexible IgG3 hinge region.

2. Construction of β2-microglobulin. The fragment is generated by standard PCR using plasmid DNA as template and the following primers: (1) Sense 5' **CGGGGTACCGGAGGCGGTGGGTCA** ATCGAGCGTACTCCA 3' (SEQ ID NO: 29) (linker is in bold; KpnI restriction site is bolded and underlined); and (14) Anti-sense 5' - CG**ACCGGT**CATGTCTCGATCCCACTT - 3' (SEQ ID NO:30) (AgeI restriction site is in bold). The PCR product is gel purified according to standard procedure.

3. Assembled Chimeric IgG1-β2-microglobulin product. The above fragments "1" and "2" are joined by restriction digestion at the KpnI site followed by ligation employing standard protocols. The complete gene is designed for insertion in frame with a C terminal 6-His tag into the Drosophila expression vector pMT/VS-His (Invitrogen). This strategy is not limited to the use of this vector or a drosophila expression system. Specifically, the use of other expression vectors simply requires re-engineering of the restriction digestion sites flanking the complete construct (NotI and AgeI). The nucleotide and protein sequence below are presented without an inserted VH-gene. Any given VH-gene can be inserted between the BssHII (bold) and BstEII (dashed underline) sites.

The nucleotide sequence of the chimeric F(ab)-β2 microglobulin is: GCGGCCGCAAACCATGGGATGGAGCTGTATCATCCTCTTCTTGGTA GCAACAGCTACAG**GCGCGC**ATATGGTCACCGTCTCCTCAGCCTCC ACCAAGGGCCCATCGGTCTTCCCCCTGGCACCCTCCTCCAAGAGCA CCTCTGGGGGCACAGCGGCCCTGGGCTGCCTGGTCAAGGACTACTT CCCCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTCGTGACCGTGCCCTCCAGCAGCTTGGGCACCCA GACCTACATCTGCAACGTGAATCACAAGCCCAGCAACACCAAGGT GGACAAGAAA**GGAGGCGGTGGGTCAGGTACCGGAGGCGGTGG GTCA**ATCCAGCGTACTCCAAAGATTCAGGTTTACTCACGTCATCCA GCAGAGAATGGAAAGTCAAATTTCCTGAATTGCTATGTGTCTGGGT TTCATCCATCCGACATTGAAGTTGACTTACTGAAGAATGGAGAGAG AATTGAAAAAGTGGAGCATTCAGACTTGTCTTTCAGCAAGGACTGG TCTTTCTATCTCTTGTACTACACTGAATTCACCCCCACTGAAAAAGA TGAGTATGCCTGCCGTGTGAACCATGTGACTTTGTCACAGCCCAAG ATAGTTAAGTGGGATCGAGACATGACCGGT*CATCATCACCATCACCA T*TGA - 780 (SEQ ID NO:31) (double underline: NotI restriction site; single underline: Signal sequence; bold: BssHII restriction site; dashed underline: BstEII restriction site; bold and underlined: linker; wavy underline: AgeI restriction site; italics and underlined: His Tag).

The nucleotide sequence of the chimeric F(ab)-β2 microglobulin is: MGWSCIILFLVATATGAHMVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKK**GGGGSGTGGGGS**IQRTPKIQVY SRHPAENGKSNFLNCYVSGFHPSDIEVDLLKNGERIEKVEHSDLVFSKD WSFYLLYYTEFTPTEKDEYACRVNHVTLSQPKIVKWDRDMT*GHHHH HH* (SEQ ID NO:32). The variable gene sequence is introduced at the upstream histidine in bold and the rest of the bolded amino acids are removed upon insertion of the variable gene sequence. The linker is bolded and underlined.

The nucleotide sequence of the chimeric F(ab')2-β2-microglobulin is: GCGGCCGCAAACCATGGGATGGAGCTGTATCATCCTCTTCTTGGTA GCAACAGCTACAG**GCGCGC**ATATGGTCACCGTCTCCTCAGCCTCC ACCAAGGGCCCATCGGTCTTCCCCCTGGCACCCTCCTCCAAGAGCA CCTCTGGGGGCACAGCGGCCCTGGGCTGCCTGGTCAAGGACTACTT CCCCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTCGTGACCGTGCCCTCCAGCAGCTTGGGCACCCA GACCTACATCTGCAACGTGAATCACAAGCCCAGCAACACCAAGGT GGACAAGAAAGTTGAGCCCAAATCTTGTGACAAAACTCACACATG CCCACCGTGCCCAGGA**GGCGGTGGGTCAGGTACCGGAGGCGGT** GGGTCAATCCAGCGTACTCCAAAGATTCAGGTTTACTCACGTCATC CAGCAGAGAATGGAAAGTCAAATTTCCTGAATTGCTATGTGTCTGG GTTTCATCCATCCGACATTGAAGTTGACTTACTGAAGAATGGAGAG AGAATTGAAAAAGTGGAGCATTCAGACTTGTCTTTCAGCAAGGACT GGTCTTTCTATCTCTTGTACTACACTGAATTCACCCCCACTGAAAAA GATGAGTATGCCTGCCGTGTGAACCATGTGACTTTGTCACAGCCCA AGATAGTTAAGTGGGATCGAGACATGACCGGTC*ATCATCACCATCAC CAT*TGA (SEQ ID NO:33) (double underline: NotI restriction site; single underline: signal sequence; bold: BssHII restriction site; dashed underline: BstEII restriction site; bold and underlined: linker; wavy underline: AgeI restriction site; italics and underlined: His Tag).

The polypeptide sequence of the chimeric F(ab')2-β2-microglobulin is: MGWSCIILFLVATATGA**HM**VTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCPGGGGSG TGGGGSIQRTPKIQVYSRHPAENGKSNFLNCYVSGFHPSDIEVDLLKN GERIEKVEHSDLVFSKDWSFYLLYYTEFTPTEKDEYACRVNHVTLSQP KIVKWDRDMTG*HHHHHH* (SEQ ID NO:34). The variable gene sequence is introduced at the histidine in bold and the rest of the bolded amino acids are removed upon insertion of the variable gene sequence. The linker is bolded and underlined.

The nucleotide sequence of the chimeric full IgG1-β2 microglobulin is: GCGGCCGCAAACCATGGGATGGAGCTGTATCATCCTCTTCTTG GTAGCAACAGCTACAG**GCGCGC**ATATGGTCACCGTCTCCTCAGCC TCCACCAAGGGCCCATCGGTCTTCCCCCTGGCACCCTCCTCCAAGA GCACCTCTGGGGGCACAGCGGCCCTGGGCTGCCTGGTCAAGGACTA CTTCCCCGAACCGGTGACGGTGTCGTGGAACTCAGGCGCCCTGACC AGCGGCGTGCACACCTTCCCGGCTGTCCTACAGTCCTCAGGACTCT ACTCCCTCAGCAGCGTCGTGACCGTGCCCTCCAGCAGCTTGGGCAC CCAGACCTACATCTGCAACGTGAATCACAAGCCCAGCAACACCAA GGTGGACAAGAAAGTTGAGCCCAAATCTTGTGACAAAACTCACAC ATGCCCACCGTGCCCAGCACCTGAACTCCTGGGGGGACCGTCAGTC TTCCTCTTCCCCCCAAAACCCAAGGACACCCTCATGATCTCCCGGA CCCCTGAGGTCACATGCGTGGTGGTGGACGTGAGCCACGAAGACC CTGAGGTCAAGTTCAACTGGTACGTGGACGGCGTGGAGGTGCATA ATGCCAAGACAAAGCCGCGGGAGGAGCAGTACAACAGCACGTACC GTGTGGTCAGCGTCCTCACCGTCCTGCACCAGGACTGGCTGAATGG CAAGGAGTACAAGTGCAAGGTCTCCAACAAAGCCCTCCCAGCCCC CATCGAGAAAACCATCTCCAAAGCCAAAGGGCAGCCCCGAGAACC ACAGGTGTACACCCTGCCCCCATCCCGGGATGAGCTGACCAAGAAC CAGGTCAGCCTGACCTGCCTGGTCAAAGGCTTCTATCCCAGCGACA TCGCCGTGGAGTGGGAGAGCAATGGGCAGCCGGAGAACAACTACA AGACCACGCCTCCCGTGCTGGACTCCGACGGCTCCTTCTTCCTCTAC AGCAAGCTCACCGTGGACAAGAGCAGGTGGCAGCAGGGGAACGTC TTCTCATGCTCCGTGATGCATGAGGCTCTGCACAACCACTACACGC AGAAGAGCCTCTCCCTGTCTCCGGGTAAA**GGAGGCGGTGGGTCA GGTACCGGAGGCGGTGGGTCA**ATCCAGCGTACTCCAAAGATTCA GGTTTACTCACGTCATCCAGCAGAGAATGGAAAGTCAAATTTCCTG AATTGCTATGTGTCTGGGTTTCATCCATCCGACATTGAAGTTGACTT ACTGAAGAATGGAGAGAGAATTGAAAAAGTGGAGCATTCAGACTT GTCTTTCAGCAAGGACTGGTCTTTCTATCTCTTGTACTACACTGAAT TCACCCCCACTGAAAAAGATGAGTATGCCTGCCGTGTGAACCATGT GACTTTGTCACAGCCCAAGATAGTTAAGTGGGATCGAGACATGACC GGT*CATCATCACCATCACCAI*TGA (SEQ ID NO:35) (double underline: NotI restriction site; single underline: Signal sequence; bold: BssHII restriction site; dashed underline: BstEII restriction site; bold and underlined: linker; wavy underline: AgeIrestriction site; and italics and underlined: His Tag).

The polypeptide sequence of the chimeric full IgG1-β2 microglobulin is: MGWSCIILFLVATATGAHMVTVSSASTKGPSVFPLAPSSKSTSGG TAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVMHEALHNHYTQKSLSLSPGK**GGGGSGTGGGGS**IQRTPKIQVYSRH PAENGKSNFLNCYVSGFHPSDIEVDLLKNGERIEKVEHSDLVFSKDWS FYLLYYTEFTPTEKDEYACRVNHVTLSQPKIVKWDRDMTG*HHHHHH* (SEQ ID NO:36). The variable gene sequence is introduced at the histidine in bold and the rest of the bolded amino acids are removed upon insertion of the variable gene sequence. The linker is bolded and underlined.

### EXAMPLE 11

### Chimeric kappa L chain-β2 microglobulin

Employing the same strategy described above for fusion products with immunoglobulin heavy chain or heavy chain fragments, a chimeric kappa L chain is coupled in frame with β2-microglobulin. Assembly takes place in a three-step process. In step one, PCR is employed to create the CL region preceded by a signal sequence for secretion and followed by a linker with an embedded KpnI restriction site. The kappa light chain constant region (CK) is PCR amplified from a previously described plasmid template with a pre-configured VL insertion site that allows for directional cloning of any immunoglobulin light chain variable region gene of interest at ApaLI and XhoI restriction (U.S. Appl. Publ. No. 2003/0104402, published June 5, 2003). In step two, the β2-microglobulin gene preceded by the linker with a KpnI restriction site is amplified exactly as described above for heavy chain fusion products. Finally, in step three, the two fragments are joined by restriction digestion at the KpnI site followed by ligation employing standard protocols. The modifications of primer sequences required for amplification of the immunoglobulin light chain with either kappa or lambda light chain constant regions will be apparent to those skilled in the art.

### EXAMPLE 12

### N terminal fusion proteins

Employing strategies similar to those described above, chimeric molecules are constructed where β2 microglobulin is fused in frame with the amino terminus of the immunoglobulin heavy or light chain. These molecules are assembled in a 3 step process. In step 1, the β2 microglobulin gene, including the signal sequence, is PCR amplified from a plasmid template. This PCR modification adds a NotI site at the 5' end, and a portion of the gly, ser linker and a KPNI site at the 3' end. In step 2, the entire immunoglobulin heavy chain or light chain genes (variable and constant domains, or constant domain with restriction endonuclease sites for the insertion of a V gene), without the signal sequence, is PCR amplified. This PCR modification adds a portion of the gly,ser linker and a KpnI site at the 5' end, and an AgeI site at the 3' end. Finally, in step three, the two fragments are joined by restriction digestion at the KpnI site followed by ligation employing standard protocols. The modifications of primer sequences required for amplification of these genes and the construction of these molecules will be apparent to those skilled in the art.

### EXAMPLE 13

### Construction of extracellular Domain of CD1d. cDNA isolated from human bone marrow or spleen is used as a source of CD1d mRNA

The extracellular domain of CD1d is PCR amplified using the primers: (E1 sense): CACGGTACCGATATGGGGTGCCTGCTGTTTCTGC (SEQ ID NO:37) (KPNI site is underlined) and (E1 antisense): CAGACCGGTCCAGTAGAGGACGATGTCCTG (SEQ ID NO:38) (Age I site is underlined).

The CD1d extracellular product is digested with Kpn I and Age I and cloned into the KpnI and AgeI sites of pMT/VS-His A vector (Invitrogen). The C terminus of CD1d is in frame with a 6 His tag encoded by the vector, and contains an additional Thr and gly that are encoded by the AgeI site. The resulting sequence is: GGTACCGATATGGGGTGCCTGCTGTTTCTG CTGCTCTGGGCGCTCCTCCAGGCTTGGGGAAGCGCTGAAGTCCCGC AAAGGCTTTTCCCCCTCCGCTGCCTCCAGATCTCGTCCTTCGCCAAT AGCAGCTGGACGCGCACCGACGGCTTGGCGTGGCTGGGGGAGCTG CAGACGCACAGCTGGAGCAACGACTCGGACACCGTCCGCTCTCTGA AGCCTTGGTCCCAGGGCACGTTCAGCGACCAGCAGTGGGAGACGC TGCAGCATATATTTCGGGTTTATCGAAGCAGCTTCACCAGGGACGT GAAGGAATTCGCCAAAATGCTACGCTTATCCTATCCCTTGGAGCTC CAGGTGTCCGCTGGCTGTGAGGTGCACCCTGGGAACGCCTCAAATA ACTTCTTCCATGTAGCATTTCAAGGAAAAGATATCCTGAGTTTCCA AGGAACTTCTTGGGAGCCAACCCAAGAGGCCCCACTTTGGGTAAAC TTGGCCATTCAAGTGCTCAACCAGGACAAGTGGACGAGGGAAACA GTGCAGTGGCTCCTTAATGGCACCTGCCCCCAATTTGTCAGTGGCC TCCTTGAGTCAGGGAAGTCGGAACTGAAGAAGCAAGTGAAGCCCA AGGCCTGGCTGTCCCGTGGCCCCAGTCCTGGCCCTGGCCGTCTGCT GCTGGTGTGCCATGTCTCAGGATTCTACCCAAAGCCTGTATGGGTG AAGTGGATGCGGGGTGAGCAGGAGCAGCAGGGCACTCAGCCAGGG GACATCCTGCCCAATGCTGACGAGACATGGTATCTCCGAGCAACCC TGGATGTGGTGGCTGGGGAGGCAGCTGGCCTGTCCTGTCGGGTGAA GCACAGCAGTCTAGAGGGCCAGGACATCGTCCTCTACTGGACCGGT *CATCATCACCATCACCATT*GA (SEQ ID NO:39).

The translated polypeptide sequence is: MGCLLFLLLWALLQAWGS AEVPQRLFPLRCLQISSFANSSWTRTDGLAWLGELQTHSWSNDSDTVR SLKPWSQGTFSDQQWETLQHIFRVYRSSFTRDVKEFAKMLRLSYPLEL QVSAGCEVHPGNASNNFFHVAFQGKDILSFQGTSWEPTQEAPLWVNL AIQVLNQDKWTRETVQWLLNGTCPQFVSGLLESGKSELKKQVKPKA WLSRGPSPGPGRLLLVCHVSGFYPKPVWVKWMRGEQEQQGTQPGDIL PNADETWYLRATLDVVAGEAAGLSCRVKHSSLEGQDIVLYW*TGHHH HHH* (SEQ ID NO:40).

### EXAMPLE 14

### Production and purification of the chimeric antibody-β2-microglobulin / CD1d complex

Production of the antibody-β2-microglobulin / CD1d complex is done in 2 steps. In the first step, Drosophila S2 cells are transfected with the constructs encoding the chimeric antibody-b2M and the antibody light chain gene. High expressing clones are selected and then transfected with the CD1d construct and clones expressing high levels of the antibody-β2-microglobulin /CD1d complex are isolated.

A. Generation of Drosophila S2 cells stably transfected and producing antibody-β2-microglobulin. Drosophila S2 cells are triply transfected with plasmids that encode 1) antibody-B2M (full IgG, F(ab), or F(ab')2); 2) antibody light chain; and 3) pCOHYGRO (Invitrogen). This plasmid confers resistance to the drug hygromycin. The triply transfected cells are selected with hygromycin and clones isolated. Expression of the recombinant anibody-b2M are induced in the cells by the addition of copper sulfate or cadmium chloride, and the cell supernatants screened by ELISA using antibodies specific for antibody and B2M.

B. Production of the chimeric antibody-β2-microglobulin / CD1d complex. The highest antibody-B2M producing clones from (A) is cotransfected with plasmids encoding CD1d (I.E) and pCOBLAST (Invitrogen). pCOBLAST confers resistance to Blasticidin. Stable clones are selected using Hygromycin and Blasticidin. Expression of the recombinant antibody-b2M/CD1d complex is induced in the cells by the addition of copper sulfate or cadmium chloride, and the cell supernatants screened by ELISA using antibodies specific for Ig, B2M, and CD1d. The clones that express the highest amount of antibody-b2M/CD1d complex are expanded and used to produce quantities of this molecule.

C. Purification. The highest expressing clone from (B) is expanded to 10+ L scale cultures in serum free X-press media, and induced to express this complex by the addition of copper sulfate or cadmium chloride to the media. The complex containing media is concentrated and dialyzed against 0.15M sodium phosphate buffer (pH 7.4). The antibody-b2M/CD1d complex is purified by Ni-NTA agarose chromatography, and then further purified by HPLC. The purified complex is then incubated with a 3 fold molar excess of α-GalCer (CD1d ligand) overnight at room temperature. Excess α-GalCer is removed by dialysis.

### EXAMPLE 15

### Construction of IgG-CD1d fusion protein

An antibody fusion molecule is formed that contains all (IgG-CD1d), or a portion (F(ab)-CD1d and F(ab')2-CD1d) of an antibody molecule fused in frame with the extracellular domain of CD1d. Assembly takes place in a three-step process. In step one, PCR is used to create the cloning cassette for VH, including the appropriate IgG1 regions preceded by a signal sequence (SS) for secretion and followed by a linker with an embedded KpnI restriction site. In step two, PCR is used to amplify the extracellular region of the CD1d gene. Finally, in step three, restriction digestion at the KpnI site followed by ligation is used to combine the two fragments. The IgG1 and CD1d proteins are separated by a 12 amino acid linker.

The chimeric heavy chain along with the complementing Ig Light Chain is produced in Drosophila cells along with human β2-microglobulin. This trimeric complex is purifed.

1. Construction of VH cassette. Standard PCR is used to amplify the appropriate portion of IgG1 with a pre-configured VH insertion site from a previously described template which is a human IgG1 construct that allows for insertion of a variable gene of interest through BssHI and BstEII restriction sites. The PCR product is gel purified according to standard procedure. This construct is described elsewhere (U.S. Appl. Publ. No. 2003/0104402, published June 5, 2003) and is available as template for PCR using the following primers.

For the F(ab) fragment: (7) Sense 5' AATTGCGGCCGCAAA CCATGGGATGGAGCTGTATCATC 3' (SEQ ID NO:41) (NotI and NcoI sites in bold); and (8) Anti-sense 5' **CGGGGTACC TGACCCACCGCCTCC**TTTCTTGTCCAGCTTGGTGTT 3' (SEQ ID NO:42) (linker is in bold; KpnI site is bolded and underlined).

For the F(ab')2 fragment: (7) Sense 5' AATTGCGGCCGCAAACCATGGG ATGGAGCTGTATCATC 3' (SEQ ID NO:43) (NotI and NcoI sites in bold); and (8) Anti-sense 5' **CGGGGTACCTGACCCACCGCCTCC**TGGGCACGGTGGGCATGTGT G 3' (SEQ ID NO:44) (linker is in bold; KpnI site is bolded and underlined).

For the full IgGl: (7) Sense 5' AATTGCGGCCGCAAACCATGG GATGGAGCTGTATCATC 3' (SEQ ID NO:45) (NotI and NcoI sites in bold); and (8) Anti-sense 5' **CGGGGTACCTGACCCACCGCCTCC** TTTACCCGGAGACAGGGAGAG 3' (SEQ ID NO:46) (linker is in bold; KpnIsite is bolded and underlined).

In other embodiments, the antibody regions derive from other immunoglobulin isotypes, including IgG2, IgG3, IgG4, IgA, IgM, IgD or IgE. Particularly preferred is the longer and more flexible IgG3 hinge region.

2. Construction of extracellular CD1d. The fragment is generated by standard PCR using plasmid DNA as template and the following primers: (1) Sense 5' **CGGGGTACCGGAGGCGGTGGGTCA** GTCCCGCAAAGGCTTTTC 3' (SEQ ID NO:47) (linker is in bold; KpnI restriction site is bolded and underlined); and (14) Anti-sense 5' - CG **ACCGGT**CCAGTAGAGGACGATGTCCTG - 3' (SEQ ID NO:48) (AgeI restriction site is in bold). The PCR product is gel purified according to standard procedure.

3. Assembled chimeric antibody-CD1d product. The above fragments "1" and "2" are joined by restriction digestion at the KpnI site followed by ligation employing standard protocols. The complete gene is designed for insertion in frame with a C terminal 6-His tag into the Drosophila expression vector pMT/VS-His (Invitrogen). This strategy is not limited to the use of this vector or a drosophila expression system. Specifically, the use of other expression vectors simply requires re-engineering of the restriction digestion sites flanking the complete construct (NotI and AgeI). The nucleotide and protein sequences below are presented without an inserted VH-gene. Any given VH-gene can be inserted between the BssHII (bold) and BstEII (dashed underline) sites.

The final nucleotide sequence of the chimeric F(ab)-CD1d product is: GCGGGCGCAAACCATGGGATGGAGCTGTATCATCCTCTTCTTGGTA GCAACAGCTACAG**GCGCGC**ATATGGTCACCGTCTCCTCAGCCTCC ACCAAGGGCCCATCGGTCTTCCCCCTGGCACCCTCCTCCAAGAGCA CCTCTGGGGGCACAGCGGCCCTGGGCTGCCTGGTCAAGGACTACTT CCCCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTCGTGACCGTGCCCTCCAGCAGCTTGGGCACCCA GACCTACATCTGCAACGTGAATCACAAGCCCAGCAACACCAAGGT GGACAAGAAA**GGAGGCGGTGGGTCAGGTACCGGAGGCGGTGG** GTCAGTCCCGCAAAGGCTTTTCCCCCTCCGCTGCCTCCAGATCTCG TCCTTCGCCAATAGCAGCTGGACGCGCACCGACGGCTTGGCGTGGC TGGGGGAGCTGCAGACGCACAGCTGGAGCAACGACTCGGACACCG TCCGCTCTCTGAAGCCTTGGTCCCAGGGCACGTTCAGCGACCAGCA GTGGGAGACGCTGCAGCATATATTTCGGGTTTATCGAAGCAGCTTC ACCAGGGACGTGAAGGAATTCGCCAAAATGCTACGCTTATCCTATC CCTTGGAGCTCCAGGTGTCCGCTGGCTGTGAGGTGCACCCTGGGAA CGCCTCAAATAACTTCTTCCATGTAGCATTTCAAGGAAAAGATATC CTGAGTTTCCAAGGAACTTCTTGGGAGCCAACCCAAGAGGCCCCAC TTTGGGTAAACTTGGCCATTCAAGTGCTCAACCAGGACAAGTGGAC GAGGGAAACAGTGCAGTGGCTCCTTAATGGCACCTGCCCCCAATTT GTCAGTGGCCTCCTTGAGTCAGGGAAGTCGGAACTGAAGAAGCAA GTGAAGCCCAAGGCCTGGCTGTCCCGTGGCCCCAGTCCTGGCCCTG GCCGTCTGCTGCTGGTGTGCCATGTCTCAGGATTCTACCCAAAGCC TGTATGGGTGAAGTGGATGCGGGGTGAGCAGGAGCAGCAGGGCAC TCAGCCAGGGGACATCCTGCCCAATGCTGACGAGACATGGTATCTC CGAGCAACCCTGGATGTGGTGGCTGGGGAGGCAGCTGGCCTGTCCT GTCGGGTGAAGCACAGCAGTCTAGAGGGCCAGGACATCGTCCTCT ACTGGACCGGT*CATCATCACCATCACCAT*TGA (SEQ ID NO:49) (double underline: NotI restriction site; single underline: Signal sequence; bold: BssHII restriction site; dashed underline: BstEII restriction site; bold and underlined: linker; wavy underline: AgeIrestriction site; italics and underlined: His Tag).

The final polypeptide sequence of the chimeric F(ab)-CD1d product is: MGWSCIILFLVATATGAHMVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKK**GGGGSGTGGGGS**VPQRLFPLRC LQISSFANSSWTRTDGLAWLGELQTHSWSNDSDTVRSLKPWSQGTFS DQQWETLQHIFRVYRSSFTRDVKEFAKMLRLSYPLELQVSAGCEVHP GNASNNFFHVAFQGKDILSFQGTSWEPTQEAPLWVNLAIQVLNQDKW TRETVQWLLNGTCPQFVSGLLESGKSELKKQVKPKAWLSRGPSPGPG RLLLVCHVSGFYPKPVWVKWMRGEQEQQGTQPGDILPNADETWYLR ATLDVVAGEAAGLSCRVKHSSLEGQDIVLYWTG*HHHHHH* (SEQ ID NO:50). The variable gene sequence is introduced at the upstream histidine in bold and the rest of the bolded amino acids are removed upon insertion of the variable gene sequence. The linker is bolded and underlined.

The final nucleotide sequence of the chimeric F(ab')2-CD1d product is: GCGGCCGCAAACCATGGGATGGAGCTGTATCATCCTCTTCTTG GTAGCAACAGCTACAGGCGCGCATATGGTCACCGTCTCCTCAGCCT CCACCAAGGGCCCATCGGTCTTCCCCCTGGCACCCTCCTCCAAGAG CACCTCTGGGGGCACAGCGGCCCTGGGCTGCCTGGTCAAGGACTAC TTCCCCGAACCGGTGACGGTGTCGTGGAACTCAGGCGCCCTGACCA GCGGCGTGCACACCTTCCCGGCTGTCCTACAGTCCTCAGGACTCTA CTCCCTCAGCAGCGTCGTGACCGTGCCCTCCAGCAGCTTGGGCACC CAGACCTACATCTGCAACGTGAATCACAAGCCCAGCAACACCAAG GTGGACAAGAAAGTTGAGCCCAAATCTTGTGACAAAACTCACACA TGCCCACCGTGCCCA**GGAGGCGGTGGGTCAGGTACCGGAGGCG GTGGGTCA**GTCCCGCAAAGGCTTTTCCCCCTCCGCTGCCTCCAGAT CTCGTCCTTCGCCAATAGCAGCTGGACGCGCACCGACGGCTTGGCG TGGCTGGGGGAGCTGCAGACGCACAGCTGGAGCAACGACTCGGAC ACCGTCCGCTCTCTGAAGCCTTGGTCCCAGGGCACGTTCAGCGACC AGCAGTGGGAGACGCTGCAGCATATATTTCGGGTTTATCGAAGCAG CTTCACCAGGGACGTGAAGGAATTCGCCAAAATGCTACGCTTATCC TATCCCTTGGAGCTCCAGGTGTCCGCTGGCTGTGAGGTGCACCCTG GGAACGCCTCAAATAACTTCTTCCATGTAGCATTTCAAGGAAAAGA TATCCTGAGTTTCCAAGGAACTTCTTGGGAGCCAACCCAAGAGGCC CCACTTTGGGTAAACTTGGCCATTCAAGTGCTCAACCAGGACAAGT GGACGAGGGAAACAGTGCAGTGGCTCCTTAATGGCACCTGCCCCC AATTTGTCAGTGGCCTCCTTGAGTCAGGGAAGTCGGAACTGAAGAA GCAAGTGAAGCCCAAGGCCTGGCTGTCCCGTGGCCCCAGTCCTGGC CCTGGCCGTCTGCTGCTGGTGTGCCATGTCTCAGGATTCTACCCAA AGCCTGTATGGGTGAAGTGGATGCGGGGTGAGCAGGAGCAGCAGG GCACTCAGCCAGGGGACATCCTGCCCAATGCTGACGAGACATGGT ATCTCCGAGCAACCCTGGATGTGGTGGCTGGGGAGGCAGCTGGCCT GTCCTGTCGGGTGAAGCACAGCAGTCTAGAGGGCCAGGACATCGT CCTCTACTGGACCGGT*CATCATCACCATCACCAT*TGA (SEQ ID NO:51) (double underline: NotI restriction site; single underline: Signal sequence; bold: BssHII restriction site; dashed underline: BstEII restriction site; bold and underlined: linker; wavy underline: AgeIrestriction site; and italics and underlined: 6 His tag).

The final polypeptide sequence of the chimeric F(ab')2-CD1d product is: MGWSCIILFLVATATGAHMVTVSSASTKGPSVFPLAPSSKSTSGG TAALGCLVKDYFPEPVTVSWNSGALTSGVHTFPAVLQSSGLYSLSSVV TVPSSSLGTQTYICNVNHICPSNTKVDKKVEPKSCDKTHTCPPCP**GGGG SGTGGGGS**VPQRLFPLRCLQISSFANSSWTRTDGLAWLGELQTHSWS NDSDTVRSLKPWSQGTFSDQQWETLQHIFRVYRSSFTRDVKEFAKML RLSYPLELQVSAGCEVHPGNASNNFFHVAFQGKDILSFQGTSWEPTQE APLWVNLAIQVLNQDKWTRETVQWLLNGTCPQFVSGLLESGKSELKK QVKPKAWLSRGPSPGPGRLLLVCHVSGFYPKPVWVKWMRGEQEQQG TQPGDILPNADETWYLRATLDVVAGEAAGLSCRVKHSSLEGQDIVLY WTG*HHHHHH* (SEQ ID NO:52). The variable gene sequence is introduced at the upstream histidine in bold and the rest of the bolded amino acids are removed upon insertion of the variable gene sequence. The linker is bolded and underlined.

The final nucleotide sequence of the chimeric IgG1-CD1d product is: GCGGCCGCAAACCATGGGATGGAGCTGTATCATCCTCTTCTTGGTA GCAACAGCTACAGGCGCGCATATGGTCACCGTCTCCTCAGCCTCG ACCAAGGGCCCATCGGTCTTCCCCCTGGCACCCTCCTCCAAGAGCA CCTCTGGGGGCACAGCGGCCCTGGGCTGCCTGGTCAAGGACTACTT CCCCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTCGTGACCGTGCCCTCCAGCAGCTTGGGCACCCA GACCTACATCTGCAACGTGAATCACAAGCCCAGCAACACCAAGGT GGACAAGAAAGTTGAGCCCAAATCTTGTGACAAAACTCACACATG CCCACCGTGCCCAGCACCTGAACTCCTGGGGGGACCGTCAGTCTTC CTCTTCCCCCCAAAACCCAAGGACACCCTCATGATCTCCCGGACCC CTGAGGTCACATGCGTGGTGGTGGACGTGAGCCACGAAGACCCTG AGGTCAAGTTCAACTGGTACGTGGACGGCGTGGAGGTGCATAATG CCAAGACAAAGCCGCGGGAGGAGCAGTACAACAGCACGTACCGTG TGGTCAGCGTCCTCACCGTCCTGCACCAGGACTGGCTGAATGGCAA GGAGTACAAGTGCAAGGTCTCCAACAAAGCCCTCCCAGCCCCCATC GAGAA.A.ACCATCTCCAAAGCCAAAGGGCAGCCCCGAGAACCACAG GTGTACACCCTGCCCCCATCCCGGGATGAGCTGACCAAGAACCAGG TCAGCCTGACCTGCCTGGTCAAAGGCTTCTATCCCAGCGACATCGC CGTGGAGTGGGAGAGCAATGGGCAGCCGGAGAACAACTACAAGAC CACGCCTCCCGTGCTGGACTCCGACGGCTCCTTCTTCCTCTACAGCA AGCTCACCGTGGACAAGAGCAGGTGGCAGCAGGGGAACGTCTTCT CATGCTCCGTGATGCATGAGGCTCTGCACAACCACTACACGCAGAA GAGCCTCTCCCTGTCTCCGGGTAAAGGAGGCGGTGGGTCAGGTA CCGGAGGCGGTGGGTCAGTCCCGCAAAGGCTTTTCCCCCTCCGCT GCCTCCAGATCTCGTCCTTCGCCAATAGCAGCTGGACGCGCACCGA CGGCTTGGCGTGGCTGGGGGAGCTGCAGACGCACAGCTGGAGCAA CGACTCGGACACCGTCCGCTCTCTGAAGCCTTGGTCCCAGGGCACG TTCAGCGACCAGCAGTGGGAGACGCTGCAGCATATATTTCGGGTTT ATCGAAGCAGCTTCACCAGGGACGTGAAGGAATTCGCCAAAATGC TACGCTTATCCTATCCCTTGGAGCTCCAGGTGTCCGCTGGCTGTGAG GTGCACCCTGGGAACGCCTCAAATAACTTCTTCCATGTAGCATTTC AAGGAAAAGATATCCTGAGTTTGCAAGGAACTTCTTGGGAGCCAAC CCAAGAGGCCCCACTTTGGGTAAACTTGGCCATTCAAGTGCTCAAC CAGGACAAGTGGACGAGGGAAACAGTGCAGTGGCTCCTTAATGGC ACCTGCCCCCAATTTGTCAGTGGCCTCCTTGAGTCAGGGAAGTCGG AACTGAAGAAGCAAGTGAAGCCCAAGGCCTGGCTGTCCCGTGGCC CCAGTCCTGGCCCTGGCCGTCTGCTGCTGGTGTGCCATGTCTCAGG ATTCTACCCAAAGCCTGTATGGGTGAAGTGGATGCGGGGTGAGCA GGAGCAGCAGGGCACTCAGCCAGGGGACATCCTGCCCAATGCTGA CGAGACATGGTATCTCCGAGCAACCCTGGATGTGGTGGCTGGGGA GGCAGCTGGCCTGTCCTGTCGGGTGAAGCACAGCAGTCTAGAGGG CCAGGACATCGTCCTCTACTGGACCGGT*CATCATCACCATCACCAT*TG A (SEQ ID NO:53) (d ouble underline: NotI restriction site; single underline: Signal sequence; bold: BssHII restriction site; dashed underline: BstEII restriction site; bold and underlined: linker; wavy underline: AgeI restriction site; and italics and underlined: 6-His tag).

The final polypeptide sequence of the IgG1CD1d product is: MGWSCIILFLVATATGAHMVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK**GGGGSGTGGGGS**VPORLFPLRCLOISS FANSSWTRTDGLAWLGELQTHSWSNDSDTVRSLKPWSQGTFSDQQW ETLQHIFRVYRSSFTRDVKEFAKMLRLSYPLELQVSAGCEVHPGNASN NFFHVAFQGKDILSFQGTSWEPTQEAPLWVNLAIQVLNQDKWTRETV QWLLNGTCPQFVSGLLESGKSELKKQVKPKAWLSRGPSPGPGRLLLV CHVSGFYPKPVWVKWMRGEQEQQGTQPGDILPNADETWYLRATLDV VAGEAAGLSCRVKHSSLEGQDIVLYWTG*HHHHHH* (SEQ ID NO:54). The variable gene sequence is introduced at the histidine in bold and the rest of the bolded amino acids are removed upon insertion of the variable gene sequence. The linker is bolded and underlined.

### EXAMPLE 16

### Chimeric kappa L chain-CD1d

Employing the same strategy described above for fusion products with immunoglobulin heavy chain or heavy chain fragments, a chimeric kappa L chain is coupled in frame with CD1d. Assembly takes place in a three-step process. In step one, PCR is employed to create the CL region preceded by a signal sequence for secretion and followed by a linker with an embedded KpnI restriction site. The kappa light chain constant region (CK) is PCR amplified from a previously described plasmid template with a pre-configured VL insertion site that allows for directional cloning of any immunoglobulin light chain variable region gene of interest at ApaLI and XhoI restriction sites. In step two, the CD1d gene preceded by the linker with a KpnI restriction site is amplified exactly as described above for heavy chain fusion products. Finally, in step three, the two fragments are joined by restriction digestion at the KpnI site followed by ligation employing standard protocols. The modifications of primer sequences required for amplification of the immunoglobulin light chain with either kappa or lambda light chain constant regions will be apparent to those skilled in the art.

### EXAMPLE 17

### N terminal fusion proteins

Employing strategies similar to those described above, chimeric molecules are constructed where the extracellular domain of CD1d is fused in frame with the amino terminus of the immunoglobulin heavy or light chain. These molecules are assembled in a 3 step process. In step 1, the extracellular domain of CD1d, including the signal sequence is PCR amplified from a plasmid template. This PCR modification adds a NotI site at the 5' end, and a portion of the gly,ser linker and a KPNI site at the 3' end. In step 2, the entire immunoglobulin heavy chain or light chain genes (variable and constant domains, or constant domain with restriction endonuclease sites for the insertion of a V gene), without the signal sequence, is PCR amplified. This PCR modification adds a portion of the gly,ser linker and a KpnI site at the 5' end, and an AgeI site at the 3' end. Finally, in step three, the two fragments are joined by restriction digestion at the KpnI site followed by ligation employing standard protocols. The modifications of primer sequences required for amplification of these genes and the construction of these molecules will be apparent to those skilled in the art.

### EXAMPLE 18

### Construction of β2 microglobulin

The fragment encoding the entire open reading frame of β2-microglobulin is generated by standard PCR using plasmid DNA as template. This fragment is cloned into a Drosophila expression vector such as pMT/V5-His. Cloning is designed so that there is not a 6-His Tag at the C terminal. This is easily accomplished by incorporating a "Stop" codon in the antisense primer immediately following the β2-microglobulin open reading frame. Methods to accomplish this construction are well known to those skilled in the art.

### EXAMPLE 19

### Production of the chimeric antibody-CD1d/β2-microglobulin complex

Production of the antibody-CD1d / β2-microglobulin complex is done in 2 steps. In the first step, Drosophila S2 cells are transfected with the constructs encoding the chimeric antibody-CD1d and the antibody light chain gene. High expressing clones are selected and then transfected with the β2-microglobulin construct and clones expressing high levels of the antibody-CD1d /β-microglobulin complex isolated.

*Step* 1. Generation of Drosophila S2 cells stably transfected and producing antibody-CD1d. Drosophila S2 cells are triply transfected with plasmids that encode 1) antibody-CD1d (full IgG, F(ab), or F(ab')2 2) antibody light chain 3) pCOHYGRO (Invitrogen) Note that this plasmid confers resistance to the drug hygromycin. The triply transfected cells are selected with hygromycin and clones isolated. Expression of the recombinant anitbody-CD1d is induced in the cells by the addition of copper sulfate or cadmium chloride, and the cell supernatants screened by ELISA using antibodies specific for antibody and CD1d.

*Step 2.* Production and purification of the chimeric antibody-CD1d/β2-microglobulin complex. The highest antibody-CD1d producing clones from (A) is cotransfected with plasmids encoding β2-microglobulin (I.E) and pCOBLAST (Invitrogen). pCOBLAST confers resistance to Blasticidin. Stable clones are selected using Hygromycin and Blasticidin. Expression of the recombinant antibody-CD1d/β2M complex is induced in the cells by the addition of copper sulfate or cadmium chloride, and the cell supernatants screened by ELISA using antibodies specific for Ig, B2M, and CD1d. The clones that express the highest amount of antibody-CD1d /β2M complex are expanded and used to produce quantities of this molecule.

The highest expressing clone is expanded to 10+ L scale cultures in X-press serum free media, and induced to express this complex by the addition of copper sulfate or cadmium chloride to the media. The complex containing media is concentrated and dialyzed against 0.15M sodium phosphate buffer (pH 7.4). The antibody-CD1d/β2M complex is purified by Ni-NTA agarose chromatography, and then further purified by HPLC. The purified complex is incubated with a 3 fold molar excess of α-GalCer (CD1d ligand) overnight at room temperature. Excess α-GalCer is removed by dialysis. The final complex is purified by HPLC.

### EXAMPLE 20

### In vitro testing of complexes

The complexes prepared above are tested for the ability of the antibody part to bind the tumor antigen and for the proper conformation of the CD1d complex, by flow cytometry on target antigen-expressing cell lines revealed by an anti-CD1d mAb or by "sandwich elisa" on immobilized target antigen and revealed by anti-CD1d.

Ability of the bifunctional molecules to activate NKT cells is tested on freshly isolated mouse liver MNC or on α-GalCer-established human NKT cell lines. Due to the high degree of conservation between human and mouse CD1d, the two systems are cross reactive (Brossay et al., 1998. J Exp Med 188:1521 and Naidenko et al., 1999. J Exp Med 190:106964, 65). The read out after incubation with target cells coated with the CD1d-anti TAA complex is IFNγ release measured by Elisa or flow cytometry with CD1d-tetramer and intracellular cytokine staining (Matsuda et al., 2000. J Exp Med 192:741).

The monomer of CD1d-antibody is tested for specific activation of NKT cells when coated on a tumor cell line positive for the antigen recognized by the anti-TAA antibody fragment (i.e. human CEA transfected mouse cell line). Negative controls are either a tumor cell line that does not express the TAA, or an unrelated conjugate or α-GalCer alone. Activation of ex vivo mouse liver MNCs or enriched NKT cells (microbeads sorting) or human NKT cell lines will be tested by flow cytometry staining with CDld tetramer and intracellular cytokine such as IFNγ.

### EXAMPLE 20

### In vivo testing of complexes

The anti-tumor activity of CD1d-α-GalCer restricted NKT cells was demonstrated mainly in models of liver and lung metastasis and the favoured cell lines used were EL-4 T lymphoma and B16-BL6 melanoma, both with BL/6 background (Smyth et al., 2002. Blood 99:1259; Hayakawa et al., 2001. Eur J Immunol 31:1720; Takeda et al., 2000. Int Immunol 12:909; and Cui et al., 1997. Science 278:1623). These cells are stably transfected with the surface antigen chosen as target, for example human CEA. In this case, the bifunctional molecules in human CEA-transgenic mice that are tolerant for this antigen and do not develop anti-CEA IgGs will be tested (Clarke et al., 1998. Cancer Res 58:1469). For liver metastasis, tumor cells are injected intrasplenically with or without removing the spleen, while for lung metastasis, inj ection is intravenous in the tail vein.

The complex is tested for systemic activation of NKT cells. Different amounts of conjugate or fusion are injected in normal mice with no tumor and results are compared with injection of α-GalCer alone. It is known that as few as 2 µg of α-GalCer (ip) leads to a rapid disappearance of NKT by activation induced cell death in spleen and liver (Hayakawa et al., 2001. Eur J Immunol 31:1720, Eberl and MacDonald. 2000, Eur J Immunol 30:985). Effects of CD1d-antibody conjugate versus α-GalCer alone is evaluated on liver MNCs by ex vivo flow cytometry with CD1d tetramer. Optimal amount of the bifunctional molecule with limited systemic effect is selected for in vivo tumor treatment.

Tumor graft inhibition: the tumor cell line expressing the TAA is coated in vitro with the selected CD1d-antibody fusion or conjugate and the cells are injected intravenously in the tail vein. The negative control is either the parental cell line negative for the TAA or the same tumor cells coated with a classical MHC Class I-antibody conjugate produced in the laboratory (such as H-2Kb-OVAp-anti CEA conjugate) . After two weeks or at longer time, mice are sacrificed and metastatic nodules in the lung and possibly are counted is be compared with the known NKT-mediated effects of α-GalCer or IL-12 treatments (Hayakawa et al., 2001. Eur J Immunol 31:1720; Takeda et al., 2000. Int Immunol 12:909).

Established tumors: based on the results of the tumor protection experiments, a second set of in vivo experiments is used to test the effectiveness of CD1d-antibody bifunctional molecule on established tumors. Tumor cells are injected iv in the tail vein and treatment with the conjugate or fusion protein is started at different time points as a single or as multiple injections. Antibody fragment alone is injected in additional animals as a negative control. After two to three weeks, animals are sacrificed and metastatic nodules will be analyzed as above. Results are compared to the injection of α-GalCer alone in view of its known antimetastatic effect. In parallel experiments, liver metastasis will be favoured by intrasplenic injection of tumor cells and subsequent removal of the spleen. Similar treatments as above will be tested.

Role of NK cells: When liver and lungs are examined for metastatic nodules, MNCs are isolated and analysed by flow cytometry using anti-CD3 (or CD1d tetramer) and NK markers such as NK1.1 or DX5, allowing to evaluate the presence of CD1d-NKT (CD3+NK1.1+DX5-) and the proliferation of NK cells (CD3-NK1.1+DX5+). Results are compared with MNCs isolated from metastatic tissue treated with the antibody alone or with α-GalCer alone. Alternatively, in vivo depletion of NK cells by injections of anti-asialoGM1 are done in a group of mice at the time of tumor treatment (Smyth et al., 2002. Blood 99:1259, Hayakawa et al., 2001. Eur J Immunol 31:1720).

Human NKT cells: Human NKT cells can be propagated in vitro in contrast to mouse NKT and adoptive transfer studies in mice have shown that small numbers of NKT and NK cells can collaborate to suppress tumor metastasis (Wilson et al., 2002. Trends Mol Med 8:225; Smyth et al., 2002Curr Opin Immunol 14:165; and Smyth et al., 2002. Blood 99:1259). Human CD1d-antitumor antibody molecule is tested in vitro with human tumor cell lines that express the TAA such as CEA as targets and with freshly expanded human NKT cell lines. The read out will be IFNγ release and the respective role of NKT and NK cells will be assessed by non T cells depletion by microbeads negative sorting. Human CD1d-antitumor antibody molecule is also tested in vivo by adoptive transfer of human NKT cells in immunodeficient mice (SCID) grafted with human tumors positive for the TAA. Treatment with the bifunctional molecule of human CD1d-α-GalCer-antitumor antibody is performed as in the syngeneic mouse model.

## Claims

1. A compound for use as a medicament to generate an immune response against a tumor cell comprising:
(a) one or more antigen-loaded CD1d complexes which comprise a CD1d molecule associated with β2-microglobulin, wherein said antigen comprises a glycosylated sphingolipid, and wherein said antigen is bound in the antigen-binding groove of said CD1d molecule; and
(b) an antibody or fragment thereof specific for a cell surface marker of a tumor cell, wherein said antibody or fragment thereof is linked to said CD1d complex.

2. The compound for use as a medicament to generate an immune response against a tumor cell of claim 1, wherein said antigen is α-GalCer.

3. The compound for use as a medicament to generate an immune response against a tumor cell of claim 1, wherein said antigen is α-GalCer modified to have a shortened long-chain sphingosine base, C5 vs. C14 and acyl chain, 24 vs. C26,

4. The compound for use as a medicament to generate an immune response against a tumor cell of claim 3, wherein said modified α-GalCer is the OCH analog with a long-chain sphingosine base shortened from C14 to C5 and acyl chain from C26 to C24.

5. The compound for use as a medicament to generate an immune response against a tumor cell of any of claims 1-4, wherein said antibody or fragment thereof is a F(ab) fragment.

6. The compound for use as a medicament to generate an immune response against a tumor cell of any of claims 1-4, wherein said antibody or fragment thereof is a F(ab')2 fragment.

7. The compound for use as a medicament to generate an immune response against a tumor cell of any of claims 1-4, wherein said antibody a full-length antibody.

8. The compound for use as a medicament to generate an immune response against a tumor cell of any of claims 1-4, wherein said antibody or fragment thereof is an scFv fragment.

9. The compound for use as a medicament to generate an immune response against a tumor cell of any one of claims 1 to 8, wherein said cell surface marker is selected from the group consisting of CEA, Her2/neu, EGFR type I or type II, CD19, CD20, CD22, Muc-1, PSMA, or STEAP.

10. The compound for use as a medicament to generate an immune response against a tumor cell of any one of claims 1 to 8, wherein said cell surface marker is selected from the group consisting of : Lewis Y, erbB-3 and -4, Ep-CAM, E-cadherin neoepitope, EGFR deletion neoepitope, CA19-9, Muc-1, LeY, TF-, Tn- and sTn-antigen, TAG-72, Cora antigen, CD7, CD25, Ig-a and Ig-B, A33 and G250, CD30, MCSP and gp100, CD44-v6, MT-MMPS, (MIS) receptor type II, carboanhydrase 9, F19-antigen, Ly6, desmoglein 4, PSCA, Wue-1, GD2 and GD3 as well as TM4SF-antigens (CD63, L6, CO-29, SAS), the alpha and gamma subunit of the fetal type acetylcholinreceptor (AChR), CM-1, 28K2, E48, U36, NY-ESO-1, KU-BL 1-5, NY CO 1-48, HOM MEL 40, OV569, ChCE7, CA19-9, CA125, GM2, GD2,9-o-acetyl-GD3, or GD3.

11. The compound for use as a medicament to generate an immune response against a tumor cell of any one of claims 1-10, wherein said CD1d molecule is attached to the heavy chain of said antibody or fragment thereof.

12. The compound for use as a medicament to generate an immune response against a tumor cell of any one of claims 1-10, wherein said CD1d molecule is attached to the light chain of said antibody or fragment thereof.

13. The compound for use as a medicament to generate an immune response against a tumor cell of any one of claims 1-10, wherein said β2-microglobulin molecule is attached to the heavy chain of said antibody or fragment thereof.

14. The compound for use as a medicament to generate an immune response against a tumor cell of any one of claims 1-10, wherein said β2-microglobulin molecule is attached to the light chain of said antibody or fragment thereof.

15. The compound for use as a medicament to generate an immune response against a tumor cell of any one of claims 1-14, wherein said CD1d complexes are fused to said antibody or fragment thereof.

16. The compound for use as a medicament to generate an immune response against a tumor cell of any one of claims 1-14, wherein said CD1d complexes are attached to said antibody or fragment thereof through a linker sequence.

17. The compound for use as a medicament to generate an immune response against a tumor cell of any one of claims 1-14, wherein said CD1d complexes are attached to said antibody or fragment thereof through a multivalent compound.

18. The compound for use as a medicament to generate an immune response against a tumor cell of claim 17, wherein said multivalent compound is selected from the group consisting of streptavidin, chicken avidin and a modified GCN4-zipper motif.

19. The compound for use as a medicament to generate an immune response against a tumor cell of any of claims 1-18, further comprising a costimulatory molecule.

20. The compound for use as a medicament to generate an immune response against a tumor cell of claim 19, wherein said costimulatory molecule is B7.

21. Use of the compound of any of claims 1-20 for the preparation of a medicament that can induce an anti-tumor response in a mammal.

## Patentansprüche

1. Verbindung zur Verwendung als Arzneimittel, um eine Immunantwort gegen eine Tumorzelle zu erzeugen, umfassend:
(a) einen oder mehrere mit Antigen beladene CD1d-Komplexe, die ein mit β2-Mikroglobulin assoziiertes CD1d-Molekül umfassen, wobei das Antigen ein glykosyliertes Sphingolipid umfasst und wobei das Antigen in der antigenbindenden Furche des CD1d-Moleküls gebunden ist; und
(b) einen Antikörper oder ein Fragment davon, der bzw. das für einen Zelloberflächen-Marker einer Tumorzelle spezifisch ist, wobei der Antikörper bzw. das Fragment davon mit dem CD1d-Komplex verknüpft ist.

2. Verbindung zur Verwendung als Arzneimittel, um eine Immunantwort gegen eine Tumorzelle zu erzeugen, nach Anspruch 1, wobei es sich bei dem Antigen um α-GalCer handelt.

3. Verbindung zur Verwendung als Arzneimittel, um eine Immunantwort gegen eine Tumorzelle zu erzeugen, nach Anspruch 1, wobei es sich bei dem Antigen um α-GalCer handelt, das so modifiziert ist, dass es eine verkürzte langkettige Sphingosinbase, C5 vs. C14, und Acylkette, C24 vs. C26, aufweist.

4. Verbindung zur Verwendung als Arzneimittel, um eine Immunantwort gegen eine Tumorzelle zu erzeugen, nach Anspruch 3, wobei es sich bei dem modifizierten α-GalCer um das OCH-Analog mit von C14 auf C5 verkürzter langkettiger Sphingosinbase und Acylkette von C26 auf C24 handelt.

5. Verbindung zur Verwendung als Arzneimittel, um eine Immunantwort gegen eine Tumorzelle zu erzeugen, nach einem der Ansprüche 1-4, wobei es sich bei dem Antikörper oder Fragment davon um ein F(ab)-Fragment handelt.

6. Verbindung zur Verwendung als Arzneimittel, um eine Immunantwort gegen eine Tumorzelle zu erzeugen, nach einem der Ansprüche 1-4, wobei es sich bei dem Antikörper oder Fragment davon um ein F(ab')2-Fragment handelt.

7. Verbindung zur Verwendung als Arzneimittel, um eine Immunantwort gegen eine Tumorzelle zu erzeugen, nach einem der Ansprüche 1-4, wobei es sich bei dem Antikörper um einen Volllängen-Antikörper handelt.

8. Verbindung zur Verwendung als Arzneimittel, um eine Immunantwort gegen eine Tumorzelle zu erzeugen, nach einem der Ansprüche 1-4, wobei es sich bei dem Antikörper oder Fragment davon um ein scFv-Fragment handelt.

9. Verbindung zur Verwendung als Arzneimittel, um eine Immunantwort gegen eine Tumorzelle zu erzeugen, nach einem der Ansprüche 1 bis 8, wobei der Zelloberflächen-Marker aus der aus CEA, Her2/neu, EGFR Typ I oder Typ II, CD19, CD20, CD22, Muc-1, PSMA oder STEAP bestehenden Gruppe ausgewählt ist.

10. Verbindung zur Verwendung als Arzneimittel, um eine Immunantwort gegen eine Tumorzelle zu erzeugen, nach einem der Ansprüche 1 bis 8, wobei der Zelloberflächen-Marker aus der aus Lewis Y, erbB-3 und -4, Ep-CAM, E-Cadherin-Neoepitop, EGFR-Deletion-Neoepitop, CA19-9, Muc-1, LeY, TF-, Tn- und sTn-Antigen, TAG-72, Cora-Antigen, CD7, CD25, Ig-a und Ig-B, A33 und G250, CD30, MCSP und gp100, CD44-v6, MT-MMPS, (MIS-) Rezeptor Typ II, Carboanhydrase 9, F19-Antigen, Ly6, Desmoglein 4, PSCA, Wue-1, GD2 und GD3 ebenso wie TM4SF-Antigenen (CD63, L6, CO-29, SAS), der Alpha- und Gamma-Untereinheit des Acetylcholinrezeptors (AchR), fetaler Typ, CM-1, 28K2, E48, U36, NY-ESO-1, KU-BL 1-5, NY CO 1-48, HOM MEL 40, OV569, ChCE7, CA19-9, CA125, GM2, GD2,9-o-Acetyl-GD3 oder GD3 bestehenden Gruppe ausgewählt ist.

11. Verbindung zur Verwendung als Arzneimittel, um eine Immunantwort gegen eine Tumorzelle zu erzeugen, nach einem der Ansprüche 1-10, wobei das CD1d-Molekül an der schweren Kette des Antikörpers oder Fragments davon gebunden ist.

12. Verbindung zur Verwendung als Arzneimittel, um eine Immunantwort gegen eine Tumorzelle zu erzeugen, nach einem der Ansprüche 1-10, wobei das CD1d-Molekül an der leichten Kette des Antikörpers oder Fragments davon gebunden ist.

13. Verbindung zur Verwendung als Arzneimittel, um eine Immunantwort gegen eine Tumorzelle zu erzeugen, nach einem der Ansprüche 1-10, wobei das β2-Mikroglobulin-Molekül an der schweren Kette des Antikörpers oder Fragments davon gebunden ist.

14. Verbindung zur Verwendung als Arzneimittel, um eine Immunantwort gegen eine Tumorzelle zu erzeugen, nach einem der Ansprüche 1-10, wobei das β2-Mikroglobulin-Molekül an der leichten Kette des Antikörpers oder Fragments davon gebunden ist.

15. Verbindung zur Verwendung als Arzneimittel, um eine Immunantwort gegen eine Tumorzelle zu erzeugen, nach einem der Ansprüche 1-14, wobei die CD1d-Komplexe mit dem Antikörper oder Fragment davon fusioniert sind.

16. Verbindung zur Verwendung als Arzneimittel, um eine Immunantwort gegen eine Tumorzelle zu erzeugen, nach einem der Ansprüche 1-14, wobei die CD1d-Komplexe an den Antikörper oder das Fragment davon über eine Linker-Sequenz gebunden sind.

17. Verbindung zur Verwendung als Arzneimittel, um eine Immunantwort gegen eine Tumorzelle zu erzeugen, nach einem der Ansprüche 1-14, wobei die CD1d-Komplexe an den Antikörper oder das Fragment davon über eine multivalente Verbindung gebunden sind.

18. Verbindung zur Verwendung als Arzneimittel, um eine Immunantwort gegen eine Tumorzelle zu erzeugen, nach Anspruch 17, wobei die multivalente Verbindung aus der aus Streptavidin, Hühneravidin und einem modifizierten GCN4-Zipper-Motiv bestehenden Gruppe ausgewählt ist.

19. Verbindung zur Verwendung als Arzneimittel, um eine Immunantwort gegen eine Tumorzelle zu erzeugen, nach einem der Ansprüche 1-18, ferner umfassend ein Kostimulationsmolekül.

20. Verbindung zur Verwendung als Arzneimittel, um eine Immunantwort gegen eine Tumorzelle zu erzeugen, nach Anspruch 19, wobei es sich bei dem Kostimulationsmolekül um B7 handelt.

21. Verwendung der Verbindung nach einem der Ansprüche 1-20 zur Herstellung eines Arzneimittels, das eine Antitumorantwort bei einem Säuger induzieren kann.

## Revendications

1. Composé pour utilisation en tant que médicament, pour générer une réponse immune contre une cellule tumorale, comprenant :
(a) un ou plusieurs complexes CD1d chargés en antigène, qui comprennent une molécule de CD1d associée à la β2-microglobuline, ledit antigène comprenant un sphingolipide glycosylé, et ledit antigène étant lié dans le sillon de liaison à l'antigène de ladite molécule de CD1d ; et
(b) un anticorps ou un fragment de ce dernier, spécifique d'un marqueur de surface cellulaire d'une cellule tumorale, ledit anticorps ou fragment de ce dernier étant lié audit complexe CD1d.

2. Composé pour utilisation en tant que médicament, pour générer une réponse immune contre une cellule tumorale de la revendication 1, dans lequel ledit antigène est α-GalCer.

3. Composé pour utilisation en tant que médicament, pour générer une réponse immune contre une cellule tumorale de la revendication 1, dans lequel ledit antigène est α-GalCer modifié de façon à avoir une base de sphingosine à longue chaîne raccourcie, C5 par rapport à C14, et une chaîne acyle raccourcie, C24 par rapport à C26.

4. Composé pour utilisation en tant que médicament, pour générer une réponse immune contre une cellule tumorale de la revendication 3, dans lequel ledit α-GalCer modifié est l'analogue OCH avec une base de sphingosine à longue chaîne raccourcie de C14 à C5, et une chaîne acyle raccourcie de C26 à C24.

5. Composé pour utilisation en tant que médicament, pour générer une réponse immune contre une cellule tumorale de l'une quelconque des revendications 1 à 4, dans lequel ledit anticorps ou fragment de ce dernier est un fragment F(ab).

6. Composé pour utilisation en tant que médicament, pour générer une réponse immune contre une cellule tumorale de l'une quelconque des revendications 1 à 4, dans lequel ledit anticorps ou fragment de ce dernier est un fragment F(ab')2.

7. Composé pour utilisation en tant que médicament, pour générer une réponse immune contre une cellule tumorale de l'une quelconque des revendications 1 à 4, dans lequel ledit anticorps est un anticorps complet.

8. Composé pour utilisation en tant que médicament, pour générer une réponse immune contre une cellule tumorale de l'une quelconque des revendications 1 à 4, dans lequel ledit anticorps ou fragment de ce dernier est un fragment scFv.

9. Composé pour utilisation en tant que médicament, pour générer une réponse immune contre une cellule tumorale de l'une quelconque des revendications 1 à 8, dans lequel ledit marqueur de surface cellulaire est choisi dans le groupe consistant en CEA, Her2/neu, EGFR type I ou type II, CD19, CD20, CD22, Muc-1, PSMA ou STEAP.

10. Composé pour utilisation en tant que médicament, pour générer une réponse immune contre une cellule tumorale de l'une quelconque des revendications 1 à 8, dans lequel ledit marqueur de surface cellulaire est choisi dans le groupe consistant en : Lewis Y, erbB-3 et -4, Ep-CAM, néoépitope de E-cadhérine, néoépitope de délétion de EGFR, CA19-9, Muc-1, LeY, antigène TF, Tn et sTn, TAG-72, antigène Cora, CD7, CD25, Ig-a et Ig-B, A33 et G250, CD30, MCSP et gp100, CD44-v6, Mt-MMPS, récepteur (MIS) de type II, carboanhydrase 9, antigène F19, Ly6, desmogléine 4, PSCA, Wue-1, GD2 et GD3, ainsi que les antigènes TM4SF (CD63, L6, CO-29, SAS), la sous-unité alpha et la sous-unité gamma du récepteur de l'acétylcholine de type foetal (AChR), CM-1, 28K2, E48, U36, NY-ESO-1, KU-BL 1-5, NY CO 1-48, HOM MEL 40, OV569, ChCE7, CA19-9, CA125, GM2, GD2,9-o-acétyl-GD3 ou GD3.

11. Composé pour utilisation en tant que médicament, pour générer une réponse immune contre une cellule tumorale de l'une quelconque des revendications 1 à 10, dans lequel ladite molécule de CD1d est fixée à la chaîne lourde dudit anticorps ou fragment de ce dernier.

12. Composé pour utilisation en tant que médicament, pour générer une réponse immune contre une cellule tumorale de l'une quelconque des revendications 1 à 10, dans lequel ladite molécule de CD1d est fixée à la chaîne légère dudit anticorps ou fragment de ce dernier.

13. Composé pour utilisation en tant que médicament, pour générer une réponse immune contre une cellule tumorale de l'une quelconque des revendications 1 à 10, dans lequel ladite molécule de **β**2-microglobuline est fixée à la chaîne lourde dudit anticorps ou fragment de ce dernier.

14. Composé pour utilisation en tant que médicament, pour générer une réponse immune contre une cellule tumorale de l'une quelconque des revendications 1 à 10, dans lequel ladite molécule de **β**2-microglobuline est fixée à la chaîne légère dudit anticorps ou fragment de ce dernier.

15. Composé pour utilisation en tant que médicament, pour générer une réponse immune contre une cellule tumorale de l'une quelconque des revendications 1 à 14, dans lequel lesdits complexes CD1d sont fusionnés audit anticorps ou fragment de ce dernier.

16. Composé pour utilisation en tant que médicament, pour générer une réponse immune contre une cellule tumorale de l'une quelconque des revendications 1 à 14, dans lequel lesdits complexes CD1d sont fixés audit anticorps ou fragment de ce dernier par l'intermédiaire d'une séquence de liaison.

17. Composé pour utilisation en tant que médicament, pour générer une réponse immune contre une cellule tumorale de l'une quelconque des revendications 1 à 14, dans lequel lesdits complexes CD1d sont fixés audit anticorps ou fragment de ce dernier par l'intermédiaire d'un composé multivalent.

18. Composé pour utilisation en tant que médicament, pour générer une réponse immune contre une cellule tumorale de la revendication 17, dans lequel ledit composé multivalent est choisi dans le groupe consistant en la streptavidine, l'avidine de poulet et un motif glissière GCN4 modifié.

19. Composé pour utilisation en tant que médicament, pour générer une réponse immune contre une cellule tumorale de l'une quelconque des revendications 1 à 18, comprenant en outre une molécule co-stimulatrice.

20. Composé pour utilisation en tant que médicament, pour générer une réponse immune contre une cellule tumorale de la revendication 19, dans lequel ladite molécule co-stimulatrice est B7.

21. Utilisation du composé de l'une quelconque des revendications 1 à 20 pour la préparation d'un médicament capable d'induire une réponse anti-tumorale chez un mammifère.


### SEQUENCE LISTING

<110> Vaccinex, Inc. Robert, Bruno Donda, Alena Cesson, Valerie Mach, Jean-Pierre Zauderer, Maurice
<120> Targeted CD1d Molecules
<130> 1843.020PC00
<150> EP 02405838.0
   <151> 2002-09-27
<160> 54
<170> PatentIn version 3.2
<210> 1
   <211> 15
   <212> PRT
   <213> Artificial Sequence
<220>
   <223> Artificial linker peptide
<400> 1
   Gly Gly Gly Gly Ser Gly Gly Gly Gly Ser Gly Gly Gly Gly Ser
   1               5                   10                  15
<210> 2
   <211> 15
   <212> PRT
   <213> Artificial Sequence
<220>
   <223> Artificial linker peptide
<400> 2
   Gly Gly Gly Gly Ser Gly Gly Gly Gly Ser Gly Gly Gly Ala Ser
   1               5                   10                  15
<210> 3
   <211> 38
   <212> DNA
   <213> Artificial Sequence
<220>
   <223> F(ab)-avidin sense fragment
<400> 3
   aattgcggcc gcaaaccatg ggatggagct gtatcatc 38
<210> 4
   <211> 45
   <212> DNA
   <213> Artificial Sequence
<220>
   <223> F(ab)-avidin antisense fragment
<400> 4
   cggggtacct gacccaccgc ctcctttctt gtccaccttg gtgtt 45
<210> 5
   <211> 38
   <212> DNA
   <213> Artificial Sequence.
<220>
   <223> F(ab')2-avidin sense fragment
<400> 5
   aattgcggcc gcaaaccatg ggatggagct gtatcatc 38
<210> 6
   <211> 45
   <212> DNA
   <213> Artificial Sequence
<220>
   <223> F(ab')2-avidin antisense fragment
<400> 6
   cggggtacct gacccaccgc ctcctgggca cggtgggcat gtgtg 45
<210> 7
   <211> 38
   <212> DNA
   <213> Artificial Sequence
<220>
   <223> IgG1-avidin sense fragment
<400> 7
   aattgcggcc gcaaaccatg ggatggagct gtatcatc 38
<210> 8
   <211> 45
   <212> DNA
   <213> Artificial Sequence
<220>
   <223> IgG1-avidin antisense fragment
<400> 8
   cggggtacct gacccaccgc ctcctttacc cggagacagg gagag 45
<210> 9
   <211> 40
   <212> DNA
   <213> Artificial Sequence
<220>
   <223> Chick avidin sense primer .
<400> 9
   cggggtaccg gaggcggtgg gtcagccaga aagtgctcgc 40
<210> 10
   <211> 28
   <212> DNA
   <213> Artificial Sequence
<220>
   <223> Chick avidin antisense primer
<400> 10
   cgaccggtct ccttctgtgt gcgcaggc 28
<210> 11
   <211> 823
   <212> DNA
   <213> Artificial Sequence
<220>
   <223> F(ab)-Avidin nucleotide construct
<400> 11
   gcggccgcaa accatgggat ggagctgtat catcctcttc ttggtagcaa cagctacagg     60
   cgcgcatatg gtcaccgtct cctcagcctc caccaagggc ccatcggtct tccccctggc    120
   accctcctcc aagagcacct ctgggggcac agcggccctg ggctgcctgg tcaaggacta    180
   cttccccgaa ccggtgacgg tgtcgtggaa ctcaggcgcc ctgaccagcg gcgtgcacac    240
   cttcccggct gtcctacagt cctcaggact ctactccctc agcagcgtcg tgaccgtgcc    300
   ctccagcagc ttgggcaccc agacctacat ctgcaacgtg aatcacaagc ccagcaacac    360
   caaggtggac aagaaaggag gcggtgggtc aggtaccgga ggcggtgggt cagccagaaa    420
   gtgctcgctg actgggaaat ggaccaacga tctgggctcc aacatgacca tcggggctgt    480
   gaacagcaga ggtgaattca caggcaccta catcacagcc gtaacagcca catcaaatga    540
   gatcaaagag tcaccactgc atgggacaca aaacaccatc aacaagagga cccagcccac    600
   ctttggcttc accgtcaatt ggaagttttc agagtccacc actgtcttca cgggccagtg    660
   cttcatagac aggaatggga aggaggtcct gaagaccatg tggctgctgc ggtcaagtgt    720
   taatgacatt ggtgatgact ggaaagctac cagggtcggc atcaacatct tcactcgcct    780
   gcgcacacag aaggagaccg gtcatcatca ccatcaccat tga                       823
<210> 12
   <211> 269
   <212> PRT
   <213> Artificial Sequence
<220>
   <223> F(ab)-Avidin polypeptide construct
<400> 12
   Met Gly Trp Ser Cys Ile Ile Leu Phe Leu Val Ala Thr Ala Thr Gly
   1               5                   10                  15
   Ala His Met Val Thr Val Ser Ser Ala Ser Thr Lys Gly Pro Ser Val
               20                  25                  30
   Phe Pro Leu Ala Pro Ser Ser Lys Ser Thr Ser Gly Gly Thr Ala Ala
           35                  40                  45
   Leu Gly Cys Leu Val Lys Asp Tyr Phe Pro Glu Pro Val Thr Val Ser
       50                  55                  60
   Trp Asn Ser Gly Ala Leu Thr Ser Gly Val His Thr Phe Pro Ala Val
   65                  70                  75                  80
   Leu Gln Ser Ser Gly Leu Tyr Ser Leu Ser Ser Val Val Thr Val Pro
                   85                  90                  95
   Ser Ser Ser Leu Gly Thr Gln Thr Tyr Ile Cys Asn Val Asn His Lys
               100                 105                 110
   Pro Ser Asn Thr Lys Val Asp Lys Lys Gly Gly Gly Gly Ser Gly Thr
           115                 120                 125
   Gly Gly Gly Gly Ser Ala Arg Lys Cys Ser Leu Thr Gly Lys Trp Thr
       130                 135                 140
   Asn Asp Leu Gly Ser Asn Met Thr Ile Gly Ala Val Asn Ser Arg Gly
   145                 150                 155                 160
   Glu Phe Thr Gly Thr Tyr Ile Thr Ala Val Thr Ala Thr Ser Asn Glu
                   165                 170                 175
   Ile Lys Glu Ser Pro Leu His Gly Thr Gln Asn Thr Ile Asn Lys Arg
               180                 185                 190
   Thr Gln Pro Thr Phe Gly Phe Thr Val Asn Trp Lys Phe Ser Glu Ser
           195                 200                 205
   Thr Thr Val Phe Thr Gly Gln Cys Phe Ile Asp Arg Asn Gly Lys Glu
       210                 215                 220
   Val Leu Lys Thr Met Trp Leu Leu Arg Ser Ser Val Asn Asp Ile Gly
   225 230 235 240
   Asp Asp Trp Lys Ala Thr Arg Val Gly Ile Asn Ile Phe Thr Arg Leu
   245 250 255
   Arg Thr Gln Lys Glu Thr Gly His His His His His His
   260 265
<210> 13
   <211> 871
   <212> DNA
   <213> Artificial Sequence
<220>
   <223> F(ab')2-Avidin nucleotide construct
<400> 13
   gcggccgcaa accatgggat ggagctgtat catcctcttc ttggtagcaa cagctacagg     60
   cgcgcatatg gtcaccgtct cctcagcctc caccaagggc ccatcggtct tccccctggc    120
   accctcctcc aagagcacct ctgggggcac agcggccctg ggctgcctgg tcaaggacta    180
   cttccccgaa ccggtgacgg tgtcgtggaa ctcaggcgcc ctgaccagcg gcgtgcacac    240
   cttcccggct gtcctacagt cctcaggact ctactccctc agcagcgtcg tgaccgtgcc    300
   ctccagcagc ttgggcaccc agacctacat ctgcaacgtg aatcacaagc ccagcaacac    360
   caaggtggac aagaaagttg agcccaaatc ttgtgacaaa actcacacat gcccaccgtg    420
   cccaggaggc ggtgggtcag gtaccggagg cggtgggtca gccagaaagt gctcgctgac    480
   tgggaaatgg accaacgatc tgggctccaa catgaccatc ggggctgtga acagcagagg    540
   tgaattcaca ggcacctaca tcacagccgt aacagccaca tcaaatgaga tcaaagagtc    600
   accactgcat gggacacaaa acaccatcaa caagaggacc cagcccacct ttggcttcac    660
   cgtcaattgg aagttttcag agtccaccac tgtcttcacg ggccagtgct tcatagacag    720
   gaatgggaag gaggtcctga agaccatgtg gctgctgcgg tcaagtgtta atgacattgg    780
   tgatgactgg aaagctacca gggtcggcat caacatcttc actcgcctgc gcacacagaa    840
   ggagaccggt catcatcacc atcaccattg a                                   871
<210> 14
   <211> 285
   <212> PRT
   <213> Artificial Sequence
<220>
   <223> F(ab')2-Avidin polypetide construct
<400> 14
   Met Gly Trp Ser Cys Ile Ile Leu Phe Leu Val Ala Thr Ala Thr Gly
   1               5                   10                  15
   Ala His Met Val Thr Val Ser Ser Ala Ser Thr Lys Gly Pro Ser Val
               20                  25                  30
   Phe Pro Leu Ala Pro Ser Ser Lys Ser Thr Ser Gly Gly Thr Ala Ala
           35                  40                  45
   Leu Gly Cys Leu Val Lys Asp Tyr Phe Pro Glu Pro Val Thr Val Ser
       50                  55                  60
   Trp Asn Ser Gly Ala Leu Thr Ser Gly Val His Thr Phe Pro Ala Val
   65                  70                  75                  80
   Leu Gln Ser Ser Gly Leu Tyr Ser Leu Ser Ser Val Val Thr Val Pro
                   85                  90                  95
   Ser Ser Ser Leu Gly Thr Gln Thr Tyr Ile Cys Asn Val Asn His Lys
               100                 105                 110
   Pro Ser Asn Thr Lys Val Asp Lys Lys Val Glu Pro Lys Ser Cys Asp
           115                 120                 125
   Lys Thr His Thr Cys Pro Pro Cys Pro Gly Gly Gly Gly Ser Gly Thr
       130                 135                 140
   Gly Gly Gly Gly Ser Ala Arg Lys Cys Ser Leu Thr Gly Lys Trp Thr
   145                 150                 155                 160
   Asn Asp Leu Gly Ser Asn Met Thr Ile Gly Ala Val Asn Ser Arg Gly
                   165                 170                 175
   Glu Phe Thr Gly Thr Tyr Ile Thr Ala Val Thr Ala Thr Ser Asn Glu
               180                 185                 190
   Ile Lys Glu Ser Pro Leu His Gly Thr Gln Asn Thr Ile Asn Lys Arg
           195                 200                 205
   Thr Gln Pro Thr Phe Gly Phe Thr Val Asn Trp Lys Phe Ser Glu Ser
       210                 215                 220
   Thr Thr Val Phe Thr Gly Gln Cys Phe Ile Asp Arg Asn Gly Lys Glu
   225                 230                 235                 240
   Val Leu Lys Thr Met Trp Leu Leu Arg Ser Ser Val Asn Asp Ile Gly
                   245                 250                 255
   Asp Asp Trp Lys Ala Thr Arg Val Gly Ile Asn Ile Phe Thr Arg Leu
   260 265 270
   Arg Thr Gln Lys Glu Thr Gly His His His His His His
   275 280 285
<210> 15
   <211> 1522
   <212> DNA
   <213> Artificial Sequence
<220>
   <223> IgG1 Avidin nucleotide construct
<400> 15
   gcggccgcaa accatgggat ggagctgtat catcctcttc ttggtagcaa cagctacagg     60
   cgcgcatatg gtcaccgtct cctcagcctc caccaagggc ccatcggtct tccccctggc   120
   accctcctcc aagagcacct ctgggggcac agcggccctg ggctgcctgg tcaaggacta    180
   cttccccgaa ccggtgacgg tgtcgtggaa ctcaggcgcc ctgaccagcg gcgtgcacac    240
   cttcccggct gtcctacagt cctcaggact ctactccctc agcagcgtcg tgaccgtgcc    300
   ctccagcagc ttgggcaccc agacctacat ctgcaacgtg aatcacaagc ccagcaacac    360
   caaggtggac aagaaagttg agcccaaatc ttgtgacaaa actcacacat gcccaccgtg    420
   cccagcacct gaactcctgg ggggaccgtc agtcttcctc ttccccccaa aacccaagga    480
   caccctcatg atctcccgga cccctgaggt cacatgcgtg gtggtggacg tgagccacga    540
   agaccctgag gtcaagttca actggtacgt ggacggcgtg gaggtgcata atgccaagac    600
   aaagccgcgg gaggagcagt acaacagcac gtaccgtgtg gtcagcgtcc tcaccgtcct    660
   gcaccaggac tggctgaatg gcaaggagta caagtgcaag gtctccaaca aagccctccc    720
   agcccccatc gagaaaacca tctccaaagc caaagggcag ccccgagaac cacaggtgta    780
   caccctgccc ccatcccggg atgagctgac caagaaccag gtcagcctga cctgcctggt    840
   caaaggcttc tatcccagcg acatcgccgt ggagtgggag agcaatgggc agccggagaa    900
   caactacaag accacgcctc ccgtgctgga ctccgacggc tccttcttcc tctacagcaa    960
   gctcaccgtg gacaagagca ggtggcagca ggggaacgtc ttctcatgct ccgtgatgca   1020
   tgaggctctg cacaaccact acacgcagaa gagcctctcc ctgtctccgg gtaaaggagg   1080
   cggtgggtca ggtaccggag gcggtgggtc agccagaaag tgctcgctga ctgggaaatg   1140
   gaccaacgat ctgggctcca acatgaccat cggggctgtg aacagcagag gtgaattcac   1200
   aggcacctac atcacagccg taacagccac atcaaatgag atcaaagagt caccactgca   1260
   tgggacacaa aacaccatca acaagaggac ccagcccacc tttggcttca ccgtcaattg   1320
   gaagttttca gagtccacca ctgtcttcac gggccagtgc ttcatagaca ggaatgggaa   1380
   ggaggtcctg aagaccatgt ggctgctgcg gtcaagtgtt aatgacattg gtgatgactg 1440
   gaaagctacc agggtcggca tcaacatctt cactcgcctg cgcacacaga aggagaccgg 1500
   tcatcatcac catcaccatt ga 1522
<210> 16
   <211> 502
   <212> PRT
   <213> Artificial Sequence
<220>
   <223> IgG1 Avidin polypeptide construct
<400> 16
   Met Gly Trp Ser Cys Ile Ile Leu Phe Leu Val Ala Thr Ala Thr Gly
   1 5 10 15
   Ala His Met Val Thr Val Ser Ser Ala Ser Thr Lys Gly Pro Ser Val
   20 25 30
   Phe Pro Leu Ala Pro Ser Ser Lys Ser Thr Ser Gly Gly Thr Ala Ala
   35 40 45
   Leu Gly Cys Leu Val Lys Asp Tyr Phe Pro Glu Pro Val Thr Val Ser
   50 55 60
   Trp Asn Ser Gly Ala Leu Thr Ser Gly Val His Thr Phe Pro Ala Val
   65 70 75 80
   Leu Gln Ser Ser Gly Leu Tyr Ser Leu Ser Ser Val Val Thr Val Pro
   85 90 95
   Ser Ser Ser Leu Gly Thr Gln Thr Tyr Ile Cys Asn Val Asn His Lys
   100 105 110
   Pro Ser Asn Thr Lys Val Asp Lys Lys Val Glu Pro Lys Ser Cys Asp
   115 120 125
   Lys Thr His Thr Cys Pro Pro Cys Pro Ala Pro Glu Leu Leu Gly Gly
   130 135 140
   Pro Ser Val Phe Leu Phe Pro Pro Lys Pro Lys Asp Thr Leu Met Ile
   145 150 155 160
   Ser Arg Thr Pro Glu Val Thr Cys Val Val Val Asp Val Ser His Glu
   165 170 175
   Asp Pro Glu Val Lys Phe Asn Trp Tyr Val Asp Gly Val Glu Val His
   180 185 190
   Asn Ala Lys Thr Lys Pro Arg Glu Glu Gln Tyr Asn Ser Thr Tyr Arg
   195 200 205
   Val Val Ser Val Leu Thr Val Leu His Gln Asp Trp Leu Asn Gly Lys
   210 215 220
   Glu Tyr Lys Cys Lys Val Ser Asn Lys Ala Leu Pro Ala Pro Ile Glu
   225 230 235 240
   Lys Thr Ile Ser Lys Ala Lys Gly Gln Pro Arg Glu Pro Gln Val Tyr
   245 250 255
   Thr Leu Pro Pro Ser Arg Asp Glu Leu Thr Lys Asn Gln Val Ser Leu
   260 265 270
   Thr Cys Leu Val Lys Gly Phe Tyr Pro Ser Asp Ile Ala Val Glu Trp
   275 280 285
   Glu Ser Asn Gly Gln Pro Glu Asn Asn Tyr Lys Thr Thr Pro Pro Val
   290 295 300
   Leu Asp Ser Asp Gly Ser Phe Phe Leu Tyr Ser Lys Leu Thr Val Asp
   305 310 315 320
   Lys Ser Arg Trp Gln Gln Gly Asn Val Phe Ser Cys Ser Val Met His
   325 330 335
   Glu Ala Leu His Asn His Tyr Thr Gln Lys Ser Leu Ser Leu Ser Pro
   340 345 350
   Gly Lys Gly Gly Gly Gly Ser Gly Thr Gly Gly Gly Gly Ser Ala Arg
   355 360 365
   Lys Cys Ser Leu Thr Gly Lys Trp Thr Asn Asp Leu Gly Ser Asn Met
   370 375 380
   Thr Ile Gly Ala Val Asn Ser Arg Gly Glu Phe Thr Gly Thr Tyr Ile
   385 390 395 400
   Thr Ala Val Thr Ala Thr Ser Asn Glu Ile Lys Glu Ser Pro Leu His
   405 410 415
   Gly Thr Gln Asn Thr Ile Asn Lys Arg Thr Gln Pro Thr Phe Gly Phe
   420 425 430
   Thr Val Asn Trp Lys Phe Ser Glu Ser Thr Thr Val Phe Thr Gly Gln
   435 440 445
   Cys Phe Ile Asp Arg Asn Gly Lys Glu Val Leu Lys Thr Met Trp Leu
   450 455 460
   Leu Arg Ser Ser Val Asn Asp Ile Gly Asp Asp Trp Lys Ala Thr Arg
   465 470 475 480
   Val Gly Ile Asn Ile Phe Thr Arg Leu Arg Thr Gln Lys Glu Thr Gly
   485 490 495
   His His His His His His
   500
<210> 17
   <211> 57
   <212> DNA
   <213> Artificial Sequence
<220>
   <223> BirA Sense Sequence
<400> 17
   ccggtggtgg cggtctgaac gacatcttcg aggctcagaa aatcgaatgg cacgaat 57
<210> 18
   <211> 57
   <212> DNA
   <213> Artificial Sequence
<220>
   <223> BirA Antisense Sequence
<400> 18
   ccggattcgt gccattcgat tttctgagcc tcgaagatgt cgttcagacc gccacca 57
<210> 19
   <211> 34
   <212> DNA
   <213> Artificial Sequence
<220>
   <223> E1 sense sequence
<400> 19
   cacggtaccg atatggggtg cctgctgttt ctgc 34
<210> 20
   <211> 30
   <212> DNA
   <213> Artificial Sequence
<220>
   <223> E1 antisense
<400> 20
   cagaccggtc cagtagagga cgatgtcctg 30
<210> 21
   <211> 978
   <212> DNA
   <213> Artificial Sequence
<220>
   <223> Construct of nucleotide sequence for extracellular domain of CD1d
<400> 21
   ggtaccgata tggggtgcct gctgtttctg ctgctctggg cgctcctcca ggcttgggga 60
   agcgctgaag tcccgcaaag gcttttcccc ctccgctgcc tccagatctc gtccttcgcc 120
   aatagcagct ggacgcgcac cgacggcttg gcgtggctgg gggagctgca gacgcacagc 180
   tggagcaacg actcggacac cgtccgctct ctgaagcctt ggtcccaggg cacgttcagc 240
   gaccagcagt gggagacgct gcagcatata tttcgggttt atcgaagcag cttcaccagg 300
   gacgtgaagg aattcgccaa aatgctacgc ttatcctatc ccttggagct ccaggtgtcc 360
   gctggctgtg aggtgcaccc tgggaacgcc tcaaataact tcttccatgt agcatttcaa 420
   ggaaaagata tcctgagttt ccaaggaact tcttgggagc caacccaaga ggccccactt 480
   tgggtaaact tggccattca agtgctcaac caggacaagt ggacgaggga aacagtgcag 540
   tggctcctta atggcacctg cccccaattt gtcagtggcc tccttgagtc agggaagtcg 600
   gaactgaaga agcaagtgaa gcccaaggcc tggctgtccc gtggccccag tcctggccct 660
   ggccgtctgc tgctggtgtg ccatgtctca ggattctacc caaagcctgt atgggtgaag 720
   tggatgcggg gtgagcagga gcagcagggc actcagccag gggacatcct gcccaatgct 780
   gacgagacat ggtatctccg agcaaccctg gatgtggtgg ctggggaggc agctggcctg 840
   tcctgtcggg tgaagcacag cagtctagag ggccaggaca tcgtcctcta ctggaccggt 900
   ggtggcggtc tgaacgacat cttcgaggct cagaaaatcg aatggcacga atccggtcat 960
   catcaccatc accattga 978
<210> 22
   <211> 322
   <212> PRT
   <213> Artificial Sequence
<220>
   <223> Construct of polypeptide sequence for extracellular domain of CD1d
<400> 22
   Met Gly Cys Leu Leu Phe Leu Leu Leu Trp Ala Leu Leu Gln Ala Trp
   1 5 10 15
   Gly Ser Ala Glu Val Pro Gln Arg Leu Phe Pro Leu Arg Cys Leu Gln
   20 25 30
   Ile Ser Ser Phe Ala Asn Ser Ser Trp Thr Arg Thr Asp Gly Leu Ala
   35 40 45
   Trp Leu Gly Glu Leu Gln Thr His Ser Trp Ser Asn Asp Ser Asp Thr
   50 55 60
   Val Arg Ser Leu Lys Pro Trp Ser Gln Gly Thr Phe Ser Asp Gln Gln
   65 70 75 80
   Trp Glu Thr Leu Gln His Ile Phe Arg Val Tyr Arg Ser Ser Phe Thr
   85 90 95
   Arg Asp Val Lys Glu Phe Ala Lys Met Leu Arg Leu Ser Tyr Pro Leu
   100 105 110
   Glu Leu Gln Val Ser Ala Gly Cys Glu Val His Pro Gly Asn Ala Ser
   115 120 125
   Asn Asn Phe Phe His Val Ala Phe Gln Gly Lys Asp Ile Leu Ser Phe
   130 135 140
   Gln Gly Thr Ser Trp Glu Pro Thr Gln Glu Ala Pro Leu Trp Val Asn
   145 150 155 160
   Leu Ala Ile Gln Val Leu Asn Gln Asp Lys Trp Thr Arg Glu Thr Val
   165 170 175
   Gln Trp Leu Leu Asn Gly Thr Cys Pro Gln Phe Val Ser Gly Leu Leu
   180 185 190
   Glu Ser Gly Lys Ser Glu Leu Lys Lys Gln Val Lys Pro Lys Ala Trp
   195 200 205
   Leu Ser Arg Gly Pro Ser Pro Gly Pro Gly Arg Leu Leu Leu Val Cys
   210 215 220
   His Val Ser Gly Phe Tyr Pro Lys Pro Val Trp Val Lys Trp Met Arg
   225 230 235 240
   Gly Glu Gln Glu Gln Gln Gly Thr Gln Pro Gly Asp Ile Leu Pro Asn
   245 250 255
   Ala Asp Glu Thr Trp Tyr Leu Arg Ala Thr Leu Asp Val Val Ala Gly
   260 265 270
   Glu Ala Ala Gly Leu Ser Cys Arg Val Lys His Ser Ser Leu Glu Gly
   275 280 285
   Gln Asp Ile Val Leu Tyr Trp Thr Gly Gly Gly Gly Leu Asn Asp Ile
   290 295 300
   Phe Glu Ala Gln Lys Ile Glu Trp His Glu Ser Gly His His His His
   305 310 315 320
   His His
<210> 23
   <211> 38
   <212> DNA
   <213> Artificial Sequence
<220>
   <223> F(ab)-B2M sense construct
<400> 23
   aattgcggcc gcaaaccatg ggatggagct gtatcatc 38
<210> 24
   <211> 45
   <212> DNA
   <213> Artificial Sequence
<220>
   <223> F9ab)-B2M antisense construct
<400> 24
   cggggtacct gacccaccgc ctcctttctt gtccaccttg gtgtt 45
<210> 25
   <211> 37
   <212> DNA
   <213> Artificial Sequence
<220>
   <223> F(ab')2-B2M sense construct
<400> 25
   attgcggccg caaaccatgg gatggagctg tatcatc 37
<210> 26
   <211> 45
   <212> DNA
   <213> Artificial Sequence
<220>
   <223> F(ab')2-B2M antisense construct
<400> 26
   cggggtacct gacccaccgc ctcctgggca cggtgggcat gtgtg 45
<210> 27
   <211> 38
   <212> DNA
   <213> Artificial Sequence
<220>
   <223> IgG1-B2M sense construct
<400> 27
   aattgcggcc gcaaaccatg ggatggagct gtatcatc 38
<210> 28
   <211> 45
   <212> DNA
   <213> Artificial Sequence
<220>
   <223> IgG1-B2M antisense construct
<400> 28
   cggggtacct gacccaccgc ctcctttacc cggagacagg gagag 45
<210> 29
   <211> 39
   <212> DNA
   <213> Artificial Sequence
<220>
   <223> artificial sense primer to construct beta2-microglobulin
<400> 29
   cggggtaccg gaggcggtgg gtcaatccag cgtactcca 39
<210> 30
   <211> 26
   <212> DNA
   <213> Artificial Sequence
<220>
   <223> artificial antisense primer to construct beta2-microglobulin
<400> 30
   cgaccggtca tgtctcgatc ccactt 26
<210> 31
   <211> 736
   <212> DNA
   <213> Artificial Sequence
<220>
   <223> Nucleotide sequence of the chimeric F(ab)-beta2-microglobulin
<400> 31
   gcggccgcaa accatgggat ggagctgtat catcctcttc ttggtagcaa cagctacagg      60
   cgcgcatatg gtcaccgtct cctcagcctc caccaagggc ccatcggtct tccccctggc     120
   accctcctcc aagagcacct ctgggggcac agcggccctg ggctgcctgg tcaaggacta   180
   cttccccgaa ccggtgacgg tgtcgtggaa ctcaggcgcc ctgaccagcg gcgtgcacac   240
   cttcccggct gtcctacagt cctcaggact ctactccctc agcagcgtcg tgaccgtgcc   300
   ctccagcagc ttgggcaccc agacctacat ctgcaacgtg aatcacaagc ccagcaacac   360
   caaggtggac aagaaaggag gcggtgggtc aggtaccgga ggcggtgggt caatccagcg   420
   tactccaaag attcaggttt actcacgtca tccagcagag aatggaaagt caaatttcct   480
   gaattgctat gtgtctgggt ttcatccatc cgacattgaa gttgacttac tgaagaatgg   540
   agagagaatt gaaaaagtgg agcattcaga cttgtctttc agcaaggact ggtctttcta   600
   tctcttgtac tacactgaat tcacccccac tgaaaaagat gagtatgcct gccgtgtgaa   660
   ccatgtgact ttgtcacagc ccaagatagt taagtgggat cgagacatga ccggtcatca   720
   tcaccatcac cattga                                                   736
<210> 32
   <211> 240
   <212> PRT
   <213> Artificial Sequence
<220>
   <223> Polypeptide sequence of the chimeric F(ab)-beta2-microglobulin
<400> 32
   Met Gly Trp Ser Cys Ile Ile Leu Phe Leu Val Ala Thr Ala Thr Gly
   1               5                   10                  15
   Ala His Met Val Thr Val Ser Ser Ala Ser Thr Lys Gly Pro Ser Val
               20                  25                  30
   Phe Pro Leu Ala Pro Ser Ser Lys Ser Thr Ser Gly Gly Thr Ala Ala
           35                  40                  45
   Leu Gly Cys Leu Val Lys Asp Tyr Phe Pro Glu Pro Val Thr Val Ser
       50                  55                  60
   Trp Asn Ser Gly Ala Leu Thr Ser Gly Val His Thr Phe Pro Ala Val
   65                  70                  75                  80
   Leu Gln Ser Ser Gly Leu Tyr Ser Leu Ser Ser Val Val Thr Val Pro
                   85                  90                  95
   Ser Ser Ser Leu Gly Thr Gln Thr Tyr Ile Cys Asn Val Asn His Lys
   100 105 110
   Pro Ser Asn Thr Lys Val Asp Lys Lys Gly Gly Gly Gly Ser Gly Thr
   115 120 125
   Gly Gly Gly Gly Ser Ile Gln Arg Thr Pro Lys Ile Gln Val Tyr Ser
   130 135 140
   Arg His Pro Ala Glu Asn Gly Lys Ser Asn Phe Leu Asn Cys Tyr Val
   145 150 155 160
   Ser Gly Phe His Pro Ser Asp Ile Glu Val Asp Leu Leu Lys Asn Gly
   165 170 175
   Glu Arg Ile Glu Lys Val Glu His Ser Asp Leu Val Phe Ser Lys Asp
   180 185 190
   Trp Ser Phe Tyr Leu Leu Tyr Tyr Thr Glu Phe Thr Pro Thr Glu Lys
   195 200 205
   Asp Glu Tyr Ala Cys Arg Val Asn His Val Thr Leu Ser Gln Pro Lys
   210 215 220
   Ile Val Lys Trp Asp Arg Asp Met Thr Gly His His His His His His
   225 230 235 240
<210> 33
   <211> 783
   <212> DNA
   <213> Artificial Sequence
<220>
   <223> Nucleotide sequence of the chimeric F(ab')2-beta2-microglobulin
<400> 33
   gcggccgcaa accatgggat ggagctgtat catcctcttc ttggtagcaa cagctacagg 60
   cgcgcatatg gtcaccgtct cctcagcctc caccaagggc ccatcggtct tccccctggc 120
   accctcctcc aagagcacct ctgggggcac agcggccctg ggctgcctgg tcaaggacta 180
   cttccccgaa ccggtgacgg tgtcgtggaa ctcaggcgcc ctgaccagcg gcgtgcacac 240
   cttcccggct gtcctacagt cctcaggact ctactccctc agcagcgtcg tgaccgtgcc 300
   ctccagcagc ttgggcaccc agacctacat ctgcaacgtg aatcacaagc ccagcaacac 360
   caaggtggac aagaaagttg agcccaaatc ttgtgacaaa actcacacat gcccaccgtg 420
   cccaggaggc ggtgggtcag gtaccggagg cggtgggtca atccagcgta ctccaaagat 480
   tcaggtttac tcacgtcatc cagcagagaa tggaaagtca aatttcctga attgctatgt 540
   gtctgggttt catccatccg acattgaagt tgacttactg aagaatggag agagaattga      600
   aaaagtggag cattcagact tgtctttcag caaggactgg tctttctatc tcttgtacta      660
   cactgaattc acccccactg aaaagatgag tatgcctgcc gtgtgaacca tgtgactttg      720
   tcacagccca agatagttaa gtgggatcga gacatgaccg gtcatcatca ccatcaccat      780
   tga                                                                    783
<210> 34
   <211> 256
   <212> PRT
   <213> Artificial Sequence
<220>
   <223> Nucleotide sequence of the chimeric F(ab')2-beta2-microglobulin
<400> 34
   Met Gly Trp Ser Cys Ile Ile Leu Phe Leu Val Ala Thr Ala Thr Gly
   1               5                   10                  15
   Ala His Met Val Thr Val Ser Ser Ala Ser Thr Lys Gly Pro Ser Val
               20                  25                  30
   Phe Pro Leu Ala Pro Ser Ser Lys Ser Thr Ser Gly Gly Thr Ala Ala
           35                  40                  45
   Leu Gly Cys Leu Val Lys Asp Tyr Phe Pro Glu Pro Val Thr Val Ser
       50                  55                  60
   Trp Asn Ser Gly Ala Leu Thr Ser Gly Val His Thr Phe Pro Ala Val
   65                  70                  75                  80
   Leu Gln Ser Ser Gly Leu Tyr Ser Leu Ser Ser Val Val Thr Val Pro
                   85                  90                  95
   Ser Ser Ser Leu Gly Thr Gln Thr Tyr Ile Cys Asn Val Asn His Lys
               100                 105                 110
   Pro Ser Asn Thr Lys Val Asp Lys Lys Val Glu Pro Lys Ser Cys Asp
           115                 120                 125
   Lys Thr His Thr Cys Pro Pro Cys Pro Gly Gly Gly Gly Ser Gly Thr
       130                 135                 140
   Gly Gly Gly Gly Ser Ile Gln Arg Thr Pro Lys Ile Gln Val Tyr Ser
   145                 150                 155                 160
   Arg His Pro Ala Glu Asn Gly Lys Ser Asn Phe Leu Asn Cys Tyr Val
   165 170 175
   Ser Gly Phe His Pro Ser Asp Ile Glu Val Asp Leu Leu Lys Asn Gly
   180 185 190
   Glu Arg Ile Glu Lys Val Glu His Ser Asp Leu Val Phe Ser Lys Asp
   195 200 205
   Trp Ser Phe Tyr Leu Leu Tyr Tyr Thr Glu Phe Thr Pro Thr Glu Lys
   210 215 220
   Asp Glu Tyr Ala Cys Arg Val Asn His Val Thr Leu Ser Gln Pro Lys
   225 230 235 240
   Ile Val Lys Trp Asp Arg Asp Met Thr Gly His His His His His His
   245 250 255
<210> 35
   <211> 1435
   <212> DNA
   <213> Artificial Sequence
<220>
   <223> Nucleotide sequence of the chimeric full IgG1-beta2 microglobulin
<400> 35
   gcggccgcaa accatgggat ggagctgtat catcctcttc ttggtagcaa cagctacagg 60
   cgcgcatatg gtcaccgtct cctcagcctc caccaagggc ccatcggtct tccccctggc 120
   accctcctcc aagagcacct ctgggggcac agcggccctg ggctgcctgg tcaaggacta 180
   cttccccgaa ccggtgacgg tgtcgtggaa ctcaggcgcc ctgaccagcg gcgtgcacac 240
   cttcccggct gtcctacagt cctcaggact ctactccctc agcagcgtcg tgaccgtgcc 300
   ctccagcagc ttgggcaccc agacctacat ctgcaacgtg aatcacaagc ccagcaacac 360
   caaggtggac aagaaagttg agcccaaatc ttgtgacaaa actcacacat gcccaccgtg 420
   cccagcacct gaactcctgg ggggaccgtc agtcttcctc ttccccccaa aacccaagga 480
   caccctcatg atctcccgga cccctgaggt cacatgcgtg gtggtggacg tgagccacga 540
   agaccctgag gtcaagttca actggtacgt ggacggcgtg gaggtgcata atgccaagac 600
   aaagccgcgg gaggagcagt acaacagcac gtaccgtgtg gtcagcgtcc tcaccgtcct 660
   gcaccaggac tggctgaatg gcaaggagta caagtgcaag gtctccaaca aagccctccc 720
   agcccccatc gagaaaacca tctccaaagc caaagggcag ccccgagaac cacaggtgta 780
   caccctgccc ccatcccggg atgagctgac caagaaccag gtcagcctga cctgcctggt 840
   caaaggcttc tatcccagcg acatcgccgt ggagtgggag agcaatgggc agccggagaa 900
   caactacaag accacgcctc ccgtgctgga ctccgacggc tccttcttcc tctacagcaa 960
   gctcaccgtg gacaagagca ggtggcagca ggggaacgtc ttctcatgct ccgtgatgca 1020
   tgaggctctg cacaaccact acacgcagaa gagcctctcc ctgtctccgg gtaaaggagg 1080
   cggtgggtca ggtaccggag gcggtgggtc aatccagcgt actccaaaga ttcaggttta 1140
   ctcacgtcat ccagcagaga atggaaagtc aaatttcctg aattgctatg tgtctgggtt 1200
   tcatccatcc gacattgaag ttgacttact gaagaatgga gagagaattg aaaaagtgga 1260
   gcattcagac ttgtctttca gcaaggactg gtctttctat ctcttgtact acactgaatt 1320
   cacccccact gaaaaagatg agtatgcctg ccgtgtgaac catgtgactt tgtcacagcc 1380
   caagatagtt aagtgggatc gagacatgac cggtcatcat caccatcacc attga 1435
<210> 36
   <211> 473
   <212> PRT
   <213> Artificial Sequence
<220>
   <223> Nucleotide sequence of the chimeric full IgG1-beta2 microglobulin
<400> 36
   Met Gly Trp Ser Cys Ile Ile Leu Phe Leu Val Ala Thr Ala Thr Gly
   1 5 10 15
   Ala His Met Val Thr Val Ser Ser Ala Ser Thr Lys Gly Pro Ser Val
   20 25 30
   Phe Pro Leu Ala Pro Ser Ser Lys Ser Thr Ser Gly Gly Thr Ala Ala
   35 40 45
   Leu Gly Cys Leu Val Lys Asp Tyr Phe Pro Glu Pro Val Thr Val Ser
   50 55 60
   Trp Asn Ser Gly Ala Leu Thr Ser Gly Val His Thr Phe Pro Ala Val
   65 70 75 80
   Leu Gln Ser Ser Gly Leu Tyr Ser Leu Ser Ser Val Val Thr Val Pro
   85 90 95
   Ser Ser Ser Leu Gly Thr Gln Thr Tyr Ile Cys Asn Val Asn His Lys
   100 105 110
   Pro Ser Asn Thr Lys Val Asp Lys Lys Val Glu Pro Lys Ser Cys Asp
   115 120 125
   Lys Thr His Thr Cys Pro Pro Cys Pro Ala Pro Glu Leu Leu Gly Gly
           130                 135                 140
   Pro Ser Val Phe Leu Phe Pro Pro Lys Pro Lys Asp Thr Leu Met Ile
   145                 150                 155                 160
   Ser Arg Thr Pro Glu Val Thr Cys Val Val Val Asp Val Ser His Glu
                   165                 170                 175
   Asp Pro Glu Val Lys Phe Asn Trp Tyr Val Asp Gly Val Glu Val His
               180                 185                 190
   Asn Ala Lys Thr Lys Pro Arg Glu Glu Gln Tyr Asn Ser Thr Tyr Arg
           195                 200                 205
   Val Val Ser Val Leu Thr Val Leu His Gln Asp Trp Leu Asn Gly Lys
       210                 215                 220
   Glu Tyr Lys Cys Lys Val Ser Asn Lys Ala Leu Pro Ala Pro Ile Glu
   225                 230                 235                 240
   Lys Thr Ile Ser Lys Ala Lys Gly Gln Pro Arg Glu Pro Gln Val Tyr
                   245                 250                 255
   Thr Leu Pro Pro Ser Arg Asp Glu Leu Thr Lys Asn Gln Val Ser Leu
               260                 265                 270
   Thr Cys Leu Val Lys Gly Phe Tyr Pro Ser Asp Ile Ala Val Glu Trp
           275                 280                 285
   Glu Ser Asn Gly Gln Pro Glu Asn Asn Tyr Lys Thr Thr Pro Pro Val
       290                 295                 300
   Leu Asp Ser Asp Gly Ser Phe Phe Leu Tyr Ser Lys Leu Thr Val Asp
   305                 310                 315                 320
   Lys Ser Arg Trp Gln Gln Gly Asn Val Phe Ser Cys Ser Val Met His
                   325                 330                 335
   Glu Ala Leu His Asn His Tyr Thr Gln Lys Ser Leu Ser Leu Ser Pro
               340                 345                 350
   Gly Lys Gly Gly Gly Gly Ser Gly Thr Gly Gly Gly Gly Ser Ile Gln
           355                 360                 365
   Arg Thr Pro Lys Ile Gln Val Tyr Ser Arg His Pro Ala Glu Asn Gly
       370                 375                 380
   Lys Ser Asn Phe Leu Asn Cys Tyr Val Ser Gly Phe His Pro Ser Asp
   385 390 395 400
   Ile Glu Val Asp Leu Leu Lys Asn Gly Glu Arg Ile Glu Lys Val Glu
   405 410 415
   His Ser Asp Leu Val Phe Ser Lys Asp Trp Ser Phe Tyr Leu Leu Tyr
   420 425 430
   Tyr Thr Glu Phe Thr Pro Thr Glu Lys Asp Glu Tyr Ala Cys Arg Val
   435 440 445
   Asn His Val Thr Leu Ser Gln Pro Lys Ile Val Lys Trp Asp Arg Asp
   450 455 460
   Met Thr Gly His His His His His His
   465 470
<210> 37
   <211> 34
   <212> > DNA
   <213> Artificial Sequence
<220>
   <223> Primer E1 Sense
<400> 37
   cacggtaccg atatggggtg cctgctgttt ctgc 34
<210> 38
   <211> 30
   <212> DNA
   <213> Artificial Sequence
<220>
   <223> Primer E1 antisense
<400> 38
   cagaccggtc cagtagagga cgatgtcctg 30
<210> 39
   <211> 921
   <212> DNA
   <213> Artificial Sequence
<220>
   <223> Construct of extracellular domain of CD1d
<400> 39
   ggtaccgata tggggtgcct gctgtttctg ctgctctggg cgctcctcca ggcttgggga 60
   agcgctgaag tcccgcaaag gcttttcccc ctccgctgcc tccagatctc gtccttcgcc 120
   aatagcagct ggacgcgcac cgacggcttg gcgtggctgg gggagctgca gacgcacagc 180
   tggagcaacg actcggacac cgtccgctct ctgaagcctt ggtcccaggg cacgttcagc 240
   gaccagcagt gggagacgct gcagcatata tttcgggttt atcgaagcag cttcaccagg 300
   gacgtgaagg aattcgccaa aatgctacgc ttatcctatc ccttggagct ccaggtgtcc 360
   gctggctgtg aggtgcaccc tgggaacgcc tcaaataact tcttccatgt agcatttcaa 420
   ggaaaagata tcctgagttt ccaaggaact tcttgggagc caacccaaga ggccccactt 480
   tgggtaaact tggccattca agtgctcaac caggacaagt ggacgaggga aacagtgcag 540
   tggctcctta atggcacctg cccccaattt gtcagtggcc tccttgagtc agggaagtcg 600
   gaactgaaga agcaagtgaa gcccaaggcc tggctgtccc gtggccccag tcctggccct 660
   ggccgtctgc tgctggtgtg ccatgtctca ggattctacc caaagcctgt atgggtgaag 720
   tggatgcggg gtgagcagga gcagcagggc actcagccag gggacatcct gcccaatgct 780
   gacgagacat ggtatctccg agcaaccctg gatgtggtgg ctggggaggc agctggcctg 840
   tcctgtcggg tgaagcacag cagtctagag ggccaggaca tcgtcctcta ctggaccggt 900
   catcatcacc atcaccattg a 921
<210> 40
   <211> 303
   <212> PRT
   <213> Artificial Sequence
<220>
   <223> Construct of extracellular domain of CD1d
<400> 40
   Met Gly Cys Leu Leu Phe Leu Leu Leu Trp Ala Leu Leu Gln Ala Trp
   1 5 10 15
   Gly Ser Ala Glu Val Pro Gln Arg Leu Phe Pro Leu Arg Cys Leu Gln
   20 25 30
   Ile Ser Ser Phe Ala Asn Ser Ser Trp Thr Arg Thr Asp Gly Leu Ala
   35 40 45
   Trp Leu Gly Glu Leu Gln Thr His Ser Trp Ser Asn Asp Ser Asp Thr
   50 55 60
   Val Arg Ser Leu Lys Pro Trp Ser Gln Gly Thr Phe Ser Asp Gln Gln
   65 70 75 80
   Trp Glu Thr Leu Gln His Ile Phe Arg Val Tyr Arg Ser Ser Phe Thr
   85 90 95
   Arg Asp Val Lys Glu Phe Ala Lys Met Leu Arg Leu Ser Tyr Pro Leu
   100 105 110
   Glu Leu Gln Val Ser Ala Gly Cys Glu Val His Pro Gly Asn Ala Ser
   115 120 125
   Asn Asn Phe Phe His Val Ala Phe Gln Gly Lys Asp Ile Leu Ser Phe
   130 135 140
   Gln Gly Thr Ser Trp Glu Pro Thr Gln Glu Ala Pro Leu Trp Val Asn
   145 150 155 160
   Leu Ala Ile Gln Val Leu Asn Gln Asp Lys Trp Thr Arg Glu Thr Val
   165 170 175
   Gln Trp Leu Leu Asn Gly Thr Cys Pro Gln Phe Val Ser Gly Leu Leu
   180 185 190
   Glu Ser Gly Lys Ser Glu Leu Lys Lys Gln Val Lys Pro Lys Ala Trp
   195 200 205
   Leu Ser Arg Gly Pro Ser Pro Gly Pro Gly Arg Leu Leu Leu Val Cys
   210 215 220
   His Val Ser Gly Phe Tyr Pro Lys Pro Val Trp Val Lys Trp Met Arg
   225 230 235 240
   Gly Glu Gln Glu Gln Gln Gly Thr Gln Pro Gly Asp Ile Leu Pro Asn
   245 250 255
   Ala Asp Glu Thr Trp Tyr Leu Arg Ala Thr Leu Asp Val Val Ala Gly
   260 265 270
   Glu Ala Ala Gly Leu Ser Cys Arg Val Lys His Ser Ser Leu Glu Gly
   275 280 285
   Gln Asp Ile Val Leu Tyr Trp Thr Gly His His His His His His
   290 295 300
<210> 41
   <211> 38
   <212> DNA
   <213> Artificial Sequence
<220>
   <223> F(ab)-CD1d sense fragment
<400> 41
   aattgcggcc gcaaaccatg ggatggagct gtatcatc 38
<210> 42
   <211> 45
   <212> DNA
   <213> Artificial Sequence
<220>
   <223> F(ab)-CD1d antisense fragment
<400> 42
   cggggtacct gacccaccgc ctcctttctt gtccaccttg gtgtt 45
<210> 43
   <211> 38
   <212> DNA
   <213> Artificial Sequence
<220>
   <223> F(ab')2-CD1d sense fragment
<400> 43
   aattgcggcc gcaaaccatg ggatggagct gtatcatc 38
<210> 44
   <211> 45
   <212> DNA
   <213> Artificial Sequence
<220>
   <223> F(ab')2-CD1d antisense fragment
<400> 44.
   cggggtacct gacccaccgc ctcctgggca cggtgggcat gtgtg 45
<210> 45
   <211> 38
   <212> DNA
   <213> Artificial Sequence
<220>
   <223> IgG1-CD1d sense fragment
<400> 45
   aattgcggcc gcaaaccatg ggatggagct gtatcatc 38
<210> 46
   <211> 45
   <212> DNA
   <213> Artificial Sequence
<220>
   <223> IgG1-CD1d antisense fragment
<400> 46
   cggggtacct gacccaccgc ctcctttacc cggagacagg gagag 45
<210> 47
   <211> 42
   <212> DNA
   <213> Artificial Sequence
<220>
   <223> Artificial sense primer to construct extracellular CD1d
<400> 47
   cggggtaccg gaggcggtgg gtcagtcccg caaaggcttt tc 42
<210> 48
   <211> 29
   <212> DNA
   <213> Artificial Sequence
<220>
   <223> Artificial antisense primer to construct extracellular CD1d
<400> 48
   cgaccggtcc agtagaggac gatgtcctg 29
<210> 49
   <211> 1264
   <212> DNA
   <213> Artificial Sequence
<220>
   <223> Nucleotide sequence of the chimeric F(ab)-CD1d product
<400> 49
   gcggccgcaa accatgggat ggagctgtat catcctcttc ttggtagcaa cagctacagg 60
   cgcgcatatg gtcaccgtct cctcagcctc caccaagggc ccatcggtct tccccctggc 120
   accctcctcc aagagcacct ctgggggcac agcggccctg ggctgcctgg tcaaggacta 180
   cttccccgaa ccggtgacgg tgtcgtggaa ctcaggcgcc ctgaccagcg gcgtgcacac 240
   cttcccggct gtcctacagt cctcaggact ctactccctc agcagcgtcg tgaccgtgcc 300
   ctccagcagc ttgggcaccc agacctacat ctgcaacgtg aatcacaagc ccagcaacac 360
   caaggtggac aagaaaggag gcggtgggtc aggtaccgga ggcggtgggt cagtcccgca 420
   aaggcttttc cccctccgct gcctccagat ctcgtccttc gccaatagca gctggacgcg 480
   caccgacggc ttggcgtggc tgggggagct gcagacgcac agctggagca acgactcgga 540
   caccgtccgc tctctgaagc cttggtccca gggcacgttc agcgaccagc agtgggagac 600
   gctgcagcat atatttcggg tttatcgaag cagcttcacc agggacgtga aggaattcgc 660
   caaaatgcta cgcttatcct atcccttgga gctccaggtg tccgctggct gtgaggtgca 720
   ccctgggaac gcctcaaata acttcttcca tgtagcattt caaggaaaag atatcctgag 780
   tttccaagga acttcttggg agccaaccca agaggcccca ctttgggtaa acttggccat 840
   tcaagtgctc aaccaggaca agtggacgag ggaaacagtg cagtggctcc ttaatggcac 900
   ctgcccccaa tttgtcagtg gcctccttga gtcagggaag tcggaactga agaagcaagt 960
   gaagcccaag gcctggctgt cccgtggccc cagtcctggc cctggccgtc tgctgctggt 1020
   gtgccatgtc tcaggattct acccaaagcc tgtatgggtg aagtggatgc ggggtgagca 1080
   ggagcagcag ggcactcagc caggggacat cctgcccaat gctgacgaga catggtatct 1140
   ccgagcaacc ctggatgtgg tggctgggga ggcagctggc ctgtcctgtc gggtgaagca 1200
   cagcagtcta gagggccagg acatcgtcct ctactggacc ggtcatcatc accatcacca 1260
   ttga 1264
<210> 50
   <211> 416
   <212> PRT
   <213> Artificial Sequence
<220>
   <223> Polypeptide sequence of the chimeric F(ab)-CD1d product
<400> 50
   Met Gly Trp Ser Cys Ile Ile Leu Phe Leu Val Ala Thr Ala Thr Gly
   1 5 10 15
   Ala His Met Val Thr Val Ser Ser Ala Ser Thr Lys Gly Pro Ser Val
   20 25 30
   Phe Pro Leu Ala Pro Ser Ser Lys Ser Thr Ser Gly Gly Thr Ala Ala
   35 40 45
   Leu Gly Cys Leu Val Lys Asp Tyr Phe Pro Glu Pro Val Thr Val Ser
   50 55 60
   Trp Asn Ser Gly Ala Leu Thr Ser Gly Val His Thr Phe Pro Ala Val
   65 70 75 80
   Leu Gln Ser Ser Gly Leu Tyr Ser Leu Ser Ser Val Val Thr Val Pro
   85 90 95
   Ser Ser Ser Leu Gly Thr Gln Thr Tyr Ile Cys Asn Val Asn His Lys
   100 105 110
   Pro Ser Asn Thr Lys Val Asp Lys Lys Gly Gly Gly Gly Ser Gly Thr
   115 120 125
   Gly Gly Gly Gly Ser Val Pro Gln Arg Leu Phe Pro Leu Arg Cys Leu
   130 135 140
   Gln Ile Ser Ser Phe Ala Asn Ser Ser Trp Thr Arg Thr Asp Gly Leu
   145 150 155 160
   Ala Trp Leu Gly Glu Leu Gln Thr His Ser Trp Ser Asn Asp Ser Asp
   165 170 175
   Thr Val Arg Ser Leu Lys Pro Trp Ser Gln Gly Thr Phe Ser Asp Gln
   180 185 190
   Gln Trp Glu Thr Leu Gln His Ile Phe Arg Val Tyr Arg Ser Ser Phe
   195 200 205
   Thr Arg Asp Val Lys Glu Phe Ala Lys Met Leu Arg Leu Ser Tyr Pro
   210 215 220
   Leu Glu Leu Gln Val Ser Ala Gly Cys Glu Val His Pro Gly Asn Ala
   225 230 235 240
   Ser Asn Asn Phe Phe His Val Ala Phe Gln Gly Lys Asp Ile Leu Ser
   245 250 255
   Phe Gln Gly Thr Ser Trp Glu Pro Thr Gln Glu Ala Pro Leu Trp Val
   260 265 270
   Asn Leu Ala Ile Gln Val Leu Asn Gln Asp Lys Trp Thr Arg Glu Thr
   275 280 285
   Val Gln Trp Leu Leu Asn Gly Thr Cys Pro Gln Phe Val Ser Gly Leu
   290 295 300
   Leu Glu Ser Gly Lys Ser Glu Leu Lys Lys Gln Val Lys Pro Lys Ala
   305 310 315 320
   Trp Leu Ser Arg Gly Pro Ser Pro Gly Pro Gly Arg Leu Leu Leu Val
   325 330 335
   Cys His Val Ser Gly Phe Tyr Pro Lys Pro Val Trp Val Lys Trp Met
   340 345 350
   Arg Gly Glu Gln Glu Gln Gln Gly Thr Gln Pro Gly Asp Ile Leu Pro
   355 360 365
   Asn Ala Asp Glu Thr Trp Tyr Leu Arg Ala Thr Leu Asp Val Val Ala
   370 375 380
   Gly Glu Ala Ala Gly Leu Ser Cys Arg Val Lys His Ser Ser Leu Glu
   385 390 395 400
   Gly Gln Asp Ile Val Leu Tyr Trp Thr Gly His His His His His His
   405 410 415
<210> 51
   <211> 1312
   <212> DNA
   <213> Artificial Sequence
<220>
   <223> Nucleotide sequence of the chimeric F(ab')2-CD1d product
<400> 51
   gcggccgcaa accatgggat ggagctgtat catcctcttc ttggtagcaa cagctacagg      60
   cgcgcatatg gtcaccgtct cctcagcctc caccaagggc ccatcggtct tccccctggc     120
   accctcctcc aagagcacct ctgggggcac agcggccctg ggctgcctgg tcaaggacta     180
   cttccccgaa ccggtgacgg tgtcgtggaa ctcaggcgcc ctgaccagcg gcgtgcacac     240
   cttcccggct gtcctacagt cctcaggact ctactccctc agcagcgtcg tgaccgtgcc     300
   ctccagcagc ttgggcaccc agacctacat ctgcaacgtg aatcacaagc ccagcaacac     360
   caaggtggac aagaaagttg agcccaaatc ttgtgacaaa actcacacat gcccaccgtg     420
   cccaggaggc ggtgggtcag gtaccggagg cggtgggtca gtcccgcaaa ggcttttccc     480
   cctccgctgc ctccagatct cgtccttcgc caatagcagc tggacgcgca ccgacggctt     540
   ggcgtggctg ggggagctgc agacgcacag ctggagcaac gactcggaca ccgtccgctc     600
   tctgaagcct tggtcccagg gcacgttcag cgaccagcag tgggagacgc tgcagcatat     660
   atttcgggtt tatcgaagca gcttcaccag ggacgtgaag gaattcgcca aaatgctacg     720
   cttatcctat cccttggagc tccaggtgtc cgctggctgt gaggtgcacc ctgggaacgc     780
   ctcaaataac ttcttccatg tagcatttca aggaaaagat atcctgagtt tccaaggaac     840
   ttcttgggag ccaacccaag aggccccact ttgggtaaac ttggccattc aagtgctcaa      900
   ccaggacaag tggacgaggg aaacagtgca gtggctcctt aatggcacct gcccccaatt     960
   tgtcagtggc ctccttgagt cagggaagtc ggaactgaag aagcaagtga agcccaaggc    1020
   ctggctgtcc cgtggcccca gtcctggccc tggccgtctg ctgctggtgt gccatgtctc    1080
   aggattctac ccaaagcctg tatgggtgaa gtggatgcgg ggtgagcagg agcagcaggg    1140
   cactcagcca ggggacatcc tgcccaatgc tgacgagaca tggtatctcc gagcaaccct    1200
   ggatgtggtg gctggggagg cagctggcct gtcctgtcgg gtgaagcaca gcagtctaga    1260
   gggccaggac atcgtcctct actggaccgg tcatcatcac catcaccatt ga            1312
<210> 52
   <211> 432
   <212> PRT
   <213> Artificial Sequence
<220>
   <223> Polypeptide sequence of the chimeric F(ab')2-CD1d product
<400> 52
   Met Gly Trp Ser Cys Ile Ile Leu Phe Leu Val Ala Thr Ala Thr Gly
   1               5                   10                  15
   Ala His Met Val Thr Val Ser Ser Ala Ser Thr Lys Gly Pro Ser Val
               20                  25                  30
   Phe Pro Leu Ala Pro Ser Ser Lys Ser Thr Ser Gly Gly Thr Ala Ala
           35                  40                  45
   Leu Gly Cys Leu Val Lys Asp Tyr Phe Pro Glu Pro Val Thr Val Ser
       50                  55                  60
   Trp Asn Ser Gly Ala Leu Thr Ser Gly Val His Thr Phe Pro Ala Val
   65                  70                  75                  80
   Leu Gln Ser Ser Gly Leu Tyr Ser Leu Ser Ser Val Val Thr Val Pro
                   85                  90                  95
   Ser Ser Ser Leu Gly Thr Gln Thr Tyr Ile Cys Asn Val Asn His Lys
               100                 105                 110
   Pro Ser Asn Thr Lys Val Asp Lys Lys Val Glu Pro Lys Ser Cys Asp
           115                 120                 125
   Lys Thr His Thr Cys Pro Pro Cys Pro Gly Gly Gly Gly Ser Gly Thr
       130                 135                 140
   Gly Gly Gly Gly Ser Val Pro Gln Arg Leu Phe Pro Leu Arg Cys Leu
   145                 150                 155                 160
   Gln Ile Ser Ser Phe Ala Asn Ser Ser Trp Thr Arg Thr Asp Gly Leu
                   165                 170                 175
   Ala Trp Leu Gly Glu Leu Gln Thr His Ser Trp Ser Asn Asp Ser Asp
               180                 185                 190
   Thr Val Arg Ser Leu Lys Pro Trp Ser Gln Gly Thr Phe Ser Asp Gln
           195                 200                 205
   Gln Trp Glu Thr Leu Gln His Ile Phe Arg Val Tyr Arg Ser Ser Phe
       210                 215                 220
   Thr Arg Asp Val Lys Glu Phe Ala Lys Met Leu Arg Leu Ser Tyr Pro
   225                 230                 235                 240
   Leu Glu Leu Gln Val Ser Ala Gly Cys Glu Val His Pro Gly Asn Ala
   245 250 255
   Ser Asn Asn Phe Phe His Val Ala Phe Gln Gly Lys Asp Ile Leu Ser
   260 265 270
   Phe Gln Gly Thr Ser Trp Glu Pro Thr Gln Glu Ala Pro Leu Trp Val
   275 280 285
   Asn Leu Ala Ile Gln Val Leu Asn Gln Asp Lys Trp Thr Arg Glu Thr
   290 295 300
   Val Gln Trp Leu Leu Asn Gly Thr Cys Pro Gln Phe Val Ser Gly Leu
   305 310 315 320
   Leu Glu Ser Gly Lys Ser Glu Leu Lys Lys Gln Val Lys Pro Lys Ala
   325 330 335
   Trp Leu Ser Arg Gly Pro Ser Pro Gly Pro Gly Arg Leu Leu Leu Val
   340 345 350
   Cys His Val Ser Gly Phe Tyr Pro Lys Pro Val Trp Val Lys Trp Met
   355 360 365
   Arg Gly Glu Gln Glu Gln Gln Gly Thr Gln Pro Gly Asp Ile Leu Pro
   370 375 380
   Asn Ala Asp Glu Thr Trp Tyr Leu Arg Ala Thr Leu Asp Val Val Ala
   385 390 395 400
   Gly Glu Ala Ala Gly Leu Ser Cys Arg Val Lys His Ser Ser Leu Glu
   405 410 415
   Gly Gln Asp Ile Val Leu Tyr Trp Thr Gly His His His His His His
   420 425 430
<210> 53
   <211> 1963
   <212> DNA
   <213> Artificial Sequence
<220>
   <223> Nucleotide sequence of the chimeric IgG1-CD1d product
<400> 53
   gcggccgcaa accatgggat ggagctgtat catcctcttc ttggtagcaa cagctacagg 60
   cgcgcatatg gtcaccgtct cctcagcctc caccaagggc ccatcggtct tccccctggc 120
   accctcctcc aagagcacct ctgggggcac agcggccctg ggctgcctgg tcaaggacta 180
   cttccccgaa ccggtgacgg tgtcgtggaa ctcaggcgcc ctgaccagcg gcgtgcacac   240
   cttcccggct gtcctacagt cctcaggact ctactccctc agcagcgtcg tgaccgtgcc   300
   ctccagcagc ttgggcaccc agacctacat ctgcaacgtg aatcacaagc ccagcaacac   360
   caaggtggac aagaaagttg agcccaaatc ttgtgacaaa actcacacat gcccaccgtg   420
   cccagcacct gaactcctgg ggggaccgtc agtcttcctc ttccccccaa aacccaagga   480
   caccctcatg atctcccgga cccctgaggt cacatgcgtg gtggtggacg tgagccacga   540
   agaccctgag gtcaagttca actggtacgt ggacggcgtg gaggtgcata atgccaagac   600
   aaagccgcgg gaggagcagt acaacagcac gtaccgtgtg gtcagcgtcc tcaccgtcct   660
   gcaccaggac tggctgaatg gcaaggagta caagtgcaag gtctccaaca aagccctccc   720
   agcccccatc gagaaaacca tctccaaagc caaagggcag ccccgagaac cacaggtgta   780
   caccctgccc ccatcccggg atgagctgac caagaaccag gtcagcctga cctgcctggt   840
   caaaggcttc tatcccagcg acatcgccgt ggagtgggag agcaatgggc agccggagaa   900
   caactacaag accacgcctc ccgtgctgga ctccgacggc tccttcttcc tctacagcaa   960
   gctcaccgtg gacaagagca ggtggcagca ggggaacgtc ttctcatgct ccgtgatgca  1020
   tgaggctctg cacaaccact acacgcagaa gagcctctcc ctgtctccgg gtaaaggagg  1080
   cggtgggtca ggtaccggag gcggtgggtc agtcccgcaa aggcttttcc ccctccgctg  1140
   cctccagatc tcgtccttcg ccaatagcag ctggacgcgc accgacggct tggcgtggct  1200
   gggggagctg cagacgcaca gctggagcaa cgactcggac accgtccgct ctctgaagcc  1260
   ttggtcccag ggcacgttca gcgaccagca gtgggagacg ctgcagcata tatttcgggt  1320
   ttatcgaagc agcttcacca gggacgtgaa ggaattcgcc aaaatgctac gcttatccta  1380
   tcccttggag ctccaggtgt ccgctggctg tgaggtgcac cctgggaacg cctcaaataa  1440
   cttcttccat gtagcatttc aaggaaaaga tatcctgagt ttccaaggaa cttcttggga  1500
   gccaacccaa gaggccccac tttgggtaaa cttggccatt caagtgctca accaggacaa  1560
   gtggacgagg gaaacagtgc agtggctcct taatggcacc tgcccccaat ttgtcagtgg  1620
   cctccttgag tcagggaagt cggaactgaa gaagcaagtg aagcccaagg cctggctgtc  1680
   ccgtggcccc agtcctggcc ctggccgtct gctgctggtg tgccatgtct caggattcta  1740
   cccaaagcct gtatgggtga agtggatgcg gggtgagcag gagcagcagg gcactcagcc  1800
   aggggacatc ctgcccaatg ctgacgagac atggtatctc cgagcaaccc tggatgtggt  1860
   ggctggggag gcagctggcc tgtcctgtcg ggtgaagcac agcagtctag agggccagga  1920
   catcgtcctc tactggaccg gtcatcatca ccatcaccat tga                   1963
<210> 54
   <211> 649
   <212> PRT
   <213> Artificial Sequence
<220>
   <223> Polypeptide sequence of the IgG1CD1d product
<400> 54
   Met Gly Trp Ser Cys Ile Ile Leu Phe Leu Val Ala Thr Ala Thr Gly
   1               5                   10                  15
   Ala His Met Val Thr Val Ser Ser Ala Ser Thr Lys Gly Pro Ser Val
               20                  25                  30
   Phe Pro Leu Ala Pro Ser Ser Lys Ser Thr Ser Gly Gly Thr Ala Ala
           35                  40                  45
   Leu Gly Cys Leu Val Lys Asp Tyr Phe Pro Glu Pro Val Thr Val Ser
       50                  55                  60
   Trp Asn Ser Gly Ala Leu Thr Ser Gly Val His Thr Phe Pro Ala Val
   65                  70                  75                  80
   Leu Gln Ser Ser Gly Leu Tyr Ser Leu Ser Ser Val Val Thr Val Pro
                   85                  90                  95
   Ser Ser Ser Leu Gly Thr Gln Thr Tyr Ile Cys Asn Val Asn His Lys
               100                 105                 110
   Pro Ser Asn Thr Lys Val Asp Lys Lys Val Glu Pro Lys Ser Cys Asp
           115                 120                 125
   Lys Thr His Thr Cys Pro Pro Cys Pro Ala Pro Glu Leu Leu Gly Gly
       130                 135                 140
   Pro Ser Val Phe Leu Phe Pro Pro Lys Pro Lys Asp Thr Leu Met Ile
   145                 150                 155                 160
   Ser Arg Thr Pro Glu Val Thr Cys Val Val Val Asp Val Ser His Glu
                   165                 170                 175
   Asp Pro Glu Val Lys Phe Asn Trp Tyr Val Asp Gly Val Glu Val His
               180                 185                 190
   Asn Ala Lys Thr Lys Pro Arg Glu Glu Gln Tyr Asn Ser Thr Tyr Arg
           195                 200                 205
   Val Val Ser Val Leu Thr Val Leu His Gln Asp Trp Leu Asn Gly Lys
   210 215 220
   Glu Tyr Lys Cys Lys Val Ser Asn Lys Ala Leu Pro Ala Pro Ile Glu
   225 230 235 240
   Lys Thr Ile Ser Lys Ala Lys Gly Gln Pro Arg Glu Pro Gln Val Tyr
   245 250 255
   Thr Leu Pro Pro Ser Arg Asp Glu Leu Thr Lys Asn Gln Val Ser Leu
   260 265 270
   Thr Cys Leu Val Lys Gly Phe Tyr Pro Ser Asp Ile Ala Val Glu Trp
   275 280 285
   Glu Ser Asn Gly Gln Pro Glu Asn Asn Tyr Lys Thr Thr Pro Pro Val
   290 295 300
   Leu Asp Ser Asp Gly Ser Phe Phe Leu Tyr Ser Lys Leu Thr Val Asp
   305 310 315 320
   Lys Ser Arg Trp Gln Gln Gly Asn Val Phe Ser Cys Ser Val Met His
   325 330 335
   Glu Ala Leu His Asn His Tyr Thr Gln Lys Ser Leu Ser Leu Ser Pro
   340 345 350
   Gly Lys Gly Gly Gly Gly Ser Gly Thr Gly Gly Gly Gly Ser Val Pro
   355 360 365
   Gln Arg Leu Phe Pro Leu Arg Cys Leu Gln Ile Ser Ser Phe Ala Asn
   370 375 380
   Ser Ser Trp Thr Arg Thr Asp Gly Leu Ala Trp Leu Gly Glu Leu Gln
   385 390 395 400
   Thr His Ser Trp Ser Asn Asp Ser Asp Thr Val Arg Ser Leu Lys Pro
   405 410 415
   Trp Ser Gln Gly Thr Phe Ser Asp Gln Gln Trp Glu Thr Leu Gln His
   420 425 430
   Ile Phe Arg Val Tyr Arg Ser Ser Phe Thr Arg Asp Val Lys Glu Phe
   435 440 445
   Ala Lys Met Leu Arg Leu Ser Tyr Pro Leu Glu Leu Gln Val Ser Ala
   450 455 460
   Gly Cys Glu Val His Pro Gly Asn Ala Ser Asn Asn Phe Phe His Val
   465 470 475 480
   Ala Phe Gln Gly Lys Asp Ile Leu Ser Phe Gln Gly Thr Ser Trp Glu
   485 490 495
   Pro Thr Gln Glu Ala Pro Leu Trp Val Asn Leu Ala Ile Gln Val Leu
   500 505 510
   Asn Gln Asp Lys Trp Thr Arg Glu Thr Val Gln Trp Leu Leu Asn Gly
   515 520 525
   Thr Cys Pro Gln Phe Val Ser Gly Leu Leu Glu Ser Gly Lys Ser Glu
   530 535 540
   Leu Lys Lys Gln Val Lys Pro Lys Ala Trp Leu Ser Arg Gly Pro Ser
   545 550 555 560
   Pro Gly Pro Gly Arg Leu Leu Leu Val Cys His Val Ser Gly Phe Tyr
   565 570 575
   Pro Lys Pro Val Trp Val Lys Trp Met Arg Gly Glu Gln Glu Gln Gln
   580 585 590
   Gly Thr Gln Pro Gly Asp Ile Leu Pro Asn Ala Asp Glu Thr Trp Tyr
   595 600 605
   Leu Arg Ala Thr Leu Asp Val Val Ala Gly Glu Ala Ala Gly Leu Ser
   610 615 620
   Cys Arg Val Lys His Ser Ser Leu Glu Gly Gln Asp Ile Val Leu Tyr
   625 630 635 640
   Trp Thr Gly His His His His His His
   645